# EUROPEAN PATENT SPECIFICATION

(11) **EP 1 565 558 B1**
(45) Date of publication and mention of the grant of the patent: **29.10.2014**
(21) Application number: 03780645.2
(22) Date of filing: 21.11.2003
(51) Int. Cl.: C12N 15/10

(54) **METHOD FOR ISOLATING INTRACELLULAR ANTIBODIES ABLE TO NEUTRALIZE PROTEIN INTERACTIONS**
ISOLATIONSVERFAHREN FÜR INTRAZELLULÄRE ANTIKÖRPER DIE PROTEININTERAKTIONEN NEUTRALISIEREN KÖNNEN
METHODE D'ISOLEMENT D'ANTICORPS INTRACELLULAIRES VISANT A NEUTRALISER DES INTERACTIONS PROTEIQUES

(30) Priority: 21.11.2002 IT RM20020588
(43) Date of publication of application: 24.08.2005
(73) Proprietor: Rottapharm Biotech S.r.l., 20052 Monza (IT)
(72) Inventor: VISINTIN, Michela, Lay Line Genomics S.P.A., I-00128 Roma (IT); CATTANEO, Antonino, Lay Line Genomics S.p.A., I-00128 Roma (IT)
(74) Representative: Capasso, Olga
(86) International application number: PCT/IT2003/000764
(87) International publication number: WO 2004/046192

(56) References cited:
- WO-A-00/54057
- GARGANO N ET AL: "FROM PHAGE LIBRARIES TO INTRACELLULAR IMMUNIZATION" INTRACELLULAR ANTIBODIES: DEVELOPMENT AND APPLICATIONS, SPRINGER VERLAG, BERLIN,, DE, 1997, pages 173-186, XP000916894
- TSE E ET AL: "Intracellular antibody capture technology: application to selection of intracellular antibodies recognising the BCR-ABL oncogenic protein" JOURNAL OF MOLECULAR BIOLOGY, LONDON, GB, vol. 317, no. 1, 15 March 2002 (2002-03-15), pages 85-94, XP004479165 ISSN: 0022-2836
- DAUVILLIER STEPHANIE ET AL: "Intracellular single-chain variable fragments directed to the Src homology 2 domains of Syk partially inhibit FcepsilonRI signaling in the RBL-2H3 cell line" JOURNAL OF IMMUNOLOGY, vol. 169, no. 5, 1 September 2002 (2002-09-01), pages 2274-2283, XP002284889 ISSN: 0022-1767
- CATTANEO A ET AL: "The selection of intracellular antibodies" TRENDS IN BIOTECHNOLOGY, ELSEVIER, AMSTERDAM, NL, vol. 17, no. 3, March 1999 (1999-03), pages 115-121, XP004157731 ISSN: 0167-7799 cited in the application
- WHITE M A: "THE YEAST TWO-HYBRID SYSTEM: FORWARD AND REVERSE" PROCEEDINGS OF THE NATIONAL ACADEMY OF SCIENCES OF USA, NATIONAL ACADEMY OF SCIENCE. WASHINGTON, US, vol. 93, 1 September 1996 (1996-09-01), pages 10001-10003, XP000749721 ISSN: 0027-8424
- TANAKA T ET AL: "Single Domain Intracellular Antibodies: A Minimal Fragment For Direct In Vivo Selection of Antigen-specific Intrabodies" JOURNAL OF MOLECULAR BIOLOGY, LONDON, GB, vol. 331, no. 5, 29 August 2003 (2003-08-29), pages 1109-1120, XP004447480 ISSN: 0022-2836
- VISINTIN M ET AL: "From genes to intracellular antibodies: Dissecting the proteome with splint, a single pot library of stable antibodies." MOLECULAR & CELLULAR PROTEOMICS, vol. 2, no. 9, September 2003 (2003-09), page 759, XP009032195 HUPO (Human Proteomics Organisation) 2nd Annual and IUBMB (International Union of Biochemistry and M;Montreal, Quebec, Canada; October 08-11, 2003 ISSN: 1535-9476 (ISSN print)
- VISINTIN MICHELA ET AL: "Selection of antibodies for intracellular function using a two-hybrid in vivo system" PROCEEDINGS OF THE NATIONAL ACADEMY OF SCIENCES OF THE UNITED STATES, NATIONAL ACADEMY OF SCIENCE, WASHINGTON, DC, US, vol. 96, no. 21, 12 October 1999 (1999-10-12), pages 11723-11728, XP002143442 ISSN: 0027-8424
- DER MAUR ADRIAN AUF ET AL: "Direct in vivo screening of intrabody libraries constructed on a highly stable single-chain framework." JOURNAL OF BIOLOGICAL CHEMISTRY, vol. 277, no. 47, 22 November 2002 (2002-11-22), pages 45075-45085, XP002284890 ISSN: 0021-9258
- PUTHALAKATH H ET AL: "Rapid selection against truncation mutants in yeast reverse two-hybrid screens" BIOTECHNIQUES, EATON PUBLISHING, NATICK, US, vol. 30, no. 5, May 2001 (2001-05), pages 984-988, XP002969848 ISSN: 0736-6205

## Description

The present invention concerns a method for the isolation of stable intracellular antibodies, able to neutralize protein interactions. In particular, the invention concerns an antibody fragment library for isolating and expressing functional antibodies in the intracellular environment (intracellular antibodies), capable of interacting with specific target proteins in the intracellular environment. The invention concerns a method for the isolation of a set of antibody fragments against significant portion of the protein-protein interactions of a given cell (interactome) or against the protein interactions that constitute an intracellular pathway or network.

It is known that that antibodies can be used to interfere in a highly specific manner with biological processes inside the cell (Cattaneo and Biocca 1997) (Cattaneo and Biocca 1999) (Biocca, Neuberger et al. 1990). A specific intracellular expression can result in the inhibition of target protein function (protein knock-out). In this way, a phenotype of interest can be conferred to a cell or to an organism by a suitable intracellular antibody. In particular, protein knock-out represents an advantageous method to approach and resolve the bottleneck in functional genomics (from genes to function). In fact, genomic and proteomic programs provide a wealth of information for the identification of genes and proteins but not of their function. The solution of the problem of previous art could facilitate the identification and validation of target proteins for drug development as well. Protein knock-out has remarkable advantages over alternative methods (gene knock-out or RNA knock-out, antisense, ribozymes or RNAi) (Rossi 1999; Capecchi 1989; Fire 1999; Tavernarakis, Wang et al. 2000) since:
- the protein is directly correlated with its biological function;
- it permits post-transduction modified versions of the protein to be knocked out or the protein's compartmentalized subpopulations;
- it allows modulation of knock-out intensity (gradual knock-out or knock down). Since protein function very often depends on specific protein-protein interactions, antibodies or antibody fragments able to interfere with protein-protein interactions will allow selective and specific protein function neutralization, while allowing other functions of the same protein, depending on other protein interactions.

Intracellular antibody technology can be extremely useful for functional genomics since it exploits:
- a virtually unlimited repertory of antibodies against any protein;
- the possibility to direct these antibodies to sites where the protein exerts its function inside the cell, and
- the possibility to obtain a specific and localized protein knock-out.

However, its application to functional genomics and to global proteomics requires the technology be implemented in a high-throughput process. This is why intracellular antibody technology is presently not applicable to functional genomics.

Intracellular antibody technology uses genes for specific antibodies that are suitably modified and obtained from the natural immune system (mouse or human) or from antibody libraries (synthetic or natural) exposed on the surfaces of filamentous phages as antibody fragments (phage display technologies, ScFv).

In terms of affinity, stability and solubility, the quality of antibody fragments isolated with phage display technology is proportional to the size of the library used for in vitro selection (Vaughan, Williams et al. 1996), given that the probability of finding an antibody with the properties of interest is proportional to the diversity of the library. This is why it is necessary to have large antibody libraries in order to obtain high-affinity antibodies capable of recognizing any antigen (Griffiths, Malmqvist et al. 1993) (Griffiths, Williams et al. 1994) (Sheets, Amersdorfer et al. 1998) (Vaughan, Williams et al. 1996) (Sblattero and Bradbury 2000). These libraries are tested with the antigens of interest, ligated in the solid phase; the antibodies are then selected by sequential cycles of absorption, washing, elution and growth. This selection procedure, however, does not ensure that selected antibodies are suitable for intracellular expression, since not all antibodies are able to function in the intracellular environment. At the current state of the art, the performance of antibodies suited for intracellular expression is not predictable a priori (Biocca, Ruberti et al. 1995; Proba, Worn et al. 1998), since the intracellular physicochemical conditions (cytoplasm and nucleus) differ greatly from those in which the antibodies are naturally found and normally synthesized (endoplasmic reticulum and secretory pathways).

Hence, an application of the technology to functional genomics requires a robust and predictable method to select a priori antibodies capable of functioning in the intracellular environment. An improvement has been achieved with the selection method based on a double-hybrid system (IACT or Intracellular Antibody Capture Technology) (Visintin et al. 1999; Visintin et al. 2002) and described in international patent application PCT WO 00/54057. Antibodies in the IACT format are able to interact with the corresponding antigen and result to be effective and functional when expressed as ordinary intracellular antibodies in the final user system. The IACT selection strategy is effectively diagnostic and predictive for the identification of functional intracellular antibodies starting from large libraries. The IACT technology has also been used in a derived procedure to rapidly and effectively identify the epitopes recognized by antibodies selected on their respective antigens (in vivo epitope mapping, IVEM, patent application RM2000A00561) (Visintin et al. 2002).

However, the development of IACT does not yet allow intracellular antibody technology to be applied to functional genomics, which requires a parallel throughput of at least 500 to 1000 genes to several thousand genes. Moreover, the IACT procedure does not ensure that selected antibody fragments are able to neutralize the function of the target protein. Only a subset of the selected antibodies, at most, will have the property of being neutralizing antibodies.

The solution to this problem constitutes the object of the invention, allowing a rapid, effective high-volume processing from genes to validated intracellular antibodies that are then expressed in appropriate cell models to study and neutralize the function of corresponding proteins.

In a specific implementation, the procedure described in this invention allows the direct isolation of antibody fragments able to specifically disrupt protein-protein interactions, thereby having the ability to selectively and specifically neutralize protein function. Therefore, the procedure described allows to isolate antibody fragments being validated intracellularly and protein function neutralizing.

As already mentioned, the use of IACT to isolate validated intracellular antibodies is severely limited by low cell transformation efficacy (yeast or mammal) where double-hybrid selection is operated, which, in turn, does not allow the introduction of antibody libraries (of scFv or single domain antibodies) with a diversity greater than 10⁵ antibodies, a range far from that required to ensure a significant probability of finding a clone with the desired properties (10¹⁰-10¹¹).

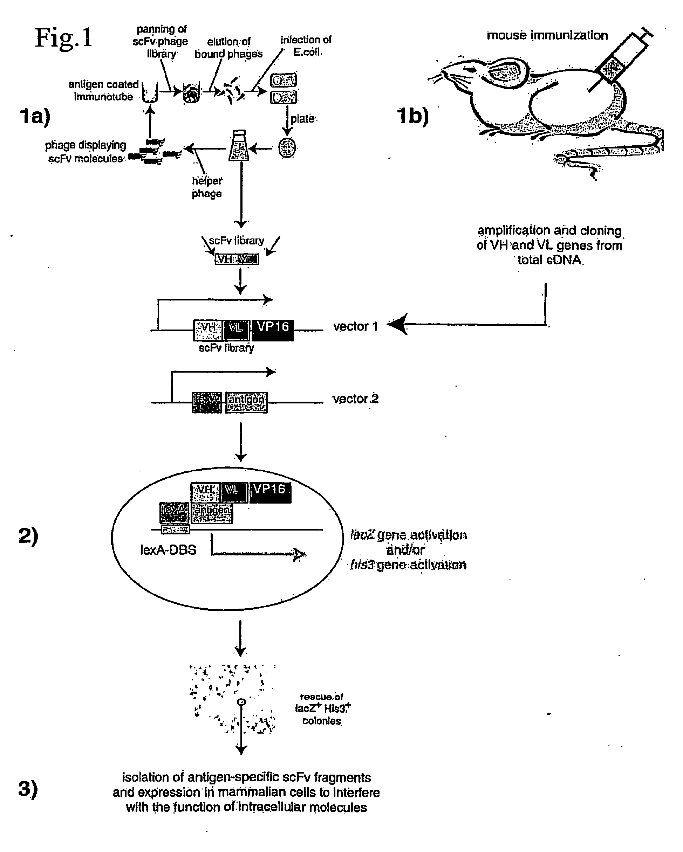

Presently, the IACT step is preceded by an enrichment of the library to reduce the diversity to levels compatible with the transformation in cells for the double-hybrid selection (Figure 1) by using:
- one or two absorption cycles on antigen columns in the solid phase in the case of a phage antibody library, or
- immunization of a mouse with the antigen if the antibody library is prepared from mouse lymphocytes.

These steps are extremely costly and slow. Furthermore, for the first step a good phage display library is needed. In both cases the enrichment step requires the availability of a protein coded by the gene of interest. This creates a highly significant bottleneck since the functional genomics programs provide the genes (cDNA, EST, ORF, etc.) but not the protein. The possible pipelines at the current state of the art are, therefore, limited by the fact that for each group of genes of interest an ad hoc preselection must be carried out each time.

The solution to this problem is offered by the construction of an antibody library expressed directly in the double-hybrid rather than in the phage display format. The entire procedure to obtain intracellular antibodies starting from the genes as input is facilitated, shortened, accelerated and rendered more parallel since the genes of interest could be directly inserted into the vector for IACT selection or its modifications, thus short-circuiting the need to express and purify the corresponding proteins.

A further implementation with respect to the IACT system is to ensure the isolation of neutralizing intracellular antibodies. In fact, the IACT system ensures only the isolation of biologically functional antibodies in an intracellular environment but does not select for neutralizing antibodies.

### DESCRIPTION OF THE INVENTION

In the following, an intracellular antibody is a synthetic or recombinant antibody in a single chain format (scFv), FAb or single domain (dAb) antibody, able to fold and to be functional in an intracellular environment (i.e. cytoplasm, nucleus).

A library of intracellular antibodies shall comprise: a) library of intracellular antibodies in which the antibody genes are V regions derived from i) mouse lymphocytes; ii) lymphocytes derived from patients with autoimmune disease; iii) lymphocytes derived from healthy people; b) a library of intracellular antibodies of point a) obtained with the method of the genetic recombination by two homologous recombination sites (BIG-SPLINT); c) a library of intracellular antibodies engineered on particular frameworks designed on the basis of the intracellular consensus sequences present in intracellular antibodies.

It is an object of the invention a method to isolate intracellular antibodies able to neutralize an interaction between a known protein ligand x and a known protein ligand y inside a cell, comprising the steps of:
a) obtaining a recombinant yeast strain wherein:
   i) **a sequence encoding the** protein ligand x and **a sequence encoding the** protein ligand y **are cloned into** of a first yeast expression vector, but
   ii) at least one of x or y is expressed in an inducible manner, and
   iii) the interaction between the protein ligand x and the protein ligand y would lead to the production either of a repressor of an essential function of the yeast cell or of a toxic agent, so that non viable yeast cells would be obtained;
b) transforming the recombinant yeast strain of point a) with an expression library of intracellular antibodies by means of a second yeast expression vector;
c) selecting transformed recombinant yeast strain of point b) by inducing the expression of both ligand x and ligand y; and by isolating recipient yeast clones able to grow;
d) isolating intracellular antibodies able to neutralize the interaction between the protein ligand x and the protein ligand y from recipient recombinant yeast clones able to grow,
wherein the expression library of intracellular antibodies is obtained from lymphocytes of non immunized mice and is expressed directly in the yeast cells without utilizing the phage display screening.

Another object of the invention is a method to isolate intracellular antibodies able to neutralize an interaction between a known protein ligand x and a known protein ligand y inside a cell, comprising the steps of:
a) obtaining a recombinant yeast strain having a mat-a or mat-alpha sexual competence wherein:
   i) **a sequence encoding the** protein ligand x and **a sequence encoding the** protein ligand y are **cloned into** a first yeast expression vector, but
   ii) at least one of x or y is expressed in an inducible manner, and
   iii) the interaction between the protein ligand x and the protein ligand y would lead to the production either of a repressor of an essential function of the yeast cell or of a toxic agent, so that non viable yeast cells would be obtained;
b) obtaining an expression library of intracellular antibodies by means of a second yeast expression vector in a yeast strain with a sexual competence opposite to that of the yeast strain in point a);
c) exposing yeasts of point a) and recombinant yeasts of point b) to conditions able to promote sexual mating and the expression of the ligands x and y;
d) selecting transformed yeast clones by inducing the expression of both ligand x and ligand y; and by isolating recipient yeast clones able to grow;
e) isolating intracellular antibodies able to neutralize the interaction between the protein ligand x and the protein ligand y from recipient recombinant yeast clones able to grow,
wherein the expression library of intracellular antibodies is obtained from lymphocytes of non immunized mice and is expressed directly in the yeast cells without utilizing the phage display screening.

In a preferred embodiment the recombinant yeast strain is obtained by:
a) cloning a first sequence encoding the protein ligand x and a second sequence encoding the protein ligand y into **the first** yeast expression vector, each first and second sequence being under the control of each one of two portions of a bidirectional promoters, the first sequence being fused to a sequence encoding a first molecule, and the second sequence being fused to a sequence encoding a second molecule, so that when the protein ligand x and the protein ligand y interact the production either of a repressor of an essential function of the yeast cell or of a toxic agent **is induced;**
b) transforming the recipient yeast strain with the **yeast expression** vector of point a); and
c) selecting transformed yeasts.

Preferably **the** bidirectional promoters **are** the promoters Gal 1 and Gal 10.

Preferably the first and the second molecule leads to the production of a repressor able to shut down the transcription of the yeast HIS3 gene.

Preferably the expression library of intracellular antibodies is obtained in a scFv or in a single domain antibody format.

It is another object of the invention a method to simultaneously identify both a protein ligand x able to bind to a known protein ligand y inside an eukaryotic cell, and an intracellular antibody able to neutralize said interaction between the protein ligand x and the known protein ligand y, comprising the steps of:
a) cloning a cDNA library containing the sequence encoding the protein ligand x and a sequence encoding the protein ligand y into a first yeast expression vector, the cDNA library sequences being under the control of one of two portions of a bidirectional inducible promoter, the cDNA library sequences being fused to a sequence encoding a first molecule; and the sequence encoding the protein ligand y being under the control of the other of two portions of the bidirectional promoter, and being fused to a sequence encoding a second molecule, so that when the protein ligand x and the protein ligand y interact said first molecule and said second molecule interact too, in a way to induce the production either of a repressor of an essential function of the yeast cell or of a toxic agent;
b) transforming a yeast strain with mat-a or mat-alpha sexual competence with the recombinant first yeast expression vector of point a);
c) selecting transformed yeasts
d) obtaining an expression library of intracellular antibodies in a yeast strain having a sexually competence opposite to that of the yeast strain in point a) by means of a second yeast expression vector;
e) submitting the yeasts of point c) and the yeasts of point d) to such conditions to promote sexual reproduction and the expression of the bidirectional promoter in an environment able to induce the expression of the protein ligand x and protein ligand y;
f) selecting viable yeast clones in which the interaction between the protein ligand x and the protein ligand y is disrupted by the intracellular antibody;
g) identifying the protein ligand x and the intracellular antibody,
**wherein the expression library of intracellular antibodies is obtained from lymphocytes of non immunized mice and is expressed directly in the yeast cells without utilizing the phage display screening.**

### DEATAILED DESCRIPTION OF THE INVENTION

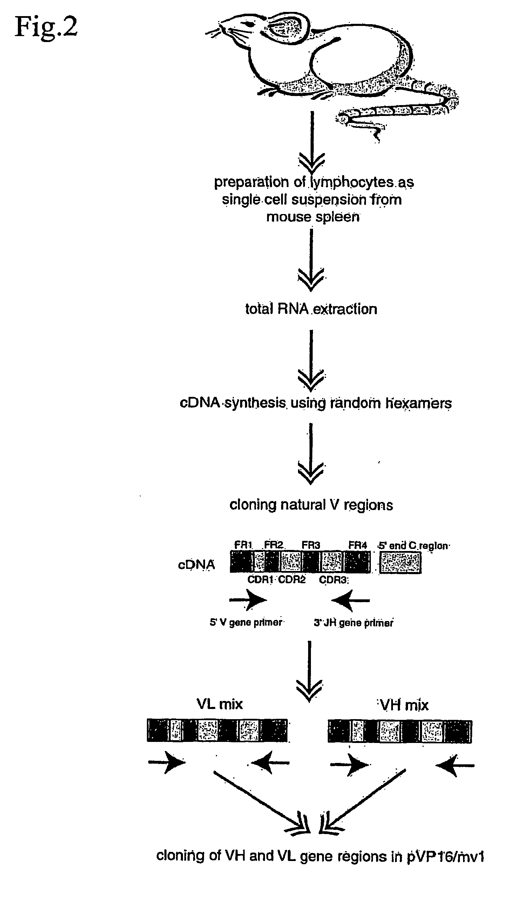

The authors have developed an intracellular antibody library according to a method comprising the following steps (Figure 2):
a) recovering DNA encoding the immunoglobulin natural V regions of B cells from spleen cells or peripheral lymphocytes by isolation of RNA, synthesis of complement DNA and amplification of DNA encoding light and heavy chains of immunoglobulins;
b) cloning said DNA using primers designed to contain restriction sites suitable for an expression vector comprising: 1) an antibiotic resistance gene different from the vector that expresses the protein x and y ligands, i.e. ampicillin; 2) a nutritional marker different from the vector that expresses the x and y protein ligands, i.e. LEU2; 3) replication origins for both E. coli (i.e. ColEl, fl) and for the yeast (i.e. 2µ); 4) a polylinker suitable for the insertion of an antibody library; 5) one or more nuclear localization signals downstream from the sequence encoding the scFv, but also FAb and antibody domains;
c) characterizing the library obtained by colony PCR-fingerprinting, and
d) selecting the SPLINT by IACT (SPLINT-2HY) on a known panel of antigens.

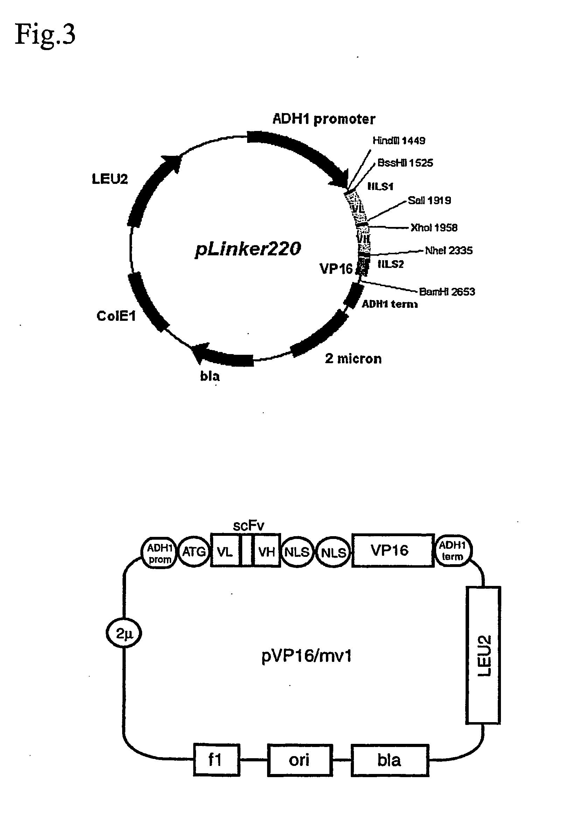

The vector of item b) is the vector pVP16/mv1 (Fig.3).

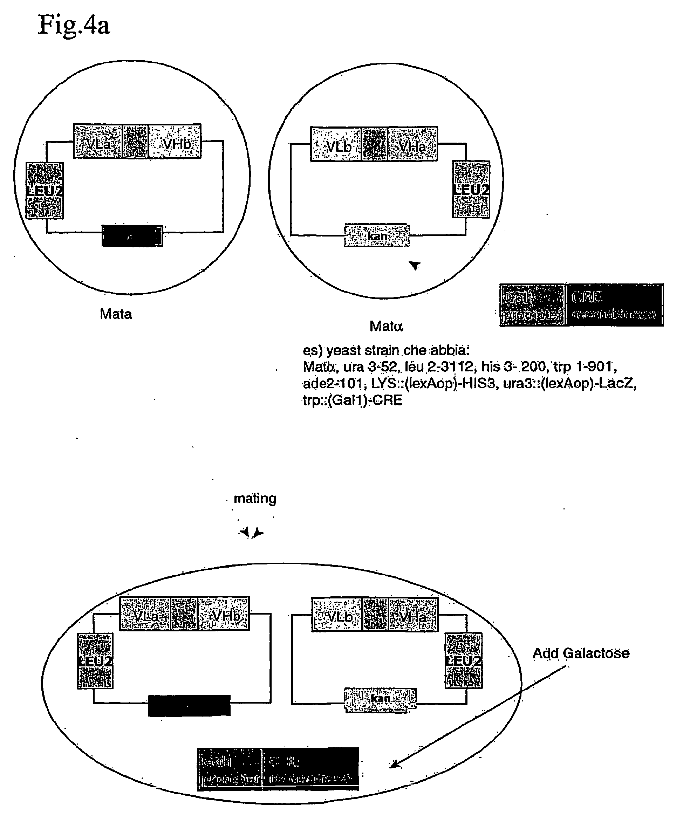

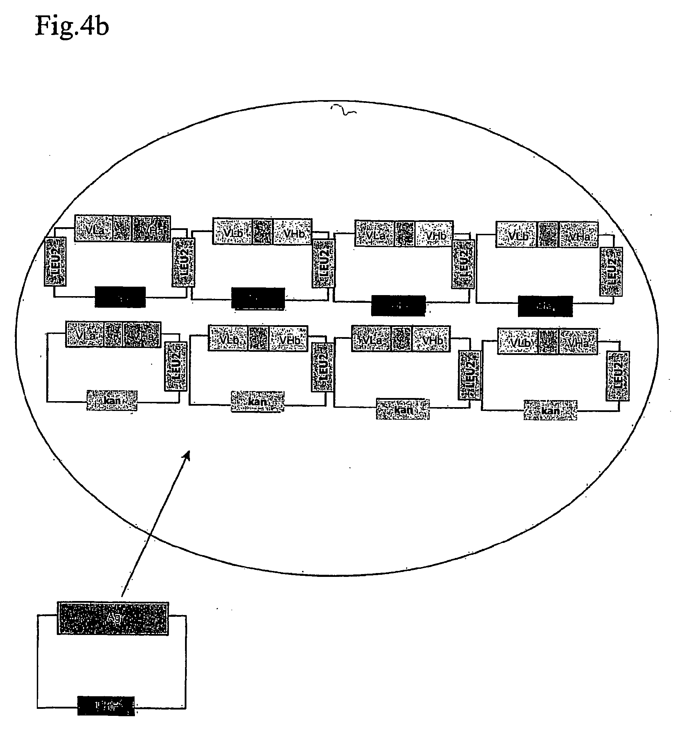

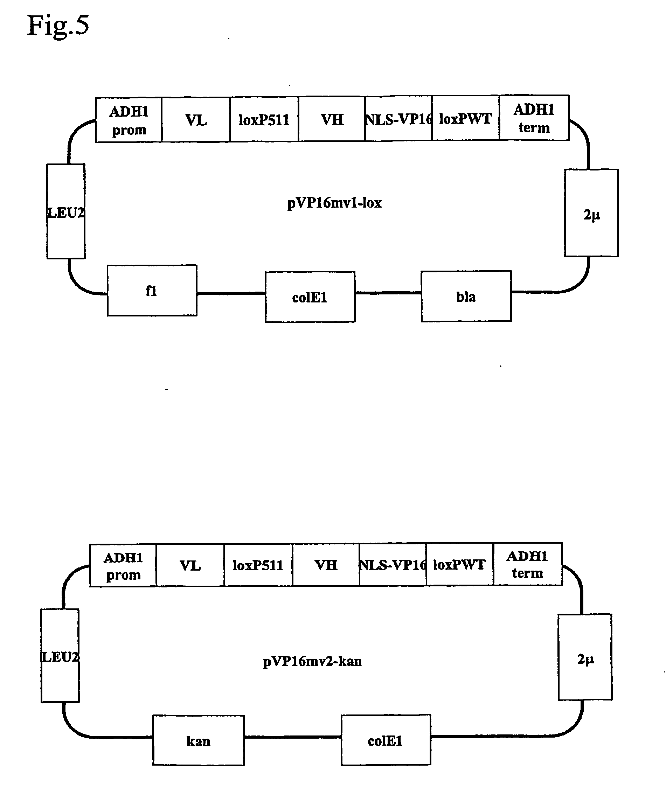

To overcome the limitation posed by the low efficiency of yeast transformation the authors set up a technology, named BIG-SPLINT (Fig. 4a, Fig. 4b), that uses recombination events to obtain a larger intracellular library after creating smaller primary libraries. A recombination strategy has been described as an alternative to create scFv fragment libraries expressed in phages (Sblattero and Bradbury, 2000; PCT WO 00311246), but it was never been applied to get intracellular antibody libraries in eukaryotic cells. The event of recombination is applied to the SPLINT intracellular library and is based on a modification of the pVP 16 vector so as to clone two lox recombination sites (lox P and lox P511) between VH and VL genes (pVP/16mv1-lox vector, Fig. 5). This system introduces a gene encoding the CRE recombinase induced by the GAL1 promoter only in the presence of galactose, integrated in the yeast genome. Initially, two mini-libraries are obtained in two pVP16/mv1-lox vectors that differ only in their nutritional and antibiotic markers. The VH and VL chains are then recombined after transformation (or mating) in the yeast strain containing the CRE recombinase gene, whose activity is induced only after the introduction of the two scFv libraries in the yeast strain (Fig. 4a, Fig. 4b).

The invention overcomes the previous technical problems by completely avoiding protein manipulation steps in the process "gene - intracellular antibody", thus allowing the isolation of intracellular antibodies against any of proteins encoded by a genome, and permitting the isolation of intracellular antibodies against all protein-protein interactions in a cell.

The SPLINT technology of the invention is predictive for sequences preferred by antibodies in intracellular environments, thus augmenting the knowledge of the structure adopted by intracellular antibodies in physiologically incompatible environments, and leading to the formulation of a family of consensus sequences of antibody scaffolds validated for intracellular expression.

A preferred embodiment of the invention makes use of antibody sequences already validated by the IACT procedure according to the following method:

### SUPER-SPLINT

As the selection procedures for intracellular antibodies are implemented, information is acquired on validated intracellular antibody sequences. Therefore various SPLINT generations (SUPER-SPLINT) may be obtained by using validated antibody sequences with the current IACT technology, as disclosed in the WO0054057 application, thus augmenting the intracellular stability and affinity of antibodies selected in vivo. The so called SUPER-SPLINT library is then obtained by grafting CDR (complementarity determining region) libraries on antibody scaffolds designed on the basis of information acquired from the study of validated intracellular antibodies. In this way, the size of the input library is smaller, thus permitting the antibody diversity to be increased in an antibody structure that is more targeted to intracellular expression.

The SPLINT libraries constitute an unlimited source of antibodies for a variety of uses in diagnosis, research, industry and therapy and, in general, for all those applications requiring more stable antibodies.

The SPLINT technology can be adapted to other cell types besides yeast, including E. coli and mammalian cells, so as to avoid the possible limitation of having antibodies against proteins expressed by the host cell.

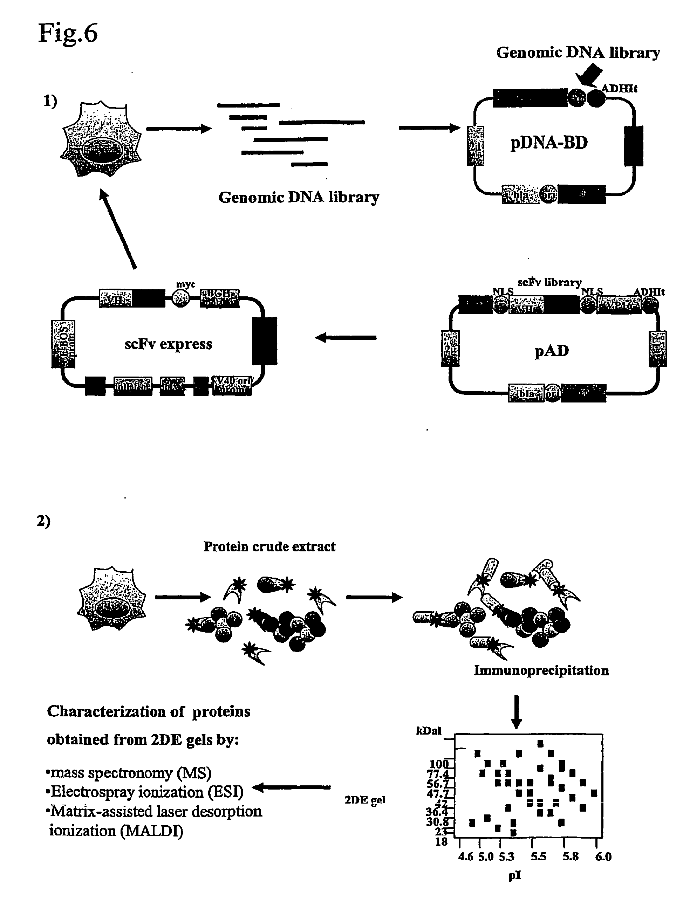

The SPLINT technology offers a new field of application for intracellular antibodies not possible before, in the so-called inverse functional genomics or AntiGENomics (Fig. 6), that leads to the identification of proteins involved in a cellular function of interest. In this format, intracellular antibody libraries derived by SPLINT (SUB-SPLINT) are used in suitable cell lines, for example mammalian cell lines, to induce a phenotype of interest and to identify the proteins responsible for the underlying biological processes. The AntiGENomics method thus allows the identification of the proteins involved in a given biological function, and solves a typical bottleneck of proteomic screenings, namely to assign a function to the proteins identified.

The invention is now described according to following examples.

### FIGURES

**Figure 1****.** Diagram of IACT technology. The intracellular antibodies can be isolated after combination between the two-hybrid system and the phage display technology or after immunization of a mouse and the subsequent construction of a scFv or antibody domains (Tomlinson and Holliger, 2000) (Wu and Yazaki, 2000; Kortt et al., 2001; Todorovska et al., 2001) (Tanaka et al., 2003)) library on a vector that has expressed an activation domain. After in vivo selection in the yeast cell, the positive testing clones are isolated and the scFv can be used for in vitro and in vivo applications.
**Figure 2****.** Diagram of the construction of a SPLINT library: the antibody variable regions can be cloned directly from a non-immunized mouse on a vector that expresses the activation domain and two NLS fused in tandem after the scFv antibody fragment. The SPLINT library is then inserted in the yeast strain in an episomal form and can then be used for IACT screening immediately after transformation of the antigen to be used as bait.
**Figure 3****.** Diagram of the plinker220 and pVP16mv1 vectors. The vectors presents 2 NLS fused at the 3' and 5' ends of scFv for pLinker220 or 2 NLS fused in tandem after the scFv in pVP16mv1. The scFv library is transcribed by the ADH1 promoter. Downstream from the library, there is an ADH term termination sequence. The vector also contains the sequence that encodes the ampicillinase gene (bla), the gene sequence LEU2 (nutritional marker for selection in yeast), an E. coli origin of replication f1 and an yeast origin of replication 2µ.
**Figure 4 a-b**. Diagram of the technology used to create a BIG-SPLINT library. In this system, two SPLINT libraries cloned in two pVP16mv1 vectors are used that differ only in antibiotic resistance to E. coli. The vectors present lox sites for heterologous recombination between the antibody chains. The gene encoding the CRE recombinase can be inserted or as a gene expressed by an inducible Gall promoter cloned in an episomal vector or can be present in the yeast strain previously constructed for this purpose.
**Figure 5****.** Diagram of the pVP16mv1-lox and pVP16m2 kan vectors. The two vectors are conceptually identical to the pVP16mv1 vector. The only difference is that upstream and downstream from the scFv-VP16 construct there are two lox511-loxPWT sequences that are recognized by the CRE enzyme that starts to recombine the variable chains directly in the intracellular environment.
**Figure 6****.** Diagram of the AntiGENomics technology. The technology uses in vivo selection of a cDNA library created from a cell line and the SPLINT antibody library. After isolation of a large panel of Ag-scFv pairs, the isolated genes are identified and the scFv they recognize are sequenced. These scFv are then expressed as a cell-specific library (SUB-SPLINT) in vivo, in the target cell of interest and used to recapture in vivo the proteins expressed in the cell, either directly or after a phenotypic selection. The intracellular scFv-protein complexes are then immunoprecipitated using the scFv sequence tag and analyzed using classic proteomic techniques such as 2Degel and MS.

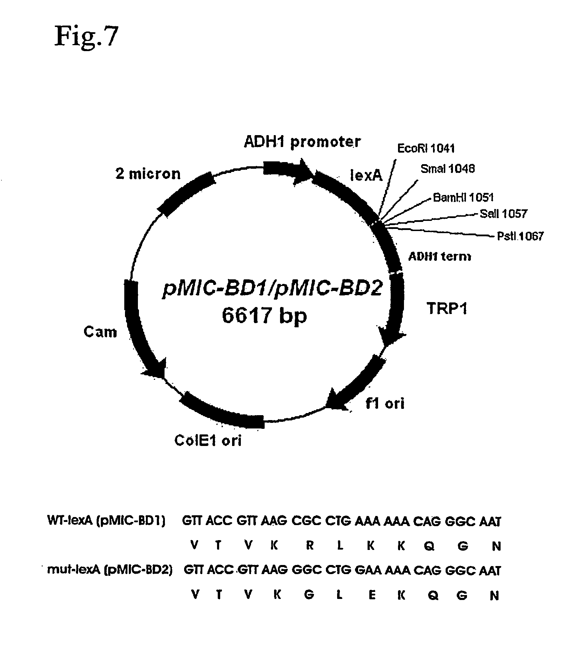

**Figure 7****.** Diagram of the vectors pMIC-BD1 and pMIC-BD2. The following vectors were constructed to express the antigen used as bait downstream from a DNA-binding domain (lexA). The two vectors differ only in the lack of NLS in pMIC-BD2, which was removed before mutagenesis. The ADH1 promoter promotes the transcription of the lexA-Ag fusion protein. Downstream from this construct there is an ADHlterm destruction sequence. The vector also has the sequence that encodes the chloramphenicol (cam) gene, the TRP1 gene sequence (nutritional marker for selection in yeast), an origin of replication f1 in E. coli and an origin of replication 2µ for the yeast.

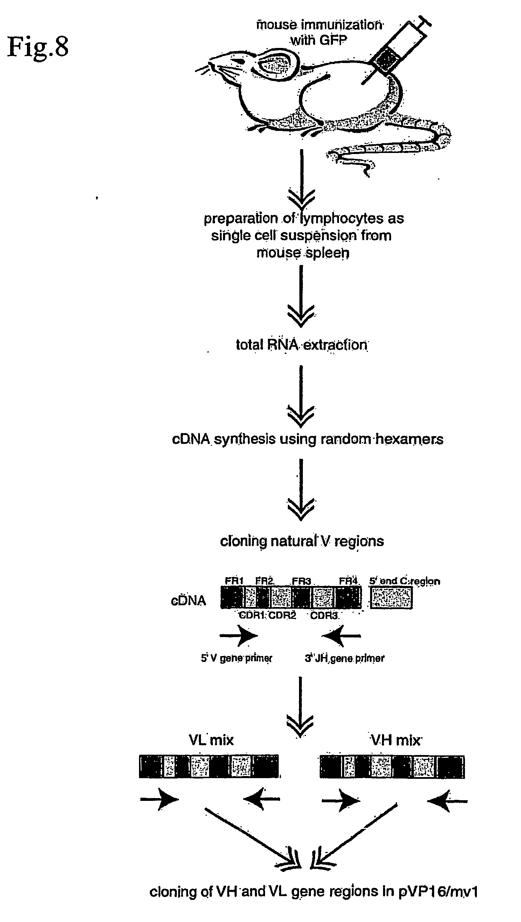

**Figure 8****.** Diagram of the construction of the anti-GFP library. A mouse is immunized with the GFP protein. The mouse spleen is then used as a source of lymphocytes to extract the RNA encoding the immunoglobulin variable chains. Once the cDNA has been prepared, the variable chains are amplified with specific degenerated primers. The anti-GFP library in the scFv format is then cloned in the pVP16mv1 vector and screened against the GFP cloned in the pBMIC-BD1 vector.

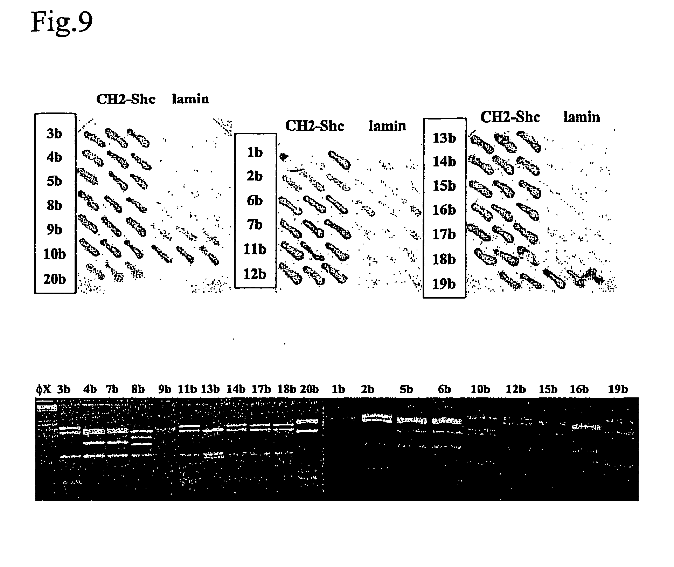

**Figure 9****.** X-gal assay and PCR-BSTNI fingerprinting on anti-CH2-Shc clones isolated from the hyperimmune library obtained after immunization of mice with GFP protein and selected with the IACT method.

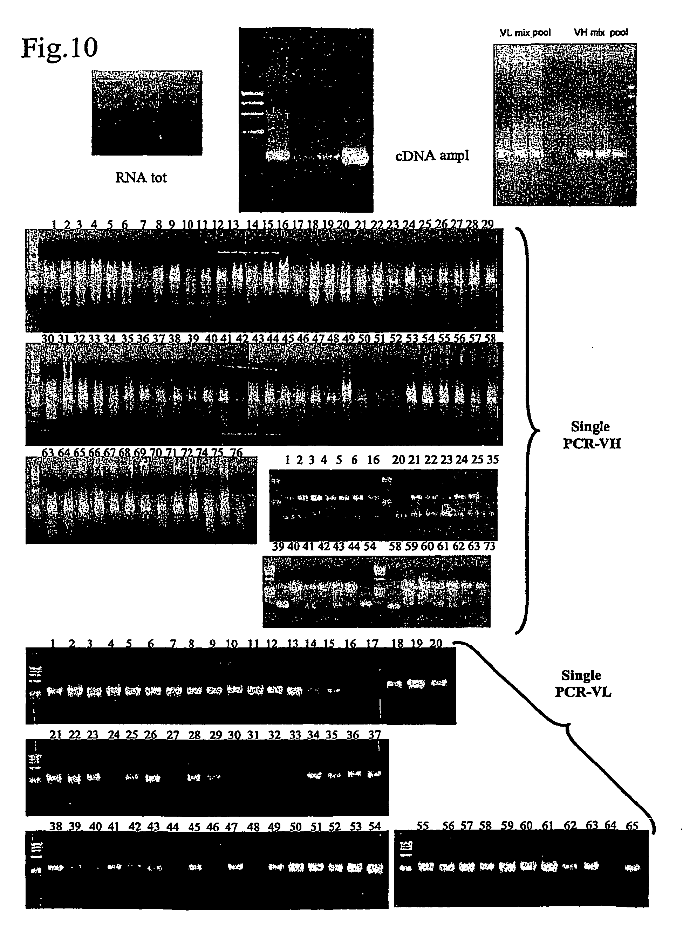

**Figure 10****.** The figure shows the various cloning steps of the SPLINT library: in the first panel to the left one can see the analysis of total RNA extracted from the nonimmunized mouse spleen. To the right, one can see the RT-PCR product reamplified with a mix of primers specific for the variable chains. The purpose of this step is to evaluate the quality of the cDNA before proceeding to amplification programmed with the various primer pairs (see table I). Below, one can see the analysis of the amplifications for the VH and VL with each degenerated primer pair. Lastly, after having quantified each single amplification for the VH and for the VL, each amplified product is mixed in equal concentrations; the final result is illustrated in the upper right photograph.

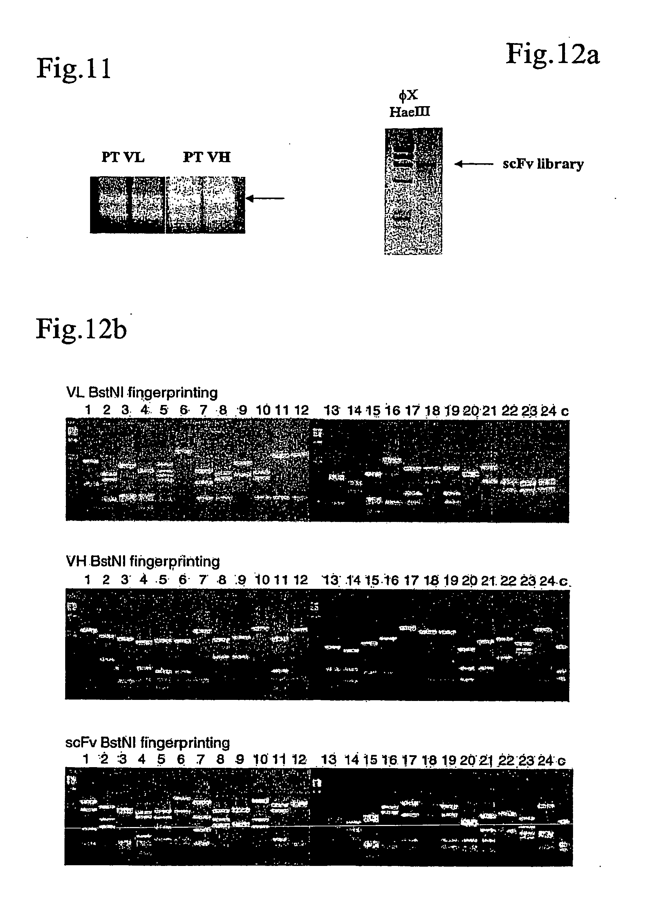

**Figure 11****.** Photograph of the VH and VL chain pull-through.
**Figure 12****. a.** Photograph of the SPLINT library assembly in the scFv format.**b**. PCR-BstNI fingerprinting of the SPLINT library after electroporation in the DH5*α*F' strain.

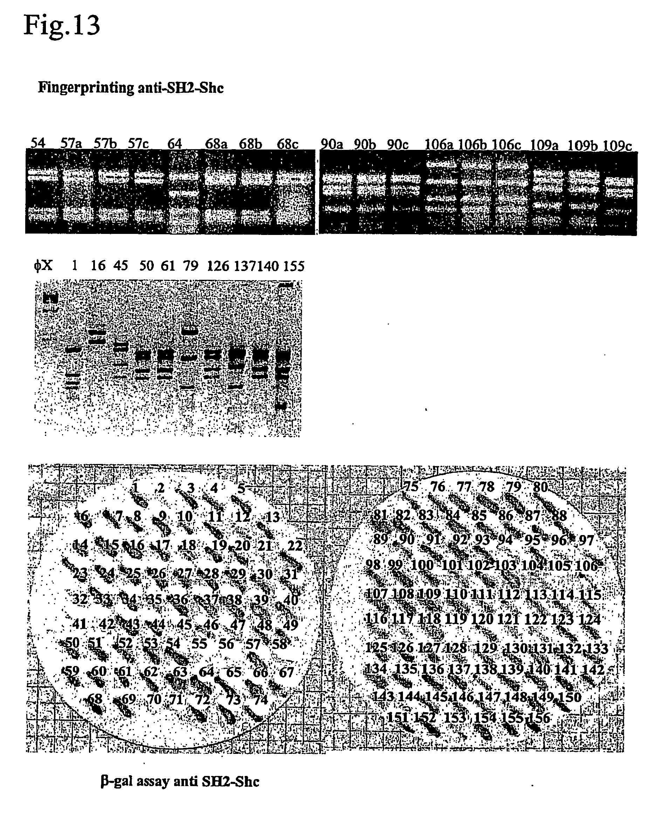

**Figure 13****.** X-gal assay and PCR-BstNI fingerprinting on anti-SH2-Shc clones isolated from the SPLINT library selected with the IACT method.

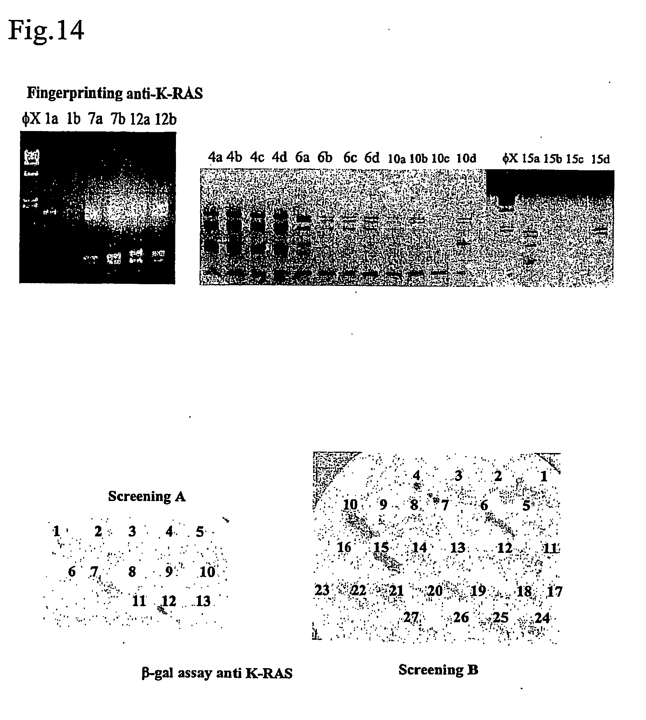

**Figure 14****.** X-gal assay and PCR-BstNI fingerprinting on anti-K-RAS clones isolated from the SPLINT library selected with the IACT method.

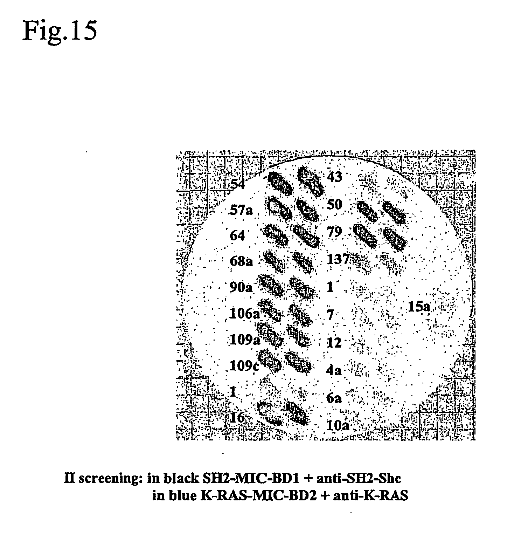

**Figure 15****.** X-gal assay for secondary screening on anti-SH2-SHc and anti-K-RAS clones with respective antigens used as baits.

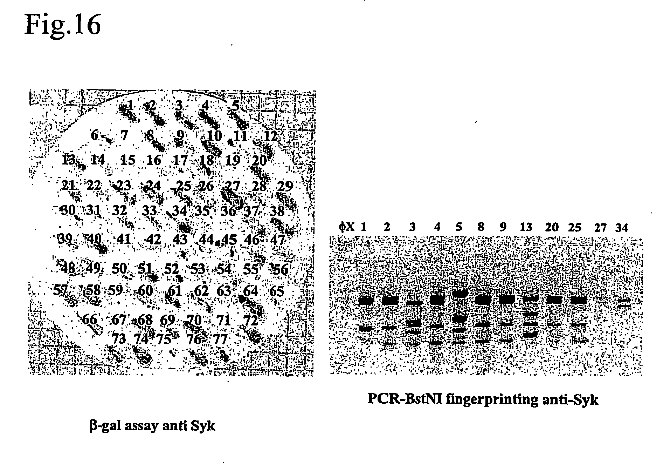

**Figure 16****.** X-gal assay and PCR-BstNI fingerprinting on anti-Syk clones isolated from the SLPINT library selected with the IACT method.

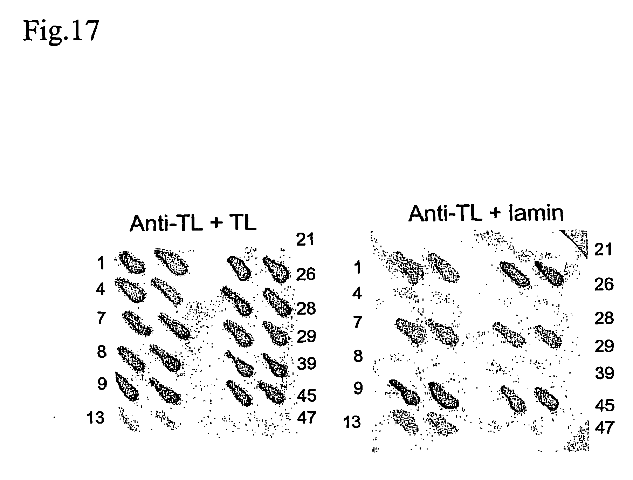

**Figures 17****.** Rescue scFv plasmids are reintroduced into original selection strain and into different bait strains to verify specificity. The isolated plasmid expressing the scFvs (i.e. anti-LTβ4) are reintroduced into strains containing target antigen (LTβ4) and another unrelated antigen (lamin). As an example clones b and h result to be specific only for LTβ4 and fail to interact with lamin antigen, thus confirming their specificity for LTβ4.

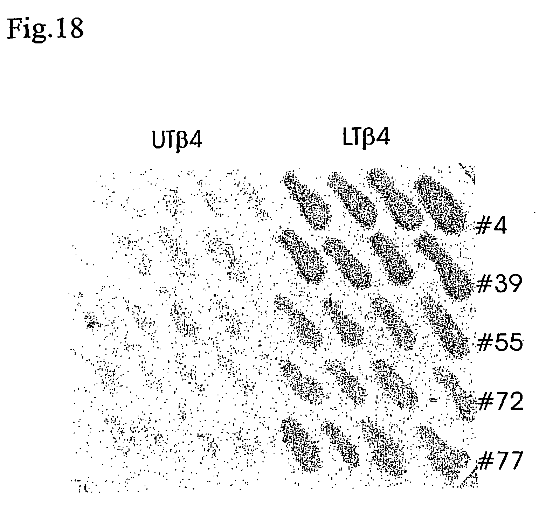

**Figure 18****.** Anti-LTβ4 scFv fragment specifically recognized LTβ4 and fail to recognize the UTβ4 bait. The thymosins are a family of small proteins entirely composed of a single WH2 domain, a ∼35 residue actin monomer-binding motif, that is found in many different regulators of the actin cytoskeleton The lymphoid-specific thymosin encodes for an extra-six-residue NH2-terminal extension. Five antibody fragments obtained from the LTβ4 screening were tested for their ability to bind UTβ4. As shown in figure, all the scFvs tested result to be specific only for the lymphoid form and not for the ubiquitous one.

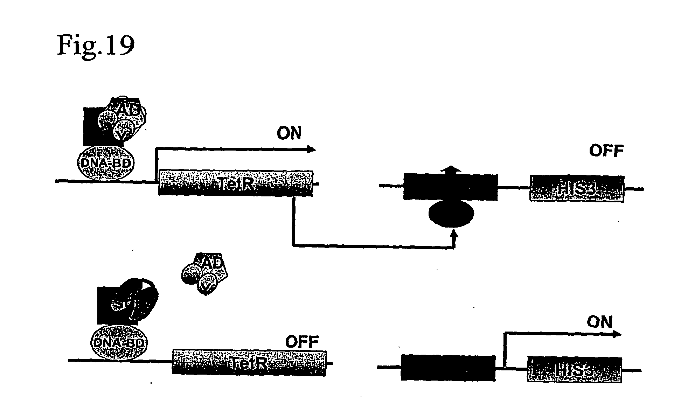

**Figures 19****.** Diagram of the method used with SPLINT in the three-hybrid system (see explanation in Results).

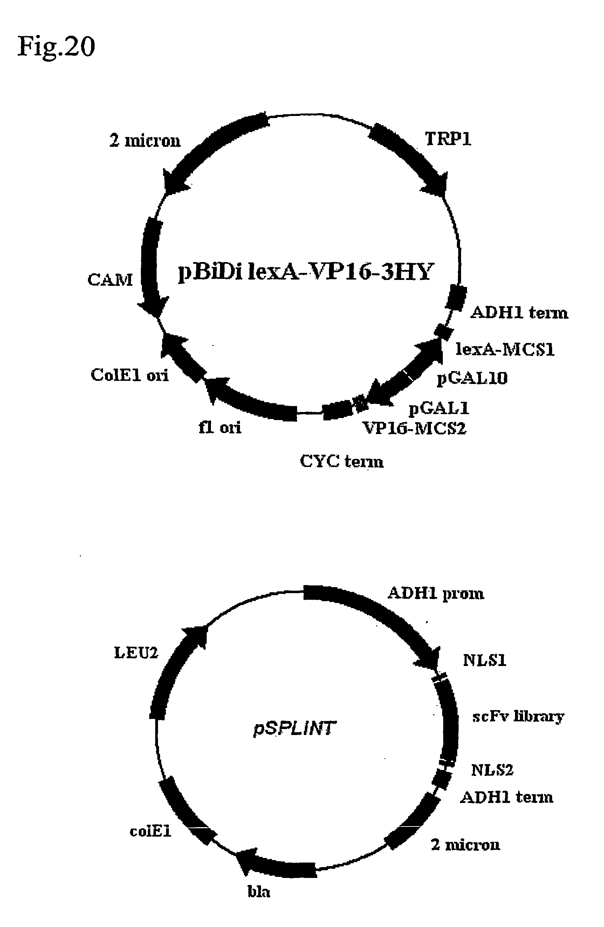

**Figure 20****.** Diagram of the pBiDi3HY, pBiDi3HYlexA-VP16 and pSLINT vectors. pBiDi3HYlexA-VP16 contains a bidirectional promoter for the expression of two cDNA libraries (1 and 2). Library cDNA1 is expressed as a fusion protein with lexA, whereas library cDNA2 is expressed as a fusion vector with VP16. Both libraries have two different stop sequences. The vector expresses as a nutritional marker for selection in the TRP1 yeast. The vector also has the sequence encoding the chloramphenicol gene (cam), an origin of replication f1 in E. coli and an origin of replication 2µ for the yeast. pSPLINT is a vector identical to pVP16mv1 but without the VP16 activation domain. This vector is used only to express scFv in the intracellular environment.

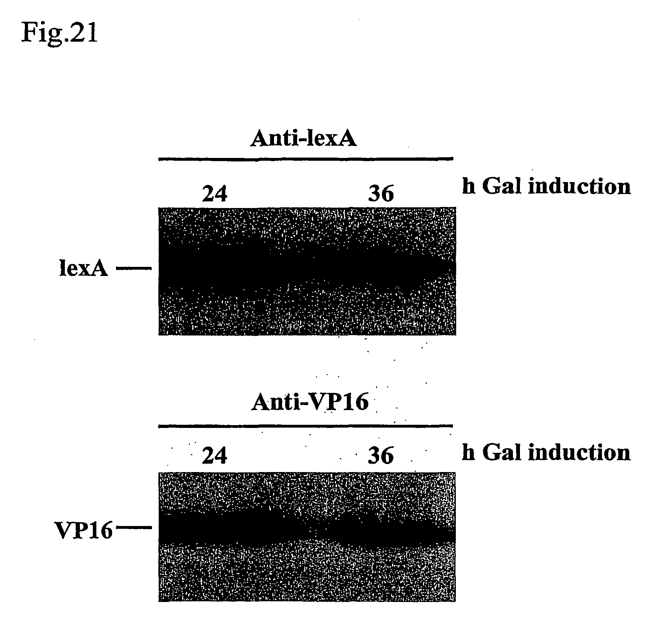

**Figure 21****.** Western blot analysis of lexA and VP16 proteins expressed upon induction with galactose in pBiDi3HYlexA-VP16 vector. After blotting, lexA was detected with the anti-lexA polyclonal antibody (Invitrogen) and VP16 with the anti-VP16 polyclonal antibody (Clontech).

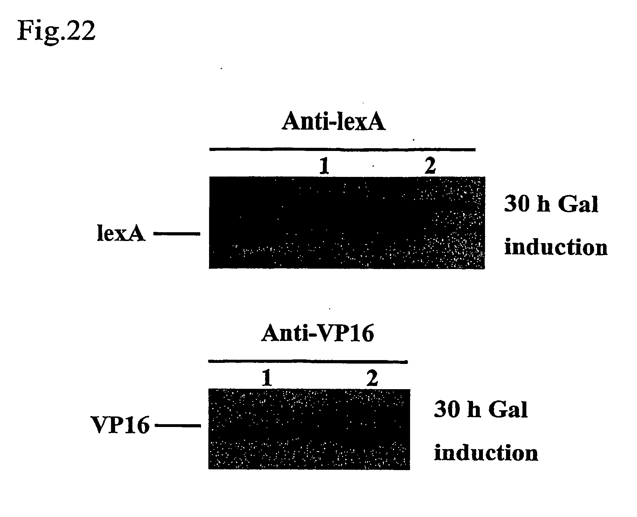

**Figure 22****.** Western blot analysis of lexA-p65 and VP16-p65 fusion proteins expressed upon induction with galactose in pBiDi3HYlexA-VP16 vector. After blotting, lexA-p65 was detected with the anti-lexA polyclonal antibody (Invitrogen) and VP16-p65 with the anti-VP16 polyclonal antibody (Clontech).

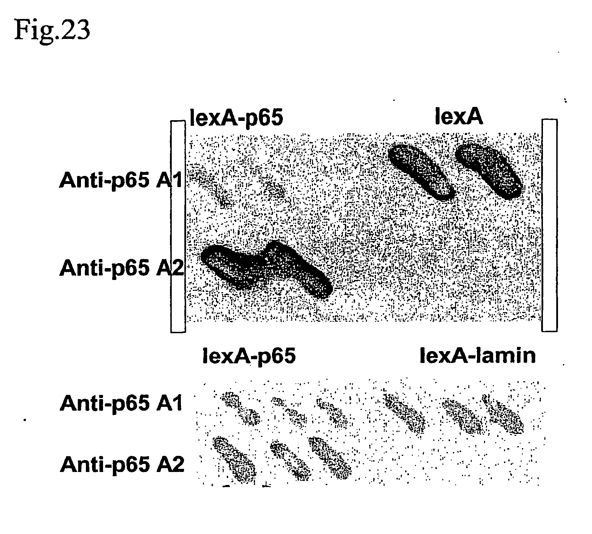

**Figure 23****.** p65 monomer-anti-p65 scFvs interactions in the yeast IACT system. L40 yeast cells were cotransformed with lexA-p65 bait vector and/or lamin bait with anti-p65 A1 or anti-p65 A2 -VP16 fusions in pLinker220 vector. Yeast colonies were grown on his plates and scored for β-ga1 assay. Clone A2 display a positive interaction with p65 monomer, thus indicating its specificity with the target protein. On the contrary, clone A1 was only able to recognize DNA-binding domain lexA.

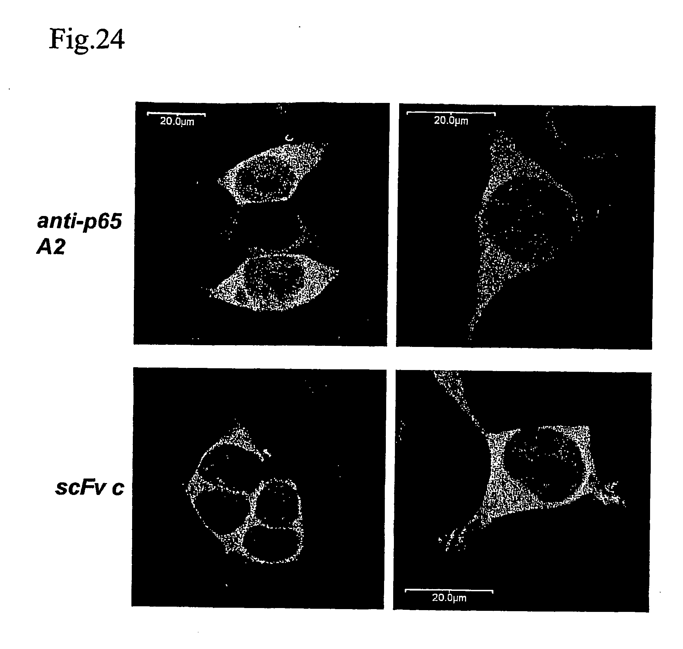

**Figure 24****.** Immunofluorescence microscopy of the anti-p65 A2 scFv transiently transfected in HEK cells. Cells were reacted with the anti-SV5 tag monoclonal antibody followed by incubation with an anti-FITC-conjugated anti-mouse (Vector).

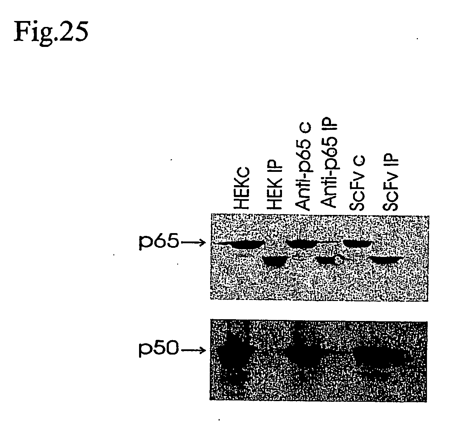

**Figure 25****.** HEK2973T cells were transiently transfected with anti-p65 A2 cloned in scFvex cyto-SV5 vector. Cell lysates were assayed for expression of p65 and p50 proteins (controls, c) and immunoprecipitation analysis with anti-p65 A2 or an irrelevant antibody.
p65 protein was detected with anti-p65 (upper lance) and anti-p50 (lower lane) polyclonal antibody (Santa Crutz).

### Vectors

### plinker220 (Fig. 3).

The vector for the expression of the scFvs or a library of scFv fragments was designed to contain a polylinker that can be used to clone antibody fragments that contain at their termini unique restriction sites identical to those present on the polylinker. To create this vector, the polylinker of the pDAN3 vector was cloned (Sblattero et al. 2000) in VP16* (Hollenberg et al. 1995). To obtain this vector, the VP16*vector was cut with the NcoI enzyme, treated with Calf Intestinal Phosphatase (CIP) and purified with a 0.75% agarose gel preparation. The pDAN3 polylinker (where a scFv was cloned beforehand) was amplified using the following oligonucleotides:
oligonucleotide POLY BACK: 
TCGATTTCCACCATGGCGCCGAAAAAAAAGAGAAAAGTGGCCCAGCCGAGCG
CGCATGCCGATATTGTG
Oligonucleotide POLY FOR:

TTCTTGGGTGCCATGGCGGAGCTCGAAACGCTAGCTGAGGAGACGGTGACCG
TGGT

The amplified fragment was then digested with the NcoI restriction enzyme and purified with 1.5% agarose gel. The two fragments were ligated using T4 DNA ligase. To confirm the perfect orientation of the insert and to confirm cloning, the vector was sequenced. The VP16 activation domain was then modified by removing several nonessential amino acids. To do this, the plinker200 was used as a template and VP16 was amplified using the following nucleotides:
VP16-220 back: TCGAGCGGTACCGCTAGCGTTTCGAGCTCCGCCATGG
MIC-AD2 for: CGGCCAGTGAATTCCTACTACCCACCGTACTCGTCAAT

The fragment containing the VP16 activation domain was then digested using the restriction enzymes NheI and EcoRI and cloned in the modified VP16 vector, cut in the same fashion, treated with Calf Intestinal Phosphatase (CIP) and purified with a 0.75% agarose gel preparation. The two fragments were ligated using T4 DNA ligase. The resulting vector was then called plinker220.

### pVP16/mv1 (Fig. 3)

The expression vector of the scFvs or a library of scFv fragments, pVP16/mv1, was designed to contain a polylinker that can be used to clone antibody fragments containing at their termini unique restriction sites identical to those present on the polylinker. The two nuclear location signals were shifted to permit their tandem expression downstream from the scFv and immediately upstream from the VP16 activation domain. This shift yields a scFv without steric hindrance at its 5'end, thus augmenting the specificity and folding of the antibody fragment fused to the VP16 activation domain at its 3'end. pVP16/mv1 was constructed in the following way:
oligonucleotide MIC-AD1 back:
   CAAACCGAAAAGCTTATGAGCGCGCATGCCGATATC
and a mixture of mouse VL oligonucleotides for the mix (Orlandi et al., 1992) were used to amplify a light chain cloned in plinker220. MIC-AD1 back contains the ATG start transcription codon and the restriction sites HindIII and BssHIII, while the mix of VL mouse oligonucleotides for the mixture contains the restriction site SalI. The DNA fragment amplified with these nucleotides was cut using the enzymes HindIII and SalI and purified with a 1.5% agarose gel preparation. The plinker220 vector was cut in the same fashion, treated with Calf Intestinal Phosphatase (CIP) and purified with a 0.75% agarose gel preparation. The two fragments were ligated using T4 DNA ligase. The resulting vector was then called pMIC-AD1.

pMIC-AD1 was used to prepare the final pVP16/mv1 vector. The following oligos were used:
MIC-AD2 back

CAAACCGCTAGCGCACCCAAGAAGAAGCGGAAGGTCGCACCCAAGAAGAAG
CGGAAGGTCCCAGGTGTACCGAGCTCG.

This nucleotide contains two nuclear location sequences (NLS) and the NheI restriction site.
MIC-AD2 for:
CGGCCAGTGAATTCCTACTACCCACCGTACTCGTCAAT.

This nucleotide contains the EcoRI restriction site.

The following nucleotides were used to amplify the VP16 activation domain from the plinker220 vector and to add upstream from it the two NLS encoded in tandem. The resulting DNA fragment was cut using NheI-EcoRI and purified with a 1.5% agarose gel preparation. The plinker220 vector was cut in the same fashion, treated with Calf Intestinal Phosphatase (CIP) and purified with a 0.75% agarose gel preparation. The two fragments were ligated using T4 DNA ligase to create the pVP16/mv1 vector.

### pMIC-BD1 (Fig. 7)

The expression vectors pVP16/mv and pMIC-BD were designed to selectively change the resistance for expression in E. coli. The introduction of two different antibiotic resistances (ampicillin and chloramphenicol, respectively) permits the removal of the bait plasmid from the cotransformed cells with the two plasmids, thus eliminating the need for classic segregation in yeast, which is very long and costly.

The vector for the antigen expression in yeast was designed to contain a prokaryotic marker selectable by the antibiotic chloramphenicol. The pMIC-BD1 vector was constructed so: the sequence containing the region encoding the DNA-binding domain lexA, the transcription stop sequence of the ADH1 gene and a part of the TRP1 yeast gene was isolated from the pBTM116 vector (Bartel, Chien et al. 1993) by digesting the plasmid with HindIII. The fragment was purified with 1.5% agarose gel. The expression vector used as a backbone, pBD-GAL4 Cam (Stratagene), was also cut using HindIII, dephosphorylated with Calf Intestinal Phosphatase (CIP), purified with 0.75% agarose gel and ligated with the lexA-ADHIt-TRP1 fragment to create the pMIC-BD1. The correct orientation of the insert was confirmed by sequence analysis.

### pMIC-BD2 (Fig. 7)

The lexA protein cloned in pMIC-BD1 was changed to abolish the nuclear location signal present inside the protein. wt-lexA GTT ACC GTT AAG CGC CTG AAA AAA CAG GGC AAT 
V T V K R L K K Q G N
mut-lexA GTT ACC GTT AAG GGC CTG GAA AAA CAG GGC AAT
V T V K G L E K Q G N
 (Rhee et al., 2000).

The CGC and AAA codons of the region encoding lexA were then mutated in the following way:
lexA-NLS back oligonucleotide
   AAGATGAAAGCGTTAACGGCCAGGCAACAAGAGGTG
   that contains the restriction site Hpal, and
lexA-NLS for 
AACGACAATTGGTTTAAACTCGCTATTTCTGGCAACAGTTCGACTTTATTGC
CCTGTTTTTCCAGGCCCTTAACGGT
that contains the two mutations and the PmeI restriction sites were used to amplify a part of lexA. The DNA fragment amplified with these oligonucleotides was cut with enzymes HpaI and PmeI and purified with a 2% agarose gel preparation. The pMIC-BD1 vector was cut in the same fashion, treated with Calf Intestinal Phosphatase (CIP) and purified with a 0.75% agarose gel preparation. The two fragments were ligated using T4 DNA ligase. The resulting vector was then called pMIC-BD2.

### pVP16mv1-lox and pVP16mv2-kan (Fig. 5)

As described in Sblattero D. et al 2000, the following recombination sequences were inserted in the pVP16mv1 vectors:
loxP511 ATA ACT TCG TAT AAT GTA TAC TAT ACG AAG TTA T
loxPWT ATA ACT TCG TAT AAT GTA TGC TAT ACG AAG TTA T

The polylinker of the vector is modified so:
loxP511 linker

TCC GGA GGG TCG ACC ATA ACT TCG TAT AAT GTA TAC TAT ACG AAG TTA TCC TCG AGC GGT ACC
BspEI SalI loxP511 XhoI KpNI

The first lox site (loxP511) is added by pull-through of the variable chains VL and VH. The primers for variable chain pull-through were:
VH PTL lox back 
GGA GGG TCG ACC ATA ACT TCG TAT AAT GTA TAC TAT ACG AAG TTA
TCC TCG AGC GGT A
VH PTMIC for
   CCA GGC CCA GCA GTG GGT TTG GGA TTG GTT TGC CGC TA
VL PTL lox for 
ACC GCT CGA GGA TAA CTT CGT ATA GTA TAC ATT ATA CGA AGT TAT
GGT CGA CCC TCC
VL PTMIC back
   CGC TGG ATT GTT ATT ACT CGC AGC AAG CGG CGC GCA TGC C

The second lox site (loxPWT) is added directly on vector pVP 16 at 3' of the VP16 activation domain. The VP16 gene is amplified from pVP16mv1 with the following primers:
VP16loxPWT for (cutting with BamHI) 
CTA TCT ATC TAg gat ccA TAA CTT CGT ATA GCA TAC ATT ATA CGA AGT
TAT CCC ACC GTA CTC GTC AAT
VP16 back (cutting with HindIII)
   GGC TAA AGC TTA TTT AGG TGA CAC TAT

The amplification products are then directly added as fragment Hind III-Bam HI on the vectors cut with the same enzymes pVP16mv1 and pVP16mv1-kan. The kan gene + ColE1 are inserted in vector pVP16* as an amplification product amplified by the plasmid pZero-2 (Invitrogen) and cloned in the sites DraIII and AaTII.
Kan DraIII back
Ccg atc gga cac CAG GTG CTG TCA GAA GAA CTC GTC AAG AAG GC
ColE1 AaTII for
AA TGA AAT GCA TCG GGA CGT CCT TCC GCT TCC TCG CTC ACT GAC

Having cloned colE1 and the gene for kanamycin resistance, the vector is changed as previously described to obtain the pVP16mv2-kan vector.

Recombination of the variable chains takes place in vivo in the yeast cell. The CRE gene, which encodes recombinase, is transcribed by the Gal1 inducible promoter. pGal1-CRE can be a plasmid integrated in the yeast strain or an episomal vector can be introduced with antibiotic resistance (neomycin) that expresses CRE under the Gall promoter. CRE can, for example, be cloned in a pESC vector (Stratagene) controlled by the Gall promoter. In this vector, antibiotic resistance (G418, neomycin) can be cloned to promote selection of this plasmid in the yeast cell. Gene G418 is transcribed by the Gal10 promoter, which is a bidirectional promoter with respect to Gal1. Both promoters are induced in the presence of galactose in a culture medium, thus permitting the control of CRE enzyme recombinase expression.

### pBiDi3HY vector (Fig.18):

to construct pBiDi3HY vector 2 vectors were used. The pESC-TRP vector (Stratagene) was PvuII cutted to isolate the bidirectional promoters Gal1-Gal10. The pMIC-BD1 vector was SphI cut and DNA polymerase I, Large (Klenow) fragment was subsequently used to remove the 3' overhangs to form blunt ends. The vector was also dephosphorylated with calf intestinal alkaline phosphatase to prevent recircularization of cloning vector. Cloning of Gal1-Gal10 PvuII digested into pMIC-BD1 SphI cut and blunt generated pBiDi3HY.

### pBiDi3HYlexA-VP16 vector (Fig.18):

the DNA fragment VP 16 was amplified from VP16* vector (Hollenberg et al., 1995) and cloned between the BamHI/ApaI of pBiDi3HY plasmid.

The DNA fragment lexA was amplified from pMIC-BD1 and cloned between the EcoRI/SpeI of pBiDi3HY-VP16 vector. The final vector pBiDi3HY-lexA-VP16 was sequenced and the expression of lexA and VP16 proteins was verified by western blot analysis of yeast crude extract after induction of promoters with galactose 20% (see figure 23).

**pBiDi3HYlexAp65-VP16p65 vector:** the member of NF-kB/Rel family p65 protein, was cloned as C-terminal fusion protein with lexA and VP16 domains. The DNA fragment p65 was amplified from pRSV NF-kB relA (p65) plasmid and cloned into SpeI to produce lexA-p65 fusion protein and between ApaI/SalI to produce VP16-p65 fusion protein. The expression of both p65 fusion proteins were verified by western blot analysis and sequences (see figure 24).

All clones were sequenced, using the Epicentre Sequitherm Excel II kit (Alsbyte, Mill Valley, CA), with a Li-Cor 4000L automatic sequencer (Lincoln, NE).

### Construction of a library of an immunized mouse (anti-GFP) (Fig. 8)

In the preparatory work leading to this invention, within the development of IACT technology, antibody libraries were constructed from the spleens of mice immunized against various antigens of interest, including EGFP (green fluorescent protein) (Prasher et al., 1992), human protein Aβ1-42 and others. The selection of these libraries against the immunized antigens does not constitute a novel aspect but is mentioned because it introduces the unexpected result described in the paragraph below.

Protein EGFP was expressed in E. coli and purified according to a standard protocol developed by (Baird et al., 1999). EGFP was then conjugated with BSA (bovine serum albumin) to augment its immunogenicity in so far as GFP is a highly compacted protein. 0.7 mg of the purified protein (both native and conjugated GFP) were used for the initial immunization of three mice. The subsequent boosts contained 1.0-1.7 mg of the purified protein (∼200-500µg of purified protein per mouse).

After about 2 months, the immunized mouse sera was analyzed by ELISA. The results showed that the mice presented a high titer of antibodies against GFP (we can find specific anti-GFP antibodies in analyzed sera also at higher dilutions, i.e. 1:10000). The spleens were then removed and used for RNA extraction.

The immunoglobulin variable regions were amplified using V-region PCR (Orlandi et al., 1992), using the following primers (Table I):

**TableI. Panel of degenerated primers used for the construction of SPLINT libraries**

| **VH mouse BACK:** |
|---|
| TTA TCC TCG AGC GGT ACC GAK GTR MAG CTT CAG GAG TC HB1 TTA TCC TCG AGC GGT ACC GAG GTB CAG CTB CAG CAG TC HB2 TTA TCC TCG AGC GGT ACC CAG GTG CAG CTG AAG SAS TC HB3 TTA TCC TCG AGC GGT ACC GAG GTC CAR CTG CAA CAR TC HB4 TTA TCC TCG AGC GGT ACC CAG GTY CAG CTB CAG CAR TC HB5 TTA TCC TCG AGC GGT ACC CAG GTY CAR CTG CAG CAG TC HB6 TTA TCC TCG AGC GGT ACC CAG GTC CAC GTG AAG CAG HB7 TTA TCC TCG AGC GGT ACC GAG GTG AAS STG GTG GAA T HB8 TTA TCC TCG AGC GGT ACC GAV GTG AWG YTG GTG GAG TC HB9 TTA TCC TCG AGC GGT ACC GAG GTG CAG SKG GTG GAG TC HB10 TTA TCC TCG AGC GGT ACC GAK GTG CAM CTG GTG GAG TC HB11 TTA TCC TCG AGC GGT ACC GAG GTG AAG CTG ATG GAR TC HB12 TTA TCC TCG AGC GGT ACC GAG GTG CAR CTT GTT GAG TC HB13 TTA TCC TCG AGC GGT ACC GAR GTR AAG CTT CTC GAG TC HB14 TTA TCC TCG AGC GGT ACC GAA GTG AAR STT GAG GAG TC HB15 TTA TCC TCG AGC GGT ACC CAG GTT ACT CTR AAA GWG TST G HB16 TTA TCC TCG AGC GGT ACC CAG GTC CAA CTV CAG CAR CC HB17 TTA TCC TCG AGC GGT ACC GAT GTG AAC TTG GAA GTG TC HB18 TTA TCC TCG AGC GGT ACC GAG GTG AAG GTC ATC GAG TC HB19 |

| **VH mouse FOR:** |
|---|
| GAT TGG TTT GCC GCT AGC TGA GGA GAC GGT GAC CGT GGT HF1 GAT TGG TTT GCC GCT AGC TGA GGA GAC TGT GAG AGT GGT HF2 GAT TGG TTT GCC GCT AGC TGC AGA GAC AGT GAC CAG AGT HF3 GAT TGG TTT GCC GCT AGC TGA GGA GAC GGT GAC TGA GGT HF4 |

| **VL mouse BACK:** |
|---|
| AGC AAG CGG CGC GCA TGC CGA YAT CCA GCT GAC TCA GCC LB1 AGC AAG CGG CGC GCA TGC CGA YAT TGT TCT CWC CCA GTC LB2 AGC AAG CGG CGC GCA TGC CGA YAT TKT GMT VAC TCA GTC LB3 AGC AAG CGG CGC GCA TGC CGA YAT TGT GYT RAC ACA GTC LB4 AGC AAG CGG CGC GCA TGC CGA YAT TGT RAT GAC MCA GTC LB5 AGC AAG CGG CGC GCA TGC CGA YAT TMA GAT RAM CCA GTC LB6 AGC AAG CGG CGC GCA TGC CGA YAT TCA GAT GAY DCA GTC LB7 AGC AAG CGG CGC GCA TGC CGA YAT YCA GAT GAC ACA GA LB8 AGC AAG CGG CGC GCA TGC CGA YAT TGT TCT CAW CCA GTC LB9 AGC AAG CGG CGC GCA TGC CGA YAT TGW GCT SAC CCA ATC LB10 AGC AAG CGG CGC GCA TGC CGA YAT TST RAT GAC CCA RTC LB11 AGC AAG CGG CGC GCA TGC CGA YRT TKT GAT GAC CCA RAC LB12 AGC AAG CGG CGC GCA TGC CGA YAT TGT GAT GAC BCA GKC LB13 AGC AAG CGG CGC GCA TGC CGA YAT TGT GAT AAC YCA GGA LB14 AGC AAG CGG CGC GCA TGC CGA YAT TGT GAT GAC CCA GWT LB15 AGC AAG CGG CGC GCA TGC CGA YAT TGT GAT GAC ACA ACC LB16 AGC AAG CGG CGC GCA TGC CGA CAG GCT GTT GTG ACT CAG GAA TC LBL |

| **VL mouse FOR:** |
|---|
| GAA GTT ATG GTC GAC CCT CCG GAA CGT TTK ATT TCC AGC TTG G LF1/2 GAA GTT ATG GTC GAC CCT CCG GAA CGT TTT ATT TCC AAC TTT G LF4 GAA GTT ATG GTC GAC CCT CCG GAA CGT TTC AGC TCC AGC TTG G LF5 GAA GTT ATG GTC GAC CCT CCG GAA CCT AGG ACA GTC AGT TTG G LFL |

To create a library that is as representative as possible, single amplifications were performed using each oligonucleotide described above according to the following schemes:

| | HB1 | HB2 | HB3 | HB4 | HB5 | HB6 | HB7 | HB8 | HB9 | HB10 | HB11 | HB12 | HB13 | HB14 | HB15 | HB16 | HB17 | HB18 | HB19 |
|---|---|---|---|---|---|---|---|---|---|---|---|---|---|---|---|---|---|---|---|
| HF1 | x | X | x | x | x | x | x | x | x | x | x | x | x | x | x | x | x | x | x |
| HF2 | x | X | x | x | x | x | x | x | x | x | x | x | x | x | x | x | x | x | x |
| HF3 | x | X | x | x | x | x | x | x | x | x | x | x | x | x | x | x | x | x | x |
| HF4 | x | X | x | x | x | x | x | x | x | x | x | x | x | x | x | x | x | x | x |

| | LB1 | LB2 | LB3 | LB4 | LB5 | LB6 | LB7 | LB8 | LB9 | LB10 | LB11 | LB12 | LB13 | LB14 | LB15 | LB16 | LBL |
|---|---|---|---|---|---|---|---|---|---|---|---|---|---|---|---|---|---|
| LF1/2 | x | x | x | x | x | x | x | x | x | x | x | x | x | x | x | x | |
| HF3 | x | x | x | x | x | x | x | x | x | x | x | x | x | x | x | x | |
| HF4 | x | x | x | x | x | x | x | x | x | x | x | x | x | x | x | x | |
| HF5 | x | x | x | x | x | x | x | x | x | x | x | x | x | x | x | x | |
| LFL | | | | | | | | | | | | | | | | | x |

This procedure allows verification of the library quality, without having to introduce a prevalence of determinate variable regions that are more easily amplified than others. In this specific case, 141 individual amplifications are carried out, starting from the cDNA obtained from the total RNA extracted from the immunized mouse spleen. The conditions used to amplify the variable chains were:
cDNA 0.5 *µ*l; H₂O 30.5*µ*l; 10X PCR buffer 5*µ*l; dNTP 2.5mM each 5*µ*l; MgCl₂ 25mM 3 *µ*l; Oligo1 (10pmol)/*µ*l) 2.5*µ*l; Oligo 2 (10pmol/*µ*l); 2.5*µ*lTaq polymerase 1*µ*l are mixed.

Mineral oil (50-100*µ*l) is added to prevent evaporation in the test tube containing the reaction mixture. The tube is then placed in a thermocycler programmed as follows: 94°C -5', (94°C -1' 60°C -1' 72°C-1') for 30 cycles; 72°C -10'; 4°C -24h.

After the variable regions have been amplified, they are analyzed on a 1.5% agarose gel and quantified. The same quantity of DNA for each amplified products is then used for cloning. A total of 10 *µ*g of a variable chain is then used for each cloning.

An initial VL library is created.

The light chains were processed as follows:
VL DNA mix 10*µ*g; BssHII (NEB 20 U/*µ*l)1.5*µ*l; Buffer 3 (NEB)10*µ*l; H₂O Q.S. (final V =100*µ*l).

The reaction mixture is incubated at 50° C for 8 h. The digested chains are then purified with 1.5% gel using a purification kit (Gel extraction kit, Qiagen). The purified DNA is then digested using the SalI enzyme according to the following scheme: VL DNA mix BssHII 100*µ*l; SalI (NEB 20 U/µl)1.5*µ*l; Buffer Sal (NEB) 2*µ*l; H₂O 78.5*µ*l.

The reaction mixture is incubated at 37° C for 8 h. The digested chains are then purified with a 1.5% gel using a purification kit (Gel extraction kit, Qiagen). After purification, the DNA is quantified on 1.5% agarose gel and analyzed under spectrophotometry at a wavelength of 260 nm.

10*µ*g of the pVP16/mv1 vector are cut in the same fashion: pVP16/mv1 DNA10*µ*g; BssHII (NEB 20 U/*µ*l) 1.5*µ*l; Buffer 3 (NEB)10*µ*l; H₂O Q.S. (final V=100*µ*l).

The reaction mixture is incubated at 50° C for 8 h. The digested chains are then purified with 0.75% gel using a purification kit (Gel extraction kit, Qiagen). The purified DNA is then digested using the SalI enzyme according to the following scheme: pVP16/mv1 BssHII 100*µ*l; SalI (NEB 20 U/µl)1.5*µ*l; Buffer Sal (NEB) 20*µ*l; H₂O 78.5*µ*l.

The reaction mixture is incubated at 37° C for 8 h. The digested vector is dephosphorylated with Calf Intestinal Phosphastase (CIF) and purified with 0.75% agarose gel. After purification, the DNA is quantified on 0.75% agarose gel and analyzed under spectrophotometry at a wavelength of 260 nm. pVP16/mv1 (BssHII-SaII-CIP) is then tested in E. coli to verify total digestion and dephosphorylation by electroporation of a known quantity of digested DNA incubated overnight with ligase or only in H₂O. After the vector background from a partial digestion of the vector or its religation has been verified (the background must be equal to or as close as possible to 0), pVP16/mv1 (BssHII-SalI-CIP) is then ligated with the light variable regions purified in a proportion of insert: vector=5:1.

About 6 µg of the vector is used in the ligase mixture. The ligase reaction is then left overnight at 16° C, the ligase is then inactivated for 10 min at 70° C; the ligase mix is then purified in the following way: 1 purification step with phenol (1:1); 1 purification step with phenol-chloroform (1:1); 1 purification step with chloroform-isoamyl (25:24:1) DNA precipitation with 3 volumes of sodium acetate/ethanol (solution 1 ml NaAc pH 5.2 + 27.5 ml EtOH). Wash the precipitated DNA with 200 µl of 70% EtOH. The DNA pellet is then resuspended in 20-30 µl of distilled H₂O.

10-40 ng of the purified ligase is then used for each electroporation. DH5*α*F' or TG1 is then made electrocompetent with the following method (Engberg et al., 1996) about 3-4 E. coli colonies are inoculated in 100 ml 2YT and grown at 37° C until O.D. 0.8-1. Two 50-ml flacon tubes are filled and left on ice for about 10 min. Thereafter, all steps are carried out in the cold room at 4° C. The tubes are centrifuged for 8 min at 3500 rpm at 4° C. The supernatant is removed. Each pellet is resuspended in 2 ml of 10% iced glycerol buffer (10% sterile glycerol in distilled sterile H₂O). The resuspended bacteria are then brought to a volume of 50 ml in a glycerol buffer. The tubes are centrifuged for 8 min at 3500 rpm at 4° C. The supernatant is eliminated. The pellets are resuspended in 2 ml of 10% iced glycerol buffer and transferred to a falcon tube. The bacteria are then brought to a volume of 50 ml in a glycerol buffer. Two further washings are performed according to the scheme described from points 6 to 8. Lastly, the final pellet is resuspended in 1 ml and brought to 3 ml in 10% glycerol buffer. The tubes are centrifuged for 8 min at 3500 rpm at 4° C. The supernatant is removed. The final pellet is resuspended in 125 µl 10% glycerol buffer. The bacteria are then divided into aliquots (30 µl per tube) in 0.5 ml Eppendorf tubes and conserved for about 1 month at -80° C.

The electroporator is set to 1800 V and the electrocompetent cells, together with the cuvettes, are placed on ice and cooled for about 10--15 min.

For each electroporation, 30 µl of bacteria plus 1 µl of ligase mixture (10-40 ng DNA) are used. After transformation, the bacteria are resuspended in 1 ml of SOC medium + 10 mM MgCl₂ and incubated for 1 h at 37° C. The bacteria are then seeded on LB + ampicillin plates. The next day, the efficacy of transformation is evaluated. To control the diversity of the VL library, 100 colonies are sampled at random from the plate and analyzed by PCR-fingerprinting using the BstNI restriction enzyme. After diversity has been confirmed, the bacteria are collected from the plate and frozen at -80° C in 20% glycerol.

The assembly of the VH chains with the VL chains is performed directly on the VL library. About 10µl is removed from the VL library glycerolate and inoculated in 50 ml LB + Amp overnight. The next day, the DNA for VH chain cloning is prepared. The heavy variable chains are processed as follows: VH DNA mix 10*µ*g; XhoI (NEB 20 U/*µ*l) 1.5*µ*l; NheI (NEB 20 U/*µ*l) 1.5*µ*l; Buffer 2 (NEB)10*µ*l; BSA (10mg/ml) 1*µ*l; H₂O Q.S. (final V=100*µ*l). The reaction mixture is incubated at 37° C for 8 h. The digested chains are then purified with a 1.5% gel using a purification kit (Gel extraction kit, Qiagen). After purification, the DNA is quantified on 1.5% agarose gel and analyzed under spectrophotometry at a wavelength of 260 nm.

10*µ*l of the VL library is cut in the same fashion: VH DNA mix10*µ*g; XhoI (NEB 20 U/*µ*l) 1.5*µ*l; NheI (NEB 20 U/*µ*l) 1.5*µ*l; Buffer 2 (NEB) 10*µ*l; BSA (10mg/ml) 1*µ*l; H₂O Q.S. (final V=100*µ*l). The reaction mixture is incubated at 37° C for 8 h. The digested vector is dephosphorylated with Calf Intestinal Phosphastase (CIF) and purified with 0.75% agarose gel. After purification, the DNA is quantified on 0.75% agarose gel and analyzed under spectrophotometry at a wavelength of 260 nm. The VL library (XhoI-NheI-CIP) is then tested in E. coli to verify total digestion and dephosphorylation by electroporation of a known quantity of digested DNA incubated overnight with ligases or only in H₂O. After the vector background from a partial digestion of the vector or its rebinding is verified (the background must be equal to or as close as possible to 0), the library is then ligated with the heavy variable regions purified in a proportion of insert: vector=5:1.

Thereafter, the procedure is continued as described above by the preparation of the VL library. A good library should have about 10⁶-10⁷ different scFv/µg of the DNA used. The pVP16/mv1 vector library is then assayed with GFP bait (immunizing antigen).

The GFP bait was amplified from pEGFP-N1. The amplification primers of the GFP gene were: GFP sense: CCG GTC GAA TTC ATG GTG AGC AAG GGC GAG GAG and GFP antisense: TGA TCT GGA TCC GCG GCC GCT TTA AGT GAT CCC GGC. Since previous experiments showed that GFP alone was able to transactivate the transcription of the reporter genes in the yeast system, a mutant of the GFP deletion was used for this experiment. The literature reports that the removal of the last 8 amino acids of the GFP maintains the fluorescent activity of the protein and its conformation unchanged (Siegel and Isacoff, 1997).

The DNA fragment amplified with these oligonucleotides was then cut with enzymes EcoRI and BamHI and purified with a 1.5% agarose gel preparation. The pmIC-BD1 vector was cut in the same fashion, treated with Calf Intestinal Phosphatase (CIP) and purified with a 0.75% agarose gel preparation. The two fragments were ligated using T4 DNA ligase.

To verify transactivation of the bait GFP, self-activation of reporter gene transcription was tested according to the transformation steps shown below:

| bait | Prey | YC selection medium | HIS3 phenotype | LacZ phenotype |
|---|---|---|---|---|
| LexA-GFP | | -W | / | white |
| LexA-GFP | | -WHUK | / | |
| LexA-GFP | VP16/mv1 | -WL | | white |
| LexA-GFP | VP16/mv1 | -WHULK | / | |

Having confirmed the non transactivation of the bait GFP and its good expression in the yeast strain (a yeast extract transformed with bait GFP is tested by western blot analysis using a primary antibody that recognizes the lexA protein), selection of the library in yeast cells is performed. L40 expressing bait GFP is transformed with 100 µg of scFv anti-GFP library according to the protocol described below:
Day 1: inoculation of L40 containing bait GFP in 5 ml of YC-UW overnight;
Day 2: inoculation of 100 ml of YC-UW with an aliquot of the overnight culture in order to have a dilution that allows the algorithmic growth phase to be reached the next day;
Day 3: the overnight culture is transferred in 11 of heated YPAD to obtain a culture with an OD 600= 0.3; the yeast is grown at 30° C for 3 h, the cells are centrifuged at 1500 rpm for 5 min at room temperature; the yeast pellet is washed with 500 ml of 1 x TE and then centrifuged at 1500 rpm for 5 min at room temperature; the pellet is resuspended in 20 ml of 1 x LiAC, 0.5 x TE and transferred to a fresh flask; 100 µg of scFv anti-GFP library and 1 ml of denatured salmon sperm are added; 140 ml of 1 x LiAc, 40% PEG 3350, 1 x TE; the product is mixed and incubated in a low-speed agitator for 30 min at 30° C; 17.6 ml of DMSO are added and mixed. Thermal shock is performed for 10 min at 42° C while agitating occasionally. The product is quickly cooled by adding 400 ml of YPA. The yeast is pelleted by centrifugation and washed once with 500 ml of YPA. After centrifugation, the pellet is resuspended in 11 of YPAD preheated to 30° C. The product is incubated for 1 h at 30° C; 1 ml of the culture is isolated and the pellet obtained by centrifugation of this ml is resuspended in 1 ml of YC-UWL. Dilutions of 1:10, 1:100, and 1:1000 are seeded on YC-UWL plates to calculate the efficiency of the transformation. The pellet obtained from the remaining culture is washed twice with YC-WHULK. The final pellet is resuspended in 10 ml of YC-WHULK. The aliquots are plated on YC-WHULK plates and after 3-4 days the colonies that have grown are analyzed to determine the interaction.

96 colonies grown on YC-WHULK and testing blue on beta-Gal assay were analyzed by PCR-fingerprinting analysis using the BstNI restriction enzyme. The analysis of digestion patterns and scFv sequences isolated from the HIS+lacZ+ colonies permit the isolation of about a dozen different scFv that recognize the bait (also further to a second screening in yeast cells, the same bait that expresses GFP with scFv isolated in L40 is transformed) for each selection. The isolated scFv were then cloned in a scFv expression vector (Persic, Righi et al. 1997) for expression in mammalian cells and simultaneously in pUC119CAT for expression and production of the protein in E. coli. The binding affinity of the proteins purified with the affinity column was analyzed using plasmonic surface resonance. On average, the scFv isolated with IACT have an affinity constant of 100-500 nM. The scFvs expressed in vivo in mammalian cells (COS, CHO, PC 12, C6, etc.) bind the target protein and in most cases manage to dislocate the same from its cellular expression environment if they are expressed with localization signals differing from those of antigen expression. Also, neutralizing scFv can be found that inhibit antigen function or the antigen can be neutralized by taking it to another cell compartment using the Ag-Ab specific binding.

The results of this selection demonstrate that the enrichment obtained by immunization allows the construction of a library with a size adequate for yeast transformation and with a diversity wide enough to ensure the isolation of a panel of numerous antibodies against the immunizing protein.

Similar results have been obtained with a library created from the spleens of mice immunized against protein Aβ 1-42, a human protein, from which numerous antibodies against the immunizing protein were derived (see table 2).

At this point, a surprising and completely unexpected result emerged from the screening of the hyperimmune spleen-derived libraries with bait constructed from proteins not relevant to immunization.

### Selection of a hyperimmune SPLINT library against antigens different from that used for immunization

The libraries obtained from immunized mouse spleens (mice immunized with GFP), as described in the paragraph above, were screened against protein She (a protein that regulates cell growth, apoptosis and lifespan) and human Aβ1-42 (the highly amyloidogenic component accumulation linked to the pathogenesis of Alzheimer's disease).

A functional domain of the Shc protein (CH2-Shc) and the human Aβ1-42 were cloned in the pMIC-BD1 vector and expressed in yeast to control the capacity of the two antigens to transactivate the reporter genes.

### Construction of the baits:

CH2-Shc/MIC-BD1: the DNA fragment CH2-Shc was PCR amplified from pGEX4T1p66Shc and cloned between EcoRI/BamHI restriction sites of pMIC-BD1 plasmid.

Aβ 1-42/MIC-BD1: the DNA fragment of beta amyloid 1-42 domain was synthesize by primers annealing and then cloned into BamHI/PstI restriction sites of pMIC-BD1 plasmid.

The pMIC-BD vector was constructed to be used in a yeast strain that kept the reporter genes under the control of the operator site for protein lexA. This vector also permits a rapid extraction of positive clones, after verification of an antibody-antigen interaction in a two-hybrid system that uses as a vector containing the activation domain an ampicillin-resistant plasmid in that it utilizes chloramphenicol as the phenotype marker in E. coli. All antigens were tested in vivo as described elsewhere (Visintin and Cattaneo 2001) (Visintin, Tse et al. 1999) and the expression of the fusion proteins was assayed after the proteins were extracted from the transformed yeast strain. All antigens were found to be expressed in optimum quantities in the yeast and not to transactivate the reporter genes (HIS3 and lacZ).

CH2-Shc was selected as the antigen for library screening. The efficiency of the transformation of the library was about 2.5 x 10⁴ clones/µg DNA. Unexpectedly, hundreds of positive colonies (TableIII) were also obtained after the library was plated after the first day of transformation (see protocol in Visintin and Cattaneo 2001). The protocol used here was the same as that described above (see Construction of a library of an anti-GFP immunized mouse)

After about 15 min., the colonies turned blue on X-Gal assay, an indication that the observed interactions were quite strong. Twenty blue colonies were isolated and the plasmids containing the scFv fragments to be analyzed were rapidly isolated after extraction of total DNA from the yeast and transformed in E. coli. Analysis by PCR-BstNI-fingerprinting and a second two-hybrid screening yielded the following results (Fig. 9): 19 scFv, 9 classes of different scFv. Of the 9 classes of scFv, 8 tested specific against CH2-Shc and not against an irrelevant antigen (laminin) used for the screening. Likewise, in a separate selection from a library derived from a spleen hyperimmune for an unrelated antigen, and challenged with the Aβ 1-42 bait, a high number of different antibody domains against the Aβ 1-42 protein were isolated (data not shown, table III).

**Table III. Selection of ICAbs with different antigen specificities.**

| Antigen | cfu/µg SPLINT-DNA in L40 | n. of His3⁺-lacZ⁺ rescued clones | n. of His3⁺-lacZ⁺ analyzed clones | n. positive clones |
|---|---|---|---|---|
| CH2-Shc | 2.4e10⁶ | 430 | 20 | 8 |
| SH2-Shc | 3.7e10⁶ | 189 | 20 | 5 |
| K-RAS | 4.4e10⁶ | 27 | 27 | 2 |
| Syk | 1.4e10⁶ | 3000 | 10 | 6 |
| UTβ4 | 3e10⁷ | 300 | 96 | 3 |
| LTβ4 | 2.1e10⁶ | 123 | 123 | 13 |
| Gephyrin | 2.6e10⁶ | 5824 | 50 | 5 |
| TFII-I | 1.29e10⁶ | 1000 | 96 | 4 |
| Aβ 1-42 | 1.2e10⁶ | 900 | 66 | 6 |
| ORFX5 | 6.8e10⁵ | 34 | 34 | 6 |
| SARK-CoV | | | | |

These results were obtained in just 1 month from the beginning of bait cloning (vector controls and rescreening included) and demonstrated the rapidity and precision of the system in obtaining antibodies against any protein of interest. The antibodies were then cloned in a phagemid vector to produce soluble scFv for the in vitro specificity tests ELISA and plasmon surface resonance.

The conclusion is that although the selection libraries were hyperimmune toward the respective immunizing antigens (and contained numerous antibodies against the immunizing proteins, as demonstrated in the paragraph above), they also represent an optimum source of intracellular antibodies against generic proteins. Hence, they represent SPLINT libraries and demonstrate that the spleen-derived libraries have unexpected properties required for SPLINT libraries, i.e. a size adequate for yeast cell transformation, although with a diversity wide enough to permit the isolation of antibodies against generic antigens.

The unexpectedly positive outcome obtained from a hyperimmune library, which should be enriched only for antibodies against immunizing antigens, laid the foundation for the experiment described below, i.e. the construction of a SPLINT library starting from a pool of mouse spleens that were not expressly immunized.

### SPLINT library from non-immune mouse lymphocytes (Fig. 2)

### Construction of SPLINT library

The spleen is rich in B cells and is an optimum tissue from which lymphocytes can be relatively easily extracted without needing to purify them by long and laborious methods. Mouse spleens (extracted from freshly sacrificed 3-6-month-old mice) were washed and cleaned in PBS and the lymphocytes released by pressing the spleen with a hypodermic needle and a sterile spatula. The cell suspension was then transferred to a sterile tube and left to deposit for 5 min. The supernatant was then removed and the cells washed twice in PBS and H₂O to remove the red blood cells. The cells were then immediately used for total RNA extraction. The total RNA was extracted using a kit (Rneasy Mini Kit, Qiagen) according to the manufacturer's protocols. After extraction, the RNA was quantified (about 30 µg from 10 mg of original tissue) and used for cDNA synthesis using random primers (GIBCO Brl). The synthesis of the DNA complementary to individual RNA filaments was obtained by using Superscript II Rnase H- Reverse Transcriptase enzyme (GIBCO Brl).

The following protocol was used for the production of cDNA: Random primers (3 µg/µl); 1µl (100 µg); RNA total 5 µl; dNTP mix (10mM each dATP, dGTP, dCTP, dTTP at physiologic pH) 1 µl; H₂O DEPC 5 µl. The mix is heated to 65° C for 5 min, then rapidly cooled on ice. The content of the tube is collected after brief centrifugation and First-strand buffer 5 x 4 µl, DTT 0.1M 2 µl are added. The tube content is mixed and then incubated for 2 min at 42° C, after which 1 µl (200U) of Superscript II is added and incubated for 50 min at 42° C. The reaction is inactivated at 70° C for 15 min. 1 µl (2U) of RNAseH is added to remove the residual RNA bound to the formed DNA and is left for 20 min at 37° C. The cDNA can then be used to amplify the variable chains.

To obtain cDNA from the immunoglobulin variable regions, the V regions were amplified by PCR using degenerated 5' and 3' primers (TableI) that permit the amplification of a greater number of variable chains.

The amplification primers of the variable chains are identical to those described in the section Construction of an anti-GFP immunized mouse library.

A total of 141 amplifications were performed to be certain that a major number of antibody families were expressed in the final library. The single antibody chain amplifications are shown in Figure 10.

After all the VL and the VH variable regions have been amplified from the cDNA, they are reamplified to increase the quantity of DNA for cloning and to add extra sites to each terminus of the variable region previously amplified with the degenerated primers. After the variable chains (PTVL and PTVH) have undergone pull through, the amplified products are purified with gels (see Figure 11). After purification, the PTVL and the PTVH are used for in vitro assembly. The pull through is used to insert a small polypeptide chain (called linker) that, after in vitro assembly, is in turn needed to reconstruct the single chain sequence (see Figure 12). The assembly was done in a single step rather than in two steps as described in the section on anti-GFP library cloning because a problem was noted in sequential cloning of the variable chains in the VP16mv vector. A possible explanation for this is the poor transformability of the pLinker220 vector in E. coli and the possible partial amplification of several variable chain families that make cloning in this vector particularly difficulty.

The primers used for pull through were:
VH PTL 220 BACK 
GGA GGG TCG ACC AGC GGT TCT GGG AAA CCA GGT TCC GGT GAA GGC
TCG AGC GGTA
VH PTMIC FOR 
CCA GGC CCA GCA GTG GGT TTG GGA TTG GTT TGC CGC TA
VL PTL 220 FOR
ACC GCT CGA GCC TTC ACC GGA ACC TGG TTT CCC AGA ACC GCT GGT
CGA CCC TCC
VL PTMIC BACK
   CGC TGG ATT GTT ATT ACT CGC AGC AAG CGG CGC GCA TGC C

The pull-through protocol was: VL 5*µ*l (∼100 ng); VL PTL 220 FOR 5 *µ*l; VL PTMIC BACK 5 *µ*l; MgCl₂ 2 *µ*l; dNTPs 10 *µ*l; buffer 10X 10 *µ*l; Taq polymerase 2 *µ*l; H₂O 61 *µ*l; VH 5 *µ*l (∼100 ng); VH PTL 220 BACK 5 *µ*l; VH PTMIC FOR 5 *µ*l; MgCl₂ 2 *µ*l; dNTPs 10 *µ*l; buffer 10X 10 *µ*l; Taq polymerase 2 *µ*l; H₂O 61 *µ*l.

Mineral oil (50-100*µ*l) is added to prevent evaporation in the test tube containing the reaction mixture. The tube is then placed in a thermocycler programmed as follows: 94°C -5'; (94°C -30", 60°C -30", 72°C -30") for 20 cycles; 72°C -10'; 4°C -24h. After the pull-though band for the VH and VL is isolated, the chains are purified with gel and the amplified products are used for in vitro assembly. In all, about 200 ng of DNA is used for the reaction (100 ng of PTVL and 100 ng of PTVH).

The assembly reaction primers are:
VH PT2 FOR
   TGG TGA TGG TGA GTA CTA TCC AGG CCC AGC AGT GGG TTT G
VL PT2 BACK
   TAC CTA TTG CCT ACG GCA GCC GCT GGA TTG TTA TTA CTC

The reaction is: PTVL 100 ng; PTVH 100 ng; VL PT2 BACK (100µM) 0.5 µl; VH PT2 FOR (100µM) 0.5 µl; dNTPs 10 µl; Buffer 10X 10 µl; MgCl₂ 2 µl; Taq polymerase2 µl; add H₂O to 100 µl. Mineral oil (50-100*µ*l) is added to prevent evaporation in the test tube containing the reaction mixture. The tube is then placed in a thermocycler programmed as follows: 94°C -5'; (94°C -30", 68°C -30", 72°C -30") for 8 cycles without primers and 12 cycles with primers; 72°C -10'; 4°C -24h.

After assembly of the VH and the VL, the assembled scFv library is purified with gel as described above. After purification, the DNA is digested with enzymes NheI and BssH2 in the following way: DNA mix 10µg; NheI (NEB 20 U/µl) 1.5µl; Buffer 2 (NEB)10µl BSA (10mg/ml)1µl; H₂O Q.S. (final V=100µl), and left to digest for 4 h at 37°C.

After digestion and purification with gel, the DNA is cut with BssH2: DNA mix 10µg BssH2 (NEB 20 U/µl) 1.5µl; Buffer 3 (NEB) 10µl; H₂O Q.S. (final V=100µl).

The reaction mixture is incubated at 50°C for 4 h. The digested chains are then purified with 1.5% gel using a purification kit (Gel extraction kit, Qiagen). After purification, the DNA is quantified on 1.5% agarose using spectrophotometry at a wavelength of 260 nm. The library is then ligated in the vector pLinker220: about 200ng of vector were used for this step; ligation was performed in gradient to optimize the reaction. The reaction mix is: pLinker220: 1 µl (200ng); VL-VH assembly: 2µl (250 ng); Ligase:1µl (400U/µl); Buffer: 1µl; H₂O: 5µl; The reaction mixture is placed in an Eppendorf tube and incubated overnight in a beaker filled with water at 18° C in the cold room. The next day, the reaction tube is removed from the cold room and 1 µl of ligase is added and left at room temperature for 2 h. After incubation, the reaction mix is purified with phenol-chloroform (as described above), resuspended in 10 µl of H₂O, and then electroporated in the bacterial strain.

For each electroporation, 30 µl of bacteria plus 1 µl of ligase mix is used (max. 100 ng per electroporation). After transformation, the bacteria are resuspended in 1 ml of SOC medium + 10 nM MgCl2 and incubated for 1 h at 37° C, after which they are seeded on LB+ ampicillin plates. The next day, the efficiency of the transformation is evaluated. The library thus obtained was estimated to be about 4.10⁵ CFU.

At this point, the SPLINT library was used for the control test such as PCR-fingerprinting to test library diversity, antibody sequences taken randomly, western blot analysis to check the expression of the individual chains in yeast and screening on a panel of known antigens.

The results demonstrate that even from this initial SPLINT library intracellular antibodies against a wide variety of antigens can be isolated.

### Description and list of antigens (tableII):

SPLINT was selected against the following antigens: two domains of Shc (Fig.13, 15), K-RAS (Fig.14, 15), Syk (Fig.16), β4-thymosin ubiquitous, β4-thymosin lymphoid (Fig.17, 18), amyloid β2-42, gephyrin, TFII-I and X5 ORF SARS-CoV proteins.

### Construction of the baits:

**SH2-Shc/MIC-BD1:** the DNA fragment SH2-Shc was PCR amplified from pGEX4T1-p66Shc and cloned **between** EcoRI/BamHI restriction sites of pMIC-BD1 plasmid.

**CH2-Shc/MIC-BD1:** the DNA fragment CH2-Shc was PCR amplified from pGEX4T1-p66Shc and cloned between EcoRI/BamHI restriction sites of pMIC-BD1 plasmid.

**Syk/MIC-BD2:** the Syk EcoRI/BamHI fragment from Syk/BTM116 (Visintin et al., 1999) was subcloned between EcoRI/BamHI restriction sites of pMIC-BD2 plasmid.

**K-RAS/MIC-BD1** and **K-RAS/MIC-BD2:** K-RAS BamHI/PstI from K-RAS/BTM116 (Visintin et al., 1999) was subloned between BamHI/PstI restriction sites of pMIC-BD1 and pMIC-BD2 respectively.

**LTβ4/MIC-BD1:** the DNA fragment LTβ4 was amplified from LTβp4/pCI-neo vector and cloned between the EcoRI/BamHI restriction sites of pMIC-BD1 plasmid. **UTβ4/MIC-BD1:** the DNA fragment LTβ4 was amplified from UTβ4/pCI-neo vector and cloned between the EcoRI/BamHI restriction sites of pMIC-BD1 plasmid. **Gephyrin/MIC-BD1:** the DNA fragment of the 153-348 domain of gephyrin protein was amplified and cloned between the EcoRI/BamHI restriction sites of pMIC-BD1 plasmid. Before starting selection of SPLINT library all baits were tested for transactivation of reporter genes as described (Visintin and Cattaneo, 2001). All baits resulted to be non transactivating and thus used for the selections.

**Aβ1-42/MIC-BD1:** the DNA fragment of β amyloid 1-42 domain was synthesize by primers annealing and then cloned into BamHI/PstI restriction sites of pMIC-BD1 plasmid.

**TFII-I 1-397/MIC-BD1:** the DNA fragment of the 1-397 domain of TFII-I transcription factor was amplified from human TFII-I cDNA and cloned into Bam HI restriction site of pMIC-BD1 plasmid)

**X5 ORF SARS-CoV/MIC-BD1:** the DNA fragment of the X5 ORF of SARS-Coronavirus was synthesized by primers annealing and then cloned into EcoRI/SalI restriction sites of pMIC-BD1 plasmid.

### Selection of scFv intracellular antibodies with different specificities by IACT.

To verify whether we could select from this library intracellular antibodies against signalling proteins, and other intracellular proteins, we screened the library against a panel of different baits (Table II). These include, two domains of Shc, K-RAS, Syk, β4-thymosin ubiquitous, β4-thymosin lymphoid, amyloid β1-42, gephyrin, TFII-I and X5 ORF SARS-CoV proteins.

**Table II.** Panel of different baits used for SPLINT-2HY selection.
- Human p66Shc CH2 domain (CH2-Shc)
- Human p66Shc SH2 domain (SH2-Shc)
- Human K-RAS
- Human Syk
- Mouse β4-Thymosin ubiquitous (TUβ4)
- Mouse β4-Thymosin lymphoid (TLβ4)
- Human Amyloid β1-42
- Rat Gephyrin
- Human TFII-I
- X5 ORF SARS-CoV

The baits chosen for the validation of the SPLINT library include proteins of different species, of viral origin and of very different function and cellular location:
Two different domains (CH2 and SH2) of human p66^{Shc} adaptor protein (Ventura et al., 2002), which contains a unique amino-terminal proline-rich region (CH2) an NH2-terminal phosphotyrosine binding domain (PTB) followed by a collagen homology domain (CH1) and a COOH-terminal SH2 domain. By virtue of its PTB and SH2 domains, Shc proteins link receptor and nonreceptor tyrosine kinase activation to downstream cytoplasmic can bind to activated receptors that have been tyrosine-phosphorylated. This protein is implicated in pathways activated by environmental stresses and regulation of life-span.

Human K-Ras is a monomeric membrane-localized G protein of 21 kd that functions as a molecular or nuclear events. The ras-signaling pathway is an attractive target for anticancer therapy because of its important role in carcinogenesis.

Syk protein-tyrosine kinase has been implicated in a variety of hematopoietic cell responses, in particular immunoreceptor signaling events that mediate diverse cellular responses including proliferation, differentiation, and phagocytosis. Syk appears also to play a general physiological function in a wide variety of cells including neuronal cells in which it is suggested to play an important role in signaling steps to neurite extension (Yanagi et al., 2001).

Thymosin β4 encodes both an ubiquitous actin-binding protein (UTβ4) with demonstrated capacity to inhibit neutrophilic infiltration, and a splice-variant limited to lymphoid tissue (LTβ4) that was shown to be activation-responsive expressed by dendritic epidermal T cells, and other intraepithelial lymphocyte, to down-regulate local inflammation (Girardi, 2003).

Gephyrin is a 93 kDa protein that copurified with the glycine receptor (GlyR,) and was found to be localized at the postsynaptic side of glycinergic synapses (Prior et al., 1992) (Sassoe-Pognetto and Fritschy, 2000; Craig and Lichtman, 2001).

The major constituent of senile plaques in Alzheimer's disease is a 42-aa peptide, referred to as beta-amyloid (Abeta). Abeta is generated from a family of differentially spliced, type-1 transmembrane domain (TM)-containing proteins, called APP, by endoproteolytic processing. The major, relatively ubiquitous pathway of APP metabolism in cell culture involves cleavage by alpha-secretase, which cleaves within the Abeta sequence, thus precluding Abeta formation and deposition. Amyloid beta-peptide (1-42) (Butterfield, 2002) may be central to the pathogenesis of AD (Sinha and Lieberburg, 1999).

TFII-I is an inducible multifunctional transcription factor that is activated in response to a variety of extracellular signals and translocates to the nucleus to turn on signal-induced genes (Roy, 2001).

Severe acute respiratory syndrome (SARS) is a life-threatening form of atypical pneumonia. SARS-CoV genome is ∼29.7 kb long and contains 14 open reading frames (ORFs) flanked by 5' and 3'-untransleted regions. Coronaviruses encode a number of non-stuctural proteins. These non-structural proteins, which vary widely among the different coronavirus species, are of unknown function and seems to be dispensable for virus replication (Rota et al., 2003; Snijder et al., 2003; Zeng et al., 2003).

DNA sequences coding for each of these proteins, fused to lexA protein to create antigen-specific baits, were introduced by transformation into yeast cells expressing the SPLINT library and IACT selection was performed as described (Visintin et al., 2002). It should be noted that no manipulation whatsoever of the corresponding protein was performed. Double trasformants were selected for histidine prototropy and lacZ activity. In the search for antigen-binding scFv fragments, about 10-20 His+/lacZ+ different clones for each antigen were analyzed in a second two-hybrid screening, except for UTβ4, LTβ4, Ab1-42, Gephyrin and TFII-I (TableII) whose screening was more exhaustive (more than 90 His+/lacZ+ different clones). Specifically binding clones were obtained in each case, demonstrated that bait-specific single chain antibodies could be expressed in the cytoplasm and in the nucleus of yeast cells (Table III). Subsequent secondary screening confirmed that true positives coild be identified that interact specifically with the original bait, but not with other lexA fusions (lexA-lamin). 8 clones for CH2-Shc, 5 clones for SH2-Shc, 2 for K-RAS, 6 for Syk, 11 for LTβ4, 2 for UTβ4and 6 for gephyrin, 6 for Aβ 1-42,4 for TFII-I and 6 for X5 ORF SARS-CoV were identified that interact with their antigens but did not interact with lexA-Lamin.

A subset of antibody fragments obtained from LTβ4 screening were also analyzed for binding to UTβ4 (Fig.18).

The lymphoid-specific thymosin encodes for a six-residue NH2-terminal extension relative to the ubiquitous form of thymosin. In order to verify if it would be possible to isolate scFv specific only for the lymphoid form we tested five antibody fragments obtained from the LTβ4 screening that were shown to have the most different aminoacid sequences among the scFv isolated, for their ability to bind UTβ4. All the scFvs tested result to be highly specific for the lymphoid form and failed to bind the ubiquitous one (Fig.18). The parallel selection of SPLINT on UTβ4 resulted to be very stringent; very few different fingerprinting-patterns were found among 96 analyzed clones. Moreover it was shown that the selection of SPLINT against the UTβ4 bait was able to isolate scFvs whose aminoacid sequences correspond to some scFvs previously isolated against LTβ4, thus indicating that the selection of SPLINT against LTβ4 was exhaustive enough for fishing out specific antibodies against both forms of the thymosin protein.

In conclusion, the results obtained confirmed that indeed the SPLINT library constructed represents a good source for validated intracellular antibodies, against a wide variety of different proteins. The procedure to isolate such antibodies, which depends directly from the format of the llibrary, requires no manipulation whatsoever of the target protein, thus obviating the need for protein expression and purification, that represents a significant experimental bottleneck for increasing the throughput of antibody isolation. The procedure described, that depends on the construction of the SPLINT library, allows isolating stable validated antibodies directly from gene sequences.

### Improving the stringency of selections.

The results summarized in Table III shows that for each target protein chosen, a number of different antigen-specific antibodies could be isolated directly from gene sequences. The procedure has been experimentally optimized and refined and is very robust, rapid and efficient.

In order to fish out very strong binders and to improve the stringency of selections, if required, we also tested our SPLINT library by using a modified bait, fused with a mutant lexA protein. The bacterial LexA protein of pMIC-BD1 vector was modified (mLexA) to abolish its intrinsic NLS, as previously described (Rhee et al., 2000) (Fig.7). In this way, only the antibody/antigen complex formed in the cytoplasm has the information to be targeted to the nucleus. To check whether it should be possible to screen SPLINT also with antigen expressed only in the yeast cytoplasm (as in the case with the pMIC-BD2 vector) we chose the protein K-RAS as target antigen. The choice of RAS was also due to the apparent difficulty of isolating intracellular antibodies in the standard selection procedure (Table III). In this modified selection procedure, the interaction between the antigen and the scFv must occur in the cytoplasm, because the antigen is expressed in yeast cells without the intrinsic nuclear localization signal of lexA. ScFv and antigen interact in the cell cytoplasm before the complex is translocated into the nucleus and activates transcription. We tried in parallel the selection of K-RAS as lexA-bait fusion protein with or without nuclear localization signal. In both cases we obtained the same number of functional intracellular antibodies.

This indicates that the technology was strong enough to allow the selection of scFv with such an affinity to bring the antibody-antigen complex into the nucleus and to activate the transcription of reporter genes.

As a general comment, the selection with K-RAS either with or without NLS, gave very few positive clones if compared with other antigens (TableII)). It should be noted that very few positive clones were also obtained in other reported IACT selection strategies when RAS was used as a bait (Tanaka and Rabbitts, 2003). This might indicate that the antigen has difficulty entering into the nucleus, and this difficulty interferes with the normal selection procedure. Alternatively, interference with the endogenous RAS protein might explain this low number of colonies obtained from this selection.

In conclusion we established that functional antigen-specific ICAbs were selected from the natural SPLINT library against a wide spectrum of very different protein antigens, including viral proteins. It should be noted that the optimized selection procedure, ensure that the selected antibodies are all functional also when expressed in mammalian cells. Indeed IACT selection predicts the property of being a functional intrabody in all cases (Visintin et al., 2002) (Tse et al., 2002). This was confirmed for a number of the ICAbs selected from SPLINT, which showed good solubility and half life when expressed in mammalian cells (data not shown). Thus, SPLINT libraries represent a rich and good source for antibodies to function in the intracellular environment.

### BIG SPLINT: Increasing the size of SPLINT

Library size is a critical variable for the isolation of antibody fragments. However, the true diversity of a library is not easily determinable; this in fact depends not only on the number of independent counted clones but also on the number of clones that actually express scFv proteins.

Notwithstanding the fact that we demonstrated here that it is possible to select functional intracellular antibodies from a SPLINT library of 10⁶-10⁷ clones, functional ICAbs could be more easily directly selected making a larger SPLINT library. This has been accomplished by directly scaling up (i.e. repetition a number of times of the procedure described above) or by recombination methods (figure 4 a-b), as has been done in bacteria (Sblattero and Bradbury, 2000) and in yeast for making large antibody libraries of surface displayed Fab fragments (Feldhaus et al., 2003). The BIG-SPLINT library obtained in this way allows not only to isolate stable intracellular antibodies but also to increase the number of possible antibody candidates for intracellular expression.

### Consensus sequence framework-based library

A further refinement of the SPLINT library will be carried out with the study of scFv sequences isolated by IACT.

It has been noted that the set of antibodies selected with these technologies is characterized by a number of highly conservative residues. These critical residues represent a sort of trademark that identifies those antibodies capable of being expressed in the intracellular environment by a multitude of scFv which are otherwise unable to function there. IACT technology combined with SPLINT is also able to select the scFv that possess an optimum sequence very similar to a consensus sequence present in the database of immunoglobulins present in nature. As the database of intracellular antibodies selected with this technology grows, the number of conserved residues will decrease. It will therefore be possible to achieve a final generic framework on which an antibody library for intracellular use may be engineered. This will allow the creation of a library compatible with the low transformability of yeast and, more generally, a phagmid or phagic library of highly stable scFv that are functional for expression in any cellular or noncellular environment. It can be concluded that both IACT and SPLINT are two technologies with the capability to surpass their intrinsic potential and to thus create ever more sophisticated and validated libraries.

### SPLINT libraries as a source of super stable antibodies, that can be isolated rapidly, directly from gene sequences.

It has been demonstrated above that a SPLINT library, as described above, represents a good generic source of intracellular antibodies, that is of antibodies/antibody domains that are able to bind any antigen of interest under conditions of intracellular expression. In light of the fact that these validated intracellular antibodies fold in the intracellular environment despite the fact that they fail to form the intrachain disulfide bond that links the two cysteine residues present in all known antibody variable regions (Visintin et al 1999; Biocca et al 1995), and in light of the fact that this disulfide bond contributes approximately 4.5 kCal/mol to the folding stability of an antibody domain (Proba et al., 1997), it can be concluded that antibodies generated through the IACT procedure from SPLINT libraries are much more stable than average antibodies in any given repertoire. In fact, when SPLINT derived antibodies are expressed under the normal oxidising conditions, thus allowing their intrachain disulfide bonds to form, their folding stability under experimentally controlled denaturing conditions (such as urea, guanidinium or thermal denaturation) is much greater than that of average antibodies (data not shown). Thus, SPLINT libraries allow isolating antibodies of superior stability. This property, together with the possibility of isolating antibodies directly from gene sequences, makes the SPLINT libraries the tool of choice to solve the bottleneck for the high throughput generation of stable antibodies, in applications such as antibody arrays, to be used as advanced diagnostic tools and for protein expression profiling in proteomics.

### Protein-protein interaction targeted antibodies from SPLINT libraries:

The unexpected demonstration that a SPLINT library as described above represents a good source for intracellular antibodies represents the essential prerequisite for the final development of this invention, namely the exploitation of the SPLINT library, after suitable engineering, for the isolation of antibodies that are intrinsically neutralizing. In fact, the antibodies isolated from SPLINT are not necessarily endowed with the property of inhibiting the biological function of their target protein. Indeed, the neutralization of protein function (PKO) is an added-on property, that has to be engineered or implemented on an ad hoc case-by case basis, with strategies such as, for instance, traffic diversion.

These methods are not general, and therefore only a subset of antibodies will be useable to neutralize protein function, notwithstanding their in built property of being validated intracellular antibodies.

We describe in the following the use of SPLINT libraries to generate rapidly (directly from gene sequences) antibodies that are intrinsically endowed with the ability to interfere with a given protein-protein interaction. These will be intrinsically neutralizing antibodies.

Protein interactions play a crucial role in the discovery of diverse biological functions. The interpretation of genes sequenced from the human genome is leading researchers to develop new technologies for the interpretation of unpredictable functions of yet undiscovered genes. Diverse interactomes have been explored (protein interactions in a single organism). It has been seen that the two-hybrid system can be used to create a vast network of connections that can be further validated by other biotechnology system such as 2D gels, mass spectrometry, etc. The currently available approaches to studying protein function are limited and poorly generic. In contrast, with the methodology adopted for DNA study, the proteins are so variable that the study of the genome by protein interaction cannot be undertaken using a single methodology.

For this reason, researchers are examining whether antibody intracellular technology may offer an approach suitable for various fields of functional genomics.

For example, one can imagine constructing an ad hoc library of antibody fragments selected on a certain proteome (Fig. 6). The technology would entail a first step in which a cDNA library is created from a cell type, a tissue, a virus or a bacterium, etc. The library would then be directly cloned in the two-hybrid vector fused to a protein that binds DNA (e.g. pMIC-BD2) and tested against a SPLINT library in vivo. After positive clones have been obtained, the scFv involved in an interaction are isolated and cloned (in pools in a library format) in a eukaryotic expression vector (e.g. scFvexpress). After the new sub-library is created, the scFv can be expressed in the cell type, tissue, virus or bacterium from which the cDNA library was originally constructed. Once the library is introduced, a protein extract is prepared and used in immunoprecipitation experiments. The immunoprecipitated proteins are separated with a bidirectional gel (2D gel) and the isolated proteins can then be analyzed by mass spectrometry. With this technology (IntrAP), antibodies directed against the entire cell proteome can be obtained in a single step.

Another possible consideration is the interactions and networks of genetic regulation at the level of a complete genome. In this way, it would be possible to conceive of the genome as a complex of functions expressed by proteins programmed to stabilize specific interactions. In this light, the total number of genes in an organism would appear less important than the complete repertory of interactions that may be potentially encoded by the same genome (interactoma). For this reason, one can conceive of constructing a subset of scFv libraries (similar to that described in the paragraph above) by isolating only the scFv that recognize protein modules or peptide sequences recognized as being a functional part of an interaction. The library could then be used to directly interfere in a known interactome through a methodology based on the three-hybrid system (Fig. 19). The 3HY-SPLINT strategy draws on various publications from the field of three-hybrid systems (Vidal M, et al. 1996; Huang J, et al. 1997; Leanna CA, et al. 1996; Shi HM, et al. 1996; Vidal M, et al. 1999).

The strategy uses a genetic selection in which the dissociation of an interaction becomes a selective advantage for the yeast strain used for this purpose. To reiterate the concept, the binding between protein X-DNA-BD and protein Y-AD is lethal for a yeast cell because in this system a toxic gene is used as a reporter gene. The dissociation by an antibody that binds one of the two interaction domains between X and Y confers a selective advantage that may be conveniently identified.

In our 3-HY system, the expression of the tetracycline repressor represses the expression of the HIS3 gene. Under these conditions, the interaction between X and Y confers an auxotrophic phenotype for histidine. When an antibody disrupts the interaction between X and Y, the TetR repressor is not expressed and the strain can survive also in the absence of histidine since gene HIS3 transcription is no longer repressed.

A novelty of our system is the use of two vectors instead of the classical set of three vectors to express either the two interacting proteins and the library of neutralizing antibodies. This allow to simplify the methodology, avoiding the difficult engineering of three vectors with different nutritional markers and different antibiotic resistance and the need of a particular yeast strain to be also engineered in which more than 3 genes must be knocked-out. Moreover a highly optimized protocols for the transformation of the yeast cells should be assessed thus resulting in very poor reproducibility of the experiments. Our three hybrid methodology allow to maintain the current methodology of IAC technology as little modified and thus accessible to non specialized researchers.

The results obtained so far are:

### STEP 1:lexA-p65 and VP16-p65 interact in two-hybrid system

### Construction of the bait and the prey:

**p65/MIC-BD1:** the DNA fragment of p65 protein member of NF-kB/Rel family was amplified from pRSV NF-kB relA (p65) plasmid and cloned between BamHI/SalI restriction sites of pMIC-BD1.

**p65/VP16c-t:** the DNA fragment of p65 was amplified from pRSV NF-kB relA (p65) plasmid and cloned between BamHI/NotI restriction sites of VP16c-t.

**Intracellular interaction of lexA-p65 and VP16-p65 fusion proteins:** L40 yeast cells were cotransfected with bait and prey vectors by using lithium acetate transformation protocol. Positive interaction was verified by using auxotrophic markers for both plasmids and for histidine prototropy. Histidine-positive colonies and controls were lysed in liquid nitrogen and assayed for b-gal activity on filters as described (Visintin and Cattaneo, 2001).

### STEP 2: lexA-p65 and VP16-p65 interact in two-hybrid system when cloned in pBiDi3HYlexA-VP16 vector

**Construction of pBiDi3HY vector:** to construct pBiDi3HY vector 2 vectors were used. The pESC-TRP vector was PvuII cutted to isolate the bidirectional promoters Gall-Gal10. The pMIC-BD1 vector was SphI cut and DNA polymerase I, Large (Klenow) fragment was subsequently used to remove the 3' overhangs to form blunt ends. The vector was also dephosphorylated with calf intestinal alkaline phosphatase to prevent recircularization of cloning vector. Cloning of Gal1-Gal10 PvuII digested into pMIC-BD1 SphI cut and blunt generated pBiDi3HY.

**Construction of pBiDi3HYlexA-VP16 vector:** the DNA fragment VP16 was amplified from VP16* vector (Hollenberg et al., 1995) and cloned between the BamHI/ApaI of pBiDi3HY plasmid.

The DNA fragment lexA was amplified from pMIC-BD and cloned between the EcoRI/SpeI of pBiDi3HY-VP16 vector. The final vector pBiDi3HY-lexA-VP16 was sequenced and the expression of lexA and VP16 proteins was verified by western blot analysis of yeast crude extract after induction of promoters with galactose 20% (see figure 20).

**Construction of pBiDi3HYlexAp65-VP16p65 vector:** the member of NF-kB/Rel family p65 protein, was cloned as C-terminal fusion protein with lexA and VP16 domains. The DNA fragment p65 was amplified from pRSV NF-kB relA (p65) plasmid (gently provided by Dr. Francesca Demarchi) and cloned into SpeI to produce lexA-p65 fusion protein and between ApaI/SalI to produce VP16-p65 fusion protein. The expression of both p65 fusion proteins were verified by western blot analysis and sequences (see figure 21).

All clones were sequenced, using the Epicentre Sequitherm Excel II kit (Alsbyte, Mill Valley, CA), with a Li-Cor 4000L automatic sequencer (Lincoln, NE).

**Construction of pSPLINT vector** (Fig.20): For construction of plasmid pSPLINT, the following cloning strategy was applied. A PCR Vk and VH cloned with 220 linker from pLINKER220 was performed by using these oligonucleotides: sense 5'-CATCATCATAAGCTTATTTAGGTGACACTA-3' and antisense 5'ACCCGGGGATCCCTAATGGTGATGGTGATGGTGAGTACTATCCAGGCCCAG CAGTGGGTTTGGGATTGGTTTGCCGACCTTCCGCTTCTT-3'. The resulting PCR product was digested with HindIII/BamHI and ligated to HindIII/BamHI -digested vector VP16* (Hollenberg et al., 1995) to give pSPLINT. The activation domain VP16 was removed while maintaining both nuclear localization signals between scFv. A tag signal SV5 was added at the C-terminal of scFv linker.

### Construction of SPLINT-3HY library.

A pool of scFv assembled from SPLINT library was also cloned into BssHII/NheI-digested pSPLINT to obtain SPLINT/pSPLINT library of 1.5∼10⁵ different clones in E.coli.

### STEP 3: lexA-p65 and VP16-p65 interaction can be abrogated by a neutralizing antibody in a SPLINT-3HY selection strategy

**Co-transformation of pBiDi3HYlexAp65-VP16p65 vector with SPLINT-3HY library in YI596 yeast strain.**

The genotype of *Saccharomyces cerevisiae* reporter strain YI596 is MATa/α, his3Δ200/his3Δ200, trp1-901 leu 2-3, 112/leu2-3, 112ade2/ade2, URA::lexAOp)₈-TetRLYS2::ADH(Tet Op)₂-HIS.

The constructed system is a positive selection method designed to identify antibody domains that disrupt protein-protein interaction in vivo. The proteins of interest (p65 homodimers) are expressed as fusion with the lexA DNA binding domain and the transcriptional activator, VP16 under the control of the inducible bidirectional promoters Gal1-Gal10. Interaction between lexA-p65 and VP16-p65 leads the expression of the tetracycline repressor protein (TetR) which prevents transcription of the HIS3 reporter gene leading to the inability of yeast cells to grow on media lacking histidine. Disruption of homodimerization of p65 protein by a neutralizing antibody restores the ability to grow on such media. LexA-p65 fusion protein was shown to display activation capability. In order to block histidine production suppressing background growth on media lacking histidine a modulation of the system with tetracycline/3-AT was performed. After the identification of growth conditions of lexAp65-VP16p65 homodimer this construct was used for the selection of SPLINT library in order to identify the neutralizing antibody that disrupt p65 homodimer formation in vivo.

SPLINT library is introduced into YI595 strain by a modified protocol of yeast transformation (Visintin and Cattaneo, 2001). Each transformant presumably contains an individual library plasmid encoding a scFv in addition to the p65 homodimer proteins. Cells containing the plasmid encoding a scFv that bind one or both p65 interacting proteins will be selected on SG-Raf-WHULK. The transformation procedures must be carefully followed to ensure a high transformation efficiency.

### SPLINT-3HY transformation

The following transformation protocol is a modification of published method (Visintin and Cattaneo, 2001).

Materials: 500 µg SPLINT/pSPLINT library; 150 ml YC-UKW; 21 YPAD; 11YPA; 1.51 YC-UKWL + 10 YC-UKWL plates (100mm); 11YC-WHULK ; 11SG-Raf-WHULK + 3AT 25mM (see recipe below); 20 SG-Raf-WHULK + 3AT 25mM plates 100 ml 10X TE; 20 ml 10X LiAc; 1 ml of 10 mg/ml denatured salmon sperm; 150 ml 50% PEG 4000; 20 ml DMSO.

### Procedure:

**Day1:** grow YI595yeast containing p65 homodimer plasmid in YC-UKW O/N
**Day2:** inoculate 100 ml of YC-UKW with an aliquot of the overnight culture in order to find a dilution that places the 100 ml culture to logarithmic phase the next day
**Day3:** transfer enough overnight culture in 11 of prewarmed to 30°C YPAD to produce an OD₆₀₀= 0.3. Grow at 30°C for 3 hours. Centrifuge the cells at 1500 X g for 5 min at room temperature. Wash pellet in 500 ml of 1X TE then centrifuge again the cells at 1500 X g for 5 min at room temperature. Resuspend pellet in 20 ml 1X LiAc, 0.5X TE and transfer to a new flask. Add 500 µg DNA library and 1 ml denatured salmon sperm. Add 140 ml of 1X LiAc, 40% PEG 3350, 1X TE; mix and incubate for 30 min at 30°C with gently shaking. Add 17.6 ml of DMSO; swirl to mix. Heat shock for 10 minutes at 42°C in a water bath swirl occasionally to mix. Rapidly cool at room temperature cells in a water bath diluting with 400 ml YPA. Pellet cells by centrifugation and wash with 500 ml YPA. After centrifugation resuspend pellet in 11 of prewarmed YPAD. Incubate at for 1 hour at 30°C with gently shaking. Pellet cells from 1 ml: resuspend in 1 ml YC-UKWL; spread 100 µl of a 1:1000, 1:100, 1: 10 dilutions for transformation efficiency controls. Pellet cells from the remaining culture. Wash pellet with 500 ml YC-UWL. Resuspend in 11 of prewarmed YC-UWL and incubate O/N at 30°C with gently shaking.
**Day4:** pellet cells and wash with 500 ml of YC-WHULK. Pellet cells and wash with 500 ml of SG-Raf-WHULK + 3AT 25mM. Resuspend final pellet in 10 ml of SG-Raf-WHULK + 3AT 25mM. Spread dilutions of the total on YC-UKWL plates to compare to the number of primary transformants Spread the remaining transformation suspension on SG-Raf-WHULK + 3AT 25mM plates.

### Synthetic Galactose Minimal Medium (SG dropout media)

**1. YNB w/o aa & (NH₄)₂SO₄:** 1.2 g yeast nitrogen base, w/o amino acids and ammonium sulfate (Difco # 0335-15-9); 20 g bacto-agar; Add H₂O to 800 ml. Autoclave 121°C for 15 min.
**2.** Salts: 5.4g NaOH; 10g succinic acid (Sigma # S-7501); 5g ammonium sulfate (Sigma # A-3920).
**3. Gal-Raf:** 20g galactose; 10g raffinose
   Add H₂O to 100 ml and dissolve all components one by one. Add galactose and raffinose and H₂O to make a final volume of 150 ml.
**4. amino acids MIX:** 5.8 g NaOH; 1 g Adenine hemisulfate salts; 1 g L-Arginine HCl (Sigma # A-5131); 1 g L-Cysteine (Sigma # C-8277); 1 g L-Threonine (Sigma # T-8625); 0.5 g L-Aspartic acid (Sigma # A-4534); 0.5 g L-Isoleucine (Sigma # I-7383) 0.5 g L-Methionine (Sigma # M-9625); 0.5 g L-Phenylalanine (Sigma # P-5030) 0.5 g L-Proline (Sigma # P-4655) ; 0.5 g L-Serine (Sigma # S-5511). Dissolve in 80 ml H₂O
**5. L-Tyrosine:** 0.5 g L-Tyrosine (Sigma # T-3754); 0.2 g NaOH; Dissolve in 10 ml by heating.
**6. Omitted amino acid solutions:**
   L-Histidine (Sigma # H-9511): 5 g/l H₂O
   **Uracil** (Sigma # U-0750) : 10 g/l H₂O (+2 pellets NaOH)
   **L-Leucine** (Sigma # L-1512): 10 g/l H₂O
   **L-Lysine HCl** (Sigma # L-1262): 10 g/l H₂O
   **L-Tryptophan** (Sigma # T-0254): 10g/1H₂O

Add to aa MIX (4.) the L-Tyrosine solution (5.) and H₂O to make a final volume of 100 ml. Filter-sterilize, aliquot and store at -20°C for up to 1 year. Filter-sterilize and aliquot individually omitted amino acids (aa) solutions (H, W, L, K, U) and store at -20°C for up to 1 year. Before preparing SG-Raf plates or media by mixing appropriate solutions, media must be adjusted to pH 5.8 and sterilized by filtering salts + aa final mix.

### Results of SPLINT-2HY selections

It was already demonstrated that IACT is a genetic selection method that allows the isolation of soluble intrabodies directly from antigen gene sequences (Visintin et al., 2002).

The major goal of IAC technology was to develop intracellular one-pot scFv libraries of sufficient size and diversity to facilitate isolation of antibodies of every conceivable specificity, including antibodies with high solubility, stability and good affinity for intracellular expression. An important determinant of the composition and diversity of this *alternative in vitro* immune system is the source of antibody genes used as building blocks to construct the library. In order to explore the probability of isolating intrabodies directly from primary mice antibody repertoire we have engineered a single pot antibody library in an activation domain vector and tested it in a two-hybrid selection strategy by using a wide panel of different antigens.

The results obtained from all the different selections have indicated that SPLINT library is a good source of antibody fragments. In fact, it was possible to select several scFv against each antigen challenged with the IAC technology. For some target antigens it was possible also to isolate several antibodies thus indicating that although the library is not very complex the diversity obtained is still sufficient to acquire immunological reagents that recognize particular antigens as for example, proteins that are highly conserved between species.

We have demonstrated that a small library which has proved to be enriched in functional antibodies can still provide a good source of antibody for ectopic expression because the method for selecting the antibody fragments act as a filtering funnel. SPLINT has demonstrated to contain a source of stable superior antibodies, naturally present in naïve repertoires but difficult to find if the methodology used is not specialized.

In the original format, SPLINT-2HY guarantee that the antibodies selected are good intracellular binder but cannot assure for neutralizing antibodies.

In order to generate a method that easily provide neutralizing antibodies, IAC technology and SPLINT library were modified to generate a novel system named SPLINT-3HY.

### Results of the 3-HY selections

In the original format of IAC technology, an antigen-antibody interaction brings the two hybrid proteins together and results in the activation of the reporter genes.

In the new generation of the technology, the three hybrid version, we can identify antibody domains that dissociate or abrogate defined macromolecular interactions.

In order to achieve the proof of principle of this technology we used SPLINT-3HY to undertake the de novo selection of scFv antibody fragments against NF-kB RelA p65 protein.

Mammalian NF-kB is a family of structurally related proteins, including p65/Re1A, which form homo- and heterodimers. Regulation of gene expression by NF-kB/rel family of transcription factors is controlled mainly by the inhibitory IkB proteins. The active complex of NF-kB is composed of homodimers and heterodimers of p50, RelA, RelB, and c-Rel. These complexes are sequestered in the cytoplas by IkBs. Extracellular signal-induced phosphorylation and subsequent degradation of IkBs is essential for nuclear translocation of NF-kB. NF-kB binds to the recognition elements in the promoter region of target genes and activate transcription. IkB proteins determine the duration of transactivation by NF-kB. IkBa is involved in transient activation of NF-kB because it is degraded rapidly upon stimulation and is resynthesized by activated NF-kB. Most of the inducers of NF-kB cause degradation of IkBα. The newly synthesized IkBα sequesters NF-kB in the cytoplasm and determines the signal.

p65 has a bipartite structure-a DNA-binding rel hologous region and a C-terminal transcription activation domain. These two functional regions are linked by a segment that contains a nuclear transport signal (Chen et al., 2000).

IκB degradation activates this PKAc that phosphorylates the NF-κB p65 subunit at Ser 276 rendering NF-κB that enters the nucleus transcriptionally active through recruitment of CREB binding protein (CBP)/p300 (Zhong et al., 1997; Zhong et al., 1998).

It is believed that p50 homodimers compete for κB site binding with NF-κB complexes containing subunits such as p65 that have transactivation domains; however, this is unlikely to be their primary mechanism of repression, as their affinity for classical κB sites is lower than that of heterodimers such as p50:p65 (Kunsch et al., 1992). One possible mechanism that has been demonstrated for other repressive DNA binding proteins (Glass and Rosenfeld, 2000) is that nuclear p50 may interact with separate inhibitory proteins that recruit corepressor complexes containing HDACs to gene promoters.

The homodimer of p65 protein was cotransformed into yeast cells YI595 with SPLINT/pSPLINT scFv library following the large scale procedure described in (Visintin and Cattaneo, 2001). The co-transformed library was spread onto SG-Raf-WHULK +25mM plates to allow the activation of Gal 1-10 promoters and the selection of the plasmid encoding a scFv that bind one or both p65 interacting proteins due to repression of tetracycline repressor that restores the transcription of His3 gene. The high efficiency of transformation (2.6e10⁷) obtained in this experiment ensured that the yield of primary transformants was three times the complexity of the library, thus the library complexity ensures that 95% of the clones represented in the library have been examined (Clarke and Carbon, 1976). After three days 4 clones that grew on -HIS plates were picked and grown on a fresh -HIS plates grid for a second verification of the transcriptional activation of his gene. After 4 days only 2 clones out of the initial 4 clones selected in the first round of selection were able to be rescued on SG-Raf-WHULK +25mM plates. Both clones from secondary screening were picked and grown for the isolation of DNA. Isolated scFv/pSPLINT plasmids were analysed by PCR-BstNI fingerprinting and sequences. The conclusion from this first part of the experiments is that both scFvs selected from SPLINT-3HY were able to abrogate the interaction between p65-p65 dimer. In order to verify that the two isolated scFvs were also able to recognise p65 as a monomer both antibody fragments were cloned in fusion with VP16 into pLINKER220 vector.

### STEP 4: are anti-p65 isolated scFv binders for p65 monomers?

**Construction of anti-p65 (A1 and A2)/pLinker220 plasmid.** Anti-p65 scFvs fragments were subcloned into pLinker220 between BssHII-NheI restriction sites.

As shown in figure 23 clone A2 display a positive interaction with p65 monomer, thus indicating its specificity with the target protein.

On the contrary, clone A1 was only able to recognized DNA-binding domain lexA. Although A1 clone result to be specific for lexA protein the result obtained indicates that scFvAl is probably able to inhibit p65-binding domain or p65-DNA-binding domain in a non conventional manner or by steric hindrance.

scFv A2 was subsequently cloned into mammalian expression vector scFvexcyto-SV5.

### STEP 5: in vivo mammalian cells studies

**Construction of anti-p65A2/scFvexcyto-SV5 plasmid.** Anti-p65 scFvs fragments were subcloned into scFvexcyto-SV5 between BssHII-NheI restriction sites.

Anti-p65 A2 was expressed in HEK cell line al leaderless cytoplasmic protein. Transfected cells display a diffuse intracellular staining, typical of soluble cytoplasmic protein (see figure 24).

To assess whether anti-p65 A2 inhibition interferes with the physical association between p65 dimer and p50, a coimmunoprecipitation experiment was carried out (Fig. 25). Fig. 25 shows controls (lane 1-3-5- p65 and p50 expressed by HEK in non transfected (c), transfected with anti-p65 (lane 3) and transfected with an irrelevant scFv (lane 5) immunostained with anti-p65 (upper part of the picture) and immunostained with an anti-p50 (lower part). Lanes 4 show the coimmunoprecipitated band corresponding to the p65 monomer (upper part) and p50 protein (lower part). The conclusion for this experiment is that anti-p65 is able to recognize endogenous p65 in HEK cell lines. In this experiment also p50 protein was pulled down with p65 thus indicating that the interaction between anti-p65 and p65 does not interfere with the natural binding sites of p65 and p50. SPLINT-3HY was also challenge with a panel of interacting pairs (see Table IV). From each selection strategy several neutralizing antibodies were isolated (data not shown) thus indicating that the SPLINT-3HY strategy should provide a robust methodology to elucidate cellular mechanisms in numerous fields, of particular salience in the area of signal transduction. The assessment of gene function may be performed using the tools of protein knock-out including peptide, aptamers and antibodies. These approaches have been superseded by intracellular antibody technology, which exhibits much more potency for the investigation of protein function than the techniques listed above. In fact they can block or stabilize macromolecular interactions, modulate enzyme function by occluding an active site, sequestering substrate or fixing the enzyme in an active or inactive conformation, they can divert proteins from their usual cellular compartments, target specifically a post-transcriptionally modified version of a given protein (including heterodimerization), they can specifically target the subcellular localization of a given protein, and finally they can selectively target non-protein antigens.

SPLINT-3HY not only provides a good source of stable antibodies but also provides in one step of selection a good source of intracellular and neutralizing antibodies, thus becoming the first antibody-based selection strategy engineered for this aim.

**Table IV. Panel of interacting pairs used for SPLINT-3HY selection**

| Protein ligand x | Protein ligand y |
|---|---|
| p65 RelA | p65 RelA |
| p65 RelA | p50 NF-κB1 |
| Amyloid *β* aa. 1-42 | Amyloid *β* aa.1-42 |
| JX-TK TrkA | PTB p66 Shc |
| Synuclein | Synphilin |

### Consensus sequence framework-based library

A further refinement of the SPLINT library will be carried out with the study of scFv sequences isolated by IACT.

It has been noted that the set of antibodies selected with these technologies is characterized by a number of highly conservative residues. These critical residues represent a sort of trademark that identifies those antibodies capable of being expressed in the **intracellular** environment by a multitude of scFv which are otherwise unable to function there. IACT technology combined with SPLINT is also able to select the scFv that possess an optimum sequence very similar to a consensus sequence present in the database of immunoglobulins present in nature. As the database of intracellular antibodies selected with this technology grows, the number of conserved residues will decrease. It will therefore be possible to achieve a final generic framework on which an antibody library for intracellular use may be engineered. This will allow the creation of a library compatible with the low transformability of yeast and, more generally, a phagmid or pbagic library of highly stable scFv that are functional for expression in any cellular or noncellular environment. It can be concluded that both IACT and SPLINT are two technologies with the capability to surpass their intrinsic potential and to thus create ever more sophisticated and validated libraries.

### Sequences:

URA3 (nutritional marker that encodes the enzyme orotidine-5'phosphate decarboxylase. This enzyme is active in the last step of uracil biosynthesis and is responsible for the conversion of compound C into uracil). 
ATGTCGAAAGCTACATATAAGGAACGTGCTGCTACTCATCCTAGTCCTGTTGCTGCCAAGCTAT
TTAATATCATGCACGAAAAGCAAACAAACTTGTGTGCTTCATTGGATGTTCGTACCACCAAGG
AATTACTGGAGTTAGTTGAAGCATTAGGTCCCAAAATTTGTTTACTAAAAACACATGTGGATA
TCTTGACTGATTTTTCCATGGAGGGCACAGTTAAGCCGCTAAAGGCATTATCCGCCAAGTACA
ATTTTTTACTCTTCGAAGACAGAAAATTTGCTGACATTGGTAATACAGTCAAATTGCAGTACTC
TGCGGGTGTATACAGAATAGCAGAATGGGCAGACATTACGAATGCACACGGTGTGGTGGGCC
CAGGTATTGTTAGCGGTTTGAAGCAGGCGGCAGAAGAAGTAACAAAGGAACCTAGAGGCCTT
TTGATGTTAGCAGAATTGTCATGCAAGGGCTCCCTATCTACTGGAGAATATACTAAGGGTACT
GTTGACATTGCGAAGAGCGACAAAGATTTTGTTATCGGCTTTATTGCTCAAAGAGACATGGGT
GGAAGAGATGAAGGTTACGATTGGTTGATTATGACACCCGGTGTGGGTTTAGATGACAAGGG
AGACGCATTGGGTCAACAGTATAGAACCGTGGATGATGTGGTCTCTACAGGATCTGACATTAT
TATTGTTGGAAGAGGACTATTTGCAAAGGGAAGGGATGCTAAGGTAGAGGGTGAACGTTACA
GAAAAGCAGGCTGGGAAGCATATTTGAGAAGATGCGGCCAGCAAAACTAA
TRP1 (nutritional marker that encodes the enzyme N-(5-phosphoribosyl) anthranilate isomerase. This enzyme is found in the next to last step of tryptophan). 
ATGTCTGTTATTAATTTCACAGGTAGTTCTGGTCCATTGGTGAAAGTTTGCGGCTTGCAGAGCA
CAGAGGCCGCAGAATGTGCTCTAGATTCCGATGCTGACTTGCTGGGTATTATATGTGTGCCCA
ATAGAAAGAGAACAATTGACCCGGTTATTGCAAGGAAAATTTCAAGTCTTGTAAAAGCATATA
AAAATAGTTCAGGCACTCCGAAATACTTGGTTGGCGTGTTTCGTAATCAACCTAAGGAGGATG
TTTTGGCTCTGGTCAATGATTACGGCATTGATATCGTCCAACTGCATGGAGATGAGTCGTGGCA
AGAATACCAAGAGTTCCTCGGTTTGCCAGTTATTAAAAGACTCGTATTTCCAAAAGACTGCAA
CATACTACTCAGTGCAGCTTCACAGAAACCTCATTCGTTTATTCCCTTGTTTGATTCAGAAGCA
GGTGGGACAGGTGAACTTTTGGATTGGAACTCGATTTCTGACTGGGTTGGAAGGCAAGAGAGC
CCCGAAAGCTTACATTTTATGTTAGCTGGTGGACTGACGCCAGAAAATGTTGGTGATGCGCTT
AGATTAAATGGCGTTATTGGTGTTGATGTAAGCGGAGGTGTGGAGACAAATGGTGTAAAAGA
CTCTAACAAAATAGCAAATTTCGTCAAAAATGCTAAGAAATAG
LEU2 (a nutritionally essential marker that encodes the enzyme *β*-isopropylamate dehydrogenase. Leu3p is a positive regulator of all 3 genes for the biosynthesis of leucine and is thought to activate the expression of the genes for isoleucyl-valine ILV2 and ILV5). 
ATGTCTGCCCCTATGTCTGCCCCTAAGAAGATCGTCGTTTTGCCAGGTGACCACGTTGGTCAAG
AAATCACAGCCGAAGCCATTAAGGTTCTTAAAGCTATTTCTGATGTTCGTTCCAATGTCAAGTT
CGATTTCGAAAATCATTTAATTGGTGGTGCTGCTATCGATGCTACAGGTGTCCCACTTCCAGAT
GAGGCGCTGGAAGCCTCCAAGAAGGTTGATGCCGTTTTGTTAGGTGCTGTGGCTGGTCCTAAA
TGGGGTACCGGTAGTGTTAGACCTGAACAAGGTTTACTAAAAATCCGTAAAGAACTTCAATTG
TACGCCAACTTAAGACCATGTAACTTTGCATCCGACTCTCTTTTAGACTTATCTCCAATCAAGC
CACAATTTGCTAAAGGTACTGACTTCGTTGTTGTCAGAGAATTAGTGGGAGGTATTTACTTTGG
TAAGAGAAAGGAAGACGATGGTGATGGTGTCGCTTGGGATAGTGAACAATACACCGTTCCAG
AAGTGCAAAGAATCACAAGAATGGCCGCTTTCATGGCCCTACAACATGAGCCACCATTGCCTA
TTTGGTCCTTGGATAAAGCTAATCTTTTGGCCTCTTCAAGATTATGGAGAAAAACTGTGGAGG
AAACCATCAAGAACGAATTCCCTACATTGAAGGTTCAACATCAATTGATTGATTCTGCCGCCA
TGATCCTAGTTAAGAACCCAACCCACCTAAATGGTATTATAATCACCAGCAACATGTTTGGTG
ATATCATCTCCGATGAAGCCTCCGTTATCCCAGGTTCCTTGGGTTTGTTGCCATCTGCGTCCTTG
GCCTCTTTGCCAGACAAGAACACCGCATTTGGTTTGTACGAACCATGCCACGGTTCTGCTCCAG
ATTTGCCAAAGAATAAGGTTGACCCTATCGCCACTATCTTGTCTGCTGCAATGATGTTGAAATT
GTCATTGAACTTGCCTGAAGAAGGTAAGGCCATTGAAGATGCAGTTAAAAAGGTTTTGGATGC
AGGTATCAGAACTGGTGATTTAGGTGGTTCCAACAGTACCACCGAAGTCGGTGATGCTGTCGC
CGAAGAAGTTAAGAAAATCCTTGCTTAA
HIS3 (nutritional marker that encodes the enzyme imidazolglycerol phosphate dehydratase. This enzyme is involved in the regulation cycle of histidine biosynthesis). 
ATGACAGAGCAGAAAGCCCTAGTAAAGCGTATTACAAATGAAACCAAGATTCAGATTGCGAT
CTCTTTAAAGGGTGGTCCCCTAGCGATAGAGCACTCGATCTTCCCAGAAAGAGGCAGAAGCAG
TAGCAGAACAGGCCACACAATCGCAAGTGATTAACGTCCACACAGGTATAGGGTTTCTGGACC
ATATGATACATGCTCTGGCCAAGCATTCCGGCTGGTCGCTAATCGTTGAGTGCATTGGTGACTT
ACACATAGACGACCATCACACCACTGAAGACTGCGGGATTGCTCTCGGTCAAGCTTTTAAAGA
GGCCCTAGGGGCCGTGCGTGGAGTAAAAAGGTTTGGATCAGGATTTGCGCCTTTGGATGAGGC
ACTTTCTAGAGCGGTGGTAGATCTTTCGAACAGGCCGTACGCAGTTGTCGAACTTGGTTTGCA
AAGGGAGAAAGTAGGAGATCTCTCTTGCGAGATGATCCCGCATTTTCTTGAAAGCTTTGCAGA
GGCTAGCAGAATTACCCTCCACGTTGATTGTCTGCGAGGCAAGAATGATCATCACCGTAGTGA
GAGTGCGTTCAAGGCTCTTGCGGTTGCCATAAGAGAAGCCACCTCGCCCAATGGTACCAACGA
TGTTCCCTCCACCAAAGGTGTTCTTATGTAG
LYS2 (nutritional marker that encodes the enzyme α-aminoadipic semialdehyde. It is thought that the product of the reaction controlled by LYS2 acts as a co-inductor together with product LYS14 to stimulate the expression of numerous genes involved in the same cycle). 
ATGACTAACGAAAAGGTCTGGATAGAGAAGTTGGATAATCCAACTCTTTCAGTGTTACCACAT
GACTTTTTACGCCCACAACAAGAACCTTATACGAAACAAGCTACATATTCGTTACAGCTACCT
CAGCTCGATGTGCCTCATGATAGTTTTTCTAACAAATACGCTGTCGCTTTGAGTGTATGGGCTG
CATTGATATATAGAGTAACCGGTGACGATGATATTGTTCTTTATATTGCGAATAACAAAATCTT
AAGATTCAATATTCAACCAACGTGGTCATTTAATGAGCTGTATTCTACAATTAACAATGAGTTG
AACAAGCTCAATTCTATTGAGGCCAATTTTTCCTTTGACGAGCTAGCTGAAAAAATTCAAAGTT
GCCAAGATCTGGAAAGGACCCCTCAGTTGTTCCGTTTGGCCTTTTTGGAAAACCAAGATTTCA
AATTAGACGAGTTCAAGCATCATTTAGTGGACTTTGCTTTGAATTTGGATACCAGTAATAATGC
GCATGTTTTGAACTTAATTTATAACAGCTTACTGTATTCGAATGAAAGAGTAACCATTGTTGCG
GACCAATTTACTCAATATTTGACTGCTGCGCTAAGCGATCCATCCAATTGCATAACTAAAATCT
CTCTGATCACCGCATCATCCAAGGATAGTTTACCTGATCCAACTAAGAACTTGGGCTGGTGCG
ATTTCGTGGGGTGTATTCACGACATTTTCCAGGACAATGCTGAAGCCTTCCCAGAGAGAACCT
GTGTTGTGGAGACTCCAACACTAAATTCCGACAAGTCCCGTTCTTTCACTTATCGCGACATCAA
CCGCACTTCTAACATAGTTGCCCATTATTTGATTAAAACAGGTATCAAAAGAGGTGATGTAGT
GATGATCTATTCTTCTAGGGGTGTGGATTTGATGGTATGTGATGGGTGTCTTGAAAGCCGGC
GCAACCTTTTCAGTTATCGACCCTGCATATCCCCCAGCCAGACAAACCATTTACTTAGGTGTTG
CTAAACCACGTGGGTTGATTGTTATTAGAGCTGCTGGACAATTGGATCAACTAGTAGAAGATT
ACATCAATGATGAATTGGAGATTGTTTCAAGAATCAATTCCATCGCTATTCAAGAAAATGGTA
CCATTGAAGGTGGCAAATTGGACAATGGCGAGGATGTTTTGGCTCCATATGATCACTACAAAG
ACACCAGAACAGGTGTTGTAGTTGGACCAGATTCCAACCCAACCCTATCTTTCACATCTGGTTC
CGAAGGTATTCCTAAGGGTGTTCTTGGTAGACATTTTTCCTTGGCTTATTATTTCAATTGGATGT
CCAAAAGGTTCAACTTAACAGAAAATGATAAATTCACAATGCTGAGCGGTATTGCACATGATC
CAATTCAAAGAGATATGTTTACACCATTATTTTTAGGTGCCCAATTGTATGTCCCTACTCAAGA
TGATATTGGTACACCGGGCCGTTTAGCGGAATGGATGAGTAAGTATGGTTGCACAGTTACCCA
TTTAACACCTGCCATGGGTCAATTACTTACTGCCCAAGCTACTACACCATTCCCTAAGTTACAT
CATGCGTTCTTTGTGGGTGACATTTTAACAAAACGTGATTGTCTGAGGTTACAAACCTTGGCAG
AAAATTGCCGTATTGTTAATATGTACGGTACCACTGAAACACAGCGTGCAGTTTCTTATTTCGA
AGTTAAATCAAAAAATGACGATCCAAACTTTTTGAAAAAATTGAAAGATGTCATGCCTGCTGG
TAAAGGTATGTTGAACGTTCAGCTACTAGTTGTTAACAGGAACGATCGTACTCAAATATGTGG
TATTGGCGAAATAGGTGAGATTTATGTTCGTGCAGGTGGTTTGGCCGAAGGTTATAGAGGATT
ACCAGAATTGAATAAAGAAAAATTTGTGAACAACTGGTTTGTTGAAAAAGATCACTGGAATTA
TTTGGATAAGGATAATGGTGAACCTTGGAGACAATTCTGGTTAGGTCCAAGAGATAGATTGTA
CAGAACGGGTGATTTAGGTCGTTATCTACCAAACGGTGACTGTGAATGTTGCGGTAGGGCTGA
TGATCAAGTTAAAATTCGTGGGTTCAGAATCGAATTAGGAGAAATAGATACGCACATTTCCCA
ACATCCATTGGTAAGAGAAAACATTACTTTAGTTCGCAAAAATGCCGACAATGAGCCAACATT
GATCACATTTATGGTCCCAAGATTTGACAAGCCAGATGACTTGTCTAAGTTCCAAAGTGATGTT
CCAAAGGAGGTTGAAACTGACCCTATAGTTAAGGGCTTAATCGGTTACCATCTTTTATCCAAG
GACATCAGGACTTTCTTAAAGAAAAGATTGGCTAGCTATGCTATGCCTTCCTTGATTGTGGTTA
TGGATAAACTACCATTGAATCCAAATGGTAAAGTTGATAAGCCTAAACTTCAATTCCCAACTC
CCAAGCAATTAAATTTGGTAGCTGAAAATACAGTTTCTGAAACTGACGACTCTCAGTTTACCA
 
ATGTTGAGCGCGAGGTTAGAGACTTATGGTTAAGTATATTACCTACCAAGCCAGCATCTGTAT
CACCAGATGATTCGTTTTCGATTTAGGTGGTCATTCTATCTTGGCTACCAAAATGATTTTACC
TTAAAGAAAAAGCTGCAAGTTGATTTACCATTGGGCACAATTTTCAAGTATCCAACGATAAAG
GCCTTTGCCGCGGAAATTGACAGAATTAAATCATCGGGTGGATCATCTCAAGGTGAGGTCGTC
GAAAATGTCACTGCAAATTATGCGGAAGACGCCAAGAAATTGGTTGAGACGCTACCAAGTTC
GTACCCCTCTCGAGAATATTTGTTGAACCTAATAGTGCCGAAGGAAAAACAACAATTAATGT
GTTTGTTACCGGTGTCACAGGATTTCTGGGCTCCTACATCCTTGCAGATTTGTTAGGACGTTCT
CCAAAGAACTACAGTTTCAAAGTGTTTGCCCACGTCAGGGCCAAGGATGAAGAAGCTGCATTT
GCAAGATTACAAAAGGCAGGTATCACCTATGGTACTTGGAACGAAAAATTTGCCTCAAATATT
AAAGTTGTATTAGGCGATTTATCTAAAAGCCAATTTGGTCTTTCAGATGAGAAGTGGATGGAT
TTGGCAAACACAGTTGATATAATTATCCATAATGGTGCGTTAGTTCACTGGGTTTATCCATATG
CCAAATTGAGGGATCCAAATGTTATTTCAACTATCAATGTTATGAGCTTAGCCGCCGTCGGCA
AGCCAAAGTTCTTTGACTTTGTTTCCTCCACTTCTACTCTTGACACTGAATACTACTTTAATTTG
TCAGATAAACTTGTTAGCGAAGGGAAGCCAGGCATTTTAGAATCAGACGATTTAATGAACTCT
GCAAGCGGGCTCACTGGTGGATATGGTCAGTCCAAATGGGCTGCTGAGTACATCATTAGACGT
GCAGGTGAAAGGGGCCTACGTGGGTGTATTGTCAGACCAGGTTACGTAACAGGTGCCTCTGCC
AATGGTTCTTCAAACACAGATGATTTCTTATTGAGATTTTTGAAAGGTTCAGTCCAATTAGGTA
AGATTCCAGATATCGAAAATTCCGTGAATATGGTTCCAGTAGATCATGTTGCTCGTGTTGTTGT
TGCTACGTCTTTGAATCCTCCCAAAGAAAATGAATTGGCCGTTGCTCAAGTAACGGGTCACCC
AAGAATATTATTCAAAGACTACTTGTATACTTTACACGATTATGGTTACGATGTCGAAATCGA
AAGCTATTCTAAATGGAAGAAATCATTGGAGGCGTCTGTTATTGACAGGAATGAAGAAAATGC
GTTGTATCCTTTGCTACACATGGTCTTAGACAACTTACCTGAAAGTACCAAAGCTCCGGAACTA
GACGATAGGAACGCCGTGGCATCTTTAAAGAAAGACACCGCATGGACAGGTGTTGATTGGTCT
AATGGAATAGGTGTTACTCCAGAAGAGGTTGGTATATATATTGCATTTTTAAACAAGGTTGGA
TTTTTACCTCCACCAACTCATAATGACAAACTTCCACTGCCAAGTATAGAACTAACTCAAGCGC
AAATAAGTCTAGTTGCTTCAGGTGCTGGTGCTCGTGGAAGCTCCGCAGCAGCTTA
KAN (antibiotic resistance: it is an aminoglycosidase that acts on protein synthesis by binding to the ribosome subunit 30S to prevent the start of the complex with 'mRNA. It can also cause a messenger misreading, leading to the formation of a nonsense peptide. Another important function of aminoglycosidase is to increase membrane permeability). 
ATGATTGAACAAGATGGATTGCACGCAGGTTCTCCGGCCGCTTGGGTGGAGAGGCTATTCGGC
TATGACTGGGCACAACAGACAATCGGCTGCTCTGATGCCGCCGTGTTCCGGCTGTCAGCGCAG
GGGCGCCCGGTTCTTTTTGTCAAGACCGACCTGTCCGGTGCCCTGAATGAACTCCAAGACGAG
GCAGCGCGGCTATCGTGGCTGGCCACGACGGGCGTTCCTTGCGCAGCTGTGCTCGACGTTGTC
ACTGAAGCGGGAAGGGACTGGCTGCTATTGGGCGAAGTGCCGGGGCAGGATCTCCTGTCATCT
CACCTTGCTCCTGCCGAGAAAGTATCCATCATGGCTGATGCAATGCGGCGGCTGCATACGCTT
GATCCGGCTACCTGCCCATTCGACCACCAAGCGAAACATCGCATCGAGCGAGCACGTACTCGG
ATGGAAGCCGGTCTTGTCGATCAGGATGATCTGGACGAAGAGCATCAGGGGCTCGCGCCAGC
CGAACTGTTCGCCAGGCTCAAGGCGCGGATGCCCGACGGCGAGGATCTCGTCGTGACCCATGG
 
CGATGCCTGCTTGCCGAATATCATGGTGGAAAATGGCCGCTTTTCTGGATTCATCGACTGTGGC
CGGCTGGGTGTGGCGGACCGCTATCAGGACATAGCGTTGGCTACCCGTGATATTGCTGAAGAG
CTTGGCGGCGAATGGGCTGACCGCTTCCTCGTGCTTTACGGTATCGCCGCTCCCGATTCGCAGC
GCATCGCCTTCTATCGCCTTCTTGACGAGTTCTTCTGA
BLA (*β*-lactamase: antibiotic resistance. The product of this gene inhibits certain enzymes involved in bacterial cell wall synthesis. *β*-lactamase hydrolyzes the *β*-lactame contained in the antibiotic ampicillin). 
ATGAGTATTCAACATTTCCGTGTCGCCCTTATTCCCTTTTTTGCGGCATTTTGCCTTCCTGTTTTT
GCTCACCCAGAAACGCTGGTGAAAGTAAAAGATGCTGAAGATCAGTTGGGTGCACGAGTGGG
TTACATCGAGCTGGATCTCAACAGCGGTAAGATCCTTGAGAGTTTTCGCCCCGAAGAACGTTT
TCCAATGATGAGCACTTTTAAAGTTCTGCTATGTGGTGCGGTATTATCCCGTGTTGACGCCGGG
CAAGAGCAACTCGGTCGCCGCATACACTATTCTCAGAATGACTTGGTTGAGTACTCACCAGTC
ACAGAAAAGCATCTTACGGATGGCATGACAGTAAGAGAATTATGCAGTGCTGCCATAACCAT
GAGTGATAACACTGCGGCCAACTTACTTCTGACAACGATCGGAGGACCGAAGGAGCTAACCG
CTTTTTTGCACAACATGGGGGATCATGTAACCCGCCTTGATCGTTGGGAACCGGAGCTGAATG
AAGCCATACCAAACGACGAGCGTGACACCACGATGCCTGCAGCAATGGCAACAACGTTGCGC
AAACTATTAACTGGCGAACTACTTACTCTAGCTTCCCGGCAACAATTAATAGACTGGATGGAG
GCGGATAAAGTTGCAGGACCACTTCTGCGCTCGGCCCTTCCGGCTGGCTGGTTTATTGCTGATA
AATCTGGAGCCGGTGAGCGTGGATCTCGCGGTATCATTGCAGCACTGGGGCCAGATGGTAAGC
CCTCCCGTATCGTAGTTATCTACACGACGGGGAGTCAGGCAACTATGGATGAACGAAATAGAC
AGATCGCTGAGATAGGTGCCTCACTGATTAAGCATTGGTAA
CAM (antibiotic resistance: the product of this gene acts by binding to the ribosome subunit 50S, thus blocking the formation of peptide binding and inhibiting the activity of peptidyl transferase. It is a potent inhibitor of protein synthesis in eukaryotic cells). 
ATGGAGAAAAAAATCACTGGATATACCACCGTTGATATATCCCAATGGCATCGTAAAGAACAT
TTTGAGGCATTTCAGTCAGTTGCTCAATGTACCTATAACCAGACCGTTCAGCTGGATATTACGG
CCTTTTTAAAGACCGTAAAGAAAAATAAGCACAAGTTTTATCCGGCCTTTATTCACATTCTTGC
CCGCCTGATGAATGCTCATCCGGAATTTCGTATGGCAATGAAAGACGGTGAGCTGGTGATATG
GGATAGTGTTCACCCTTGTTACACCGTTTTCCATGAGCAAACTGAAACGTTTTCATCGCTCTGG
AGTGAATACCACGACGATTTCCGGCAGTTTCTACACATATATTCGCAAGATGTGGCGTGTTAC
GGTGAAAACCTGGCCTATTTCCCTAAAGGGTTTATTGAGAATATGTTTTTCGTCTCAGCCAATC
CCTGGGTGAGTTTCACCAGTTTTGATTTAAACGTGGCCAATATGGACAACTTCTTCGCCCCCGT
TTTCACCATGGGCAAATATTATACGCAAGGCGACAAGGTGCTGATGCCGCTGGCGATTCAGGT
TCATCATGCCGTTTGTGATGGCTTCCATGTCGGCAGAATGCTTAATGAATTACAACAGTACTGC
GATGAGTGGCAGGGCGGGGCGTAA
Tet R (antibiotic resistance: inhibits the binding of aminoacyl tRNA to the ribosome subunit 30S in bacteria). 
ATAGTGATGGCACGACTAAGCTTGGACGACGTAATTTCAATGGCGCTCACCCTGCTGGACAGC
GAAGGGCTAGAGGGCTTGACTACGCGTAAGCTGGCGCAGTCCCTAAAAATTGAGCAACCGAC
TCTGTATTGGCACGTGCGCAACAAGCAGACTCTTATGAACATGCTTTCAGAGGCAATACTGGC
GAAGCATCACACCCGTTCAGCACCGTTACCGACTGAGAGTTGGCAGCAGTTTCTCCAGGAAAA
TGCTCTGAGTTTCCGTAAAGCATTACTGGTCCATCGTGATGGAGCCCGATTGCATATAGGGAC
CTCTCCTACGCCCCCCCAGTTTGAACAAGCAGAGGCGCAACTACGCTGTCTATGCGATGCAGG
GTTTTCGGTCGAGGAGGCTCTTTTCATTCTGCAATCTATCAGCCATTTTACGTTGGGTGCAGTA
TTAGAGGAGCAAGCAACAAACCAGATAGAAAATAATCATGTGATAGACGCTGCACCACCATT
ATTACAAGAGGCATTTAATATTCAGGCGAGAACCTCTGCTGAAATGGCCTTCCATTTCGGGCT
GAAATCATTAATATTTGGATTTTCTGCACAGTTAGATGAAAAAAAGCATACACCCATTGAGGA
TGGTAATAAATGA
G418-Neomycin (antibiotic resistance. It is an aminogycoside phosphotransferase. It is used in eukaryotic, mammalian and yeast vectors). 
ATGAGCCATATTCAACGGGAAACGTCTTGCTCGAGGCCGCGATTAAATTCCAACATGATGCTG
ATTTATATGGGTATAAATGGGCTCGCGATAATGTCGGGCAATCAGGTGCACAATCTATCGATT
GTATGGGAAGCCCGATGCGCCAGAGTTGTTTCTGAAACATGGAAAGGTAGCGTTGCCAATGAT
GTTACAGATGAGATGGTCAGACTAAACTGGCTGACGAATTTATGCCTCTTCCGACCATCAAGC
ATTTTATCCGTACTCCTGATGATGCATGGTACTCACCACTGCGATCCCCGGGAAAACAGCATTC
CAGGTATTAGAAGAATATCCTGATCAGGTGAAAATATTGTTGATGCGCTGGCAGTGTTCCTGC
GCCGGTTGCATTCGATCCTGTTTGTAATTGTCCTTTTAACAGCGATCGCGTATTTCGTCTCGCTC
AGGCGCAACACGAATGAATAACGGTTTGGTTGATGCGAGTGATTTTGATGACGAGCGTAATGG
CGGCCTGTTGAACAAGTCTGGAAAGAAATGCATAAGCTTTTGCCATTCTCACCGGATTAGTCG
TCACTCATGGTGATTTCTCACTTGATAACCTTATTTTTGACGAGGGGAAATTATAGGTTGTATT
GATGTTGGACGAGTCGGAATCGCAGACCGATACCAGGATCTTGCCTCCTATGGAACTGCCTCG
GTGAGTTTTCTCCTTCATTACAGAAACGGCTTTTTCAAAAATATGGTATTGATAATCCTGATATG
AATAAATTGCAGTTTCATTTGATGCTCGATGAGTTTTTCTA
Co1E1 (origin of replication in E.coli) 
CTGTCAGACCAAGTTTACTCATATATACTTTAGATTGATTTAAAACTTCATTTTTAATTTAAAA
GGATCTAGGTGAAGATCCTTTTTGATAATCTCATGACCAAAATCCCTTAACGTGAGTTTTCGTT
CCACTGAGCGTCAGACCCCGTAGAAAAGATCAAAGGATCTTCTTGAGATCCTTTTTTTCTGCGC
GTAATCTGCTGCTTGCAAACAAAAAAACCACCGCTACCAGCGGTGGTTTGTTTGCCGGATCAA
GAGCTACCAACTCTTTTTCCGAAGGTAACTGGCTTCAGCAGAGCGCAGATACCAAATACTGTC
CTTCTAGTGTAGCCGTAGTTAGGCCACCACTTCAAGAACTCTGTAGCACCGCCTACATACCTCG
CTCTGCTAATCCTGTTACCAGTGGCTGCTGCCAGTGGCGATAAGTCGTGTCTTACCGGGTTGGA
CTCAAGACGATAGTTACCGGATAAGGCGCAGCGGTCGGGCTGAACGGGGGGTTCGTGCACAC
AGCCCAGCTTGGAGCGAACGACCTACACCGAACTGAGATACCTACAGCGTGAGCTATGAGAA
AGCGCCACGCTTCCCGAAGGGAGAAAGGCGGACAGGTATCCGGTAAGCGGCAGGGTCGGAAC
AGGAGAGCGCACGAGGGAGCTTCCAGGGGGAAACGCCTGGTATCTTTATAGTCCTGTCGGGTT
TCGCCACCTCTGACTTGAGCGTCGATTTTTGTGATGCTCGTCAGGGGGGCGGAGCCTATGGAA
 
AAACGCCAGCAACGCGGCCTTTTTACGGTTCCTGGCCTTTTGCTGGCCTTTTGCTCACATGTTC
TTTCCTGCGTTATCCCCTGATTCTGTGGATAACCGTATTACCGCCTTTGAGTGAGCTGATACCG
CTCGCCGCAGCCGAACGACCGAGCGCAGCGAGTCAGTGAGCGAGGAAGCGGAAGA
2µ (origin of replication in S.cerevisiae) 
CATTTAAGCATAAACACGCACTATGCCGTTCTTCTCATGTATATATATATACAGGCAACACGCA
GATATAGGTGCGACGTGAACAGTGAGCTGTATGTGCGCAGCTCGCGTTGCATTTTCGGAAGCG
CTCGTTTTCGGAAACGCTTTGAAGTTCCTATTCCGAAGTTCCTATTCTCTAGCTAGAAAGTATA
GGAACTTCAGAGCGCTTTTGAAAACCAAAAGCGCTCTGAAGACGCACTTTCAAAAAACCAAA
AACGCACCGGACTGTAACGAGCTACTAAAATATTGCGAATACCGCTTCCACAAACATTGCTCA
AAAGTATCTCTTTGCTATATATCTCTGTGCTATATCCCTATATAACCTACCCATCCACCTTTCGC
TCCTTGAACTTGCATCTAAACTCGACCTCTACATTTTTTATGTTTATCTCTAGTATTACTCTTTA
GACAAAAAAATTGTAGTAAGAACTATTCATAGAGTGAATCGAAAACAATACGAAAATGTAAA
CATTTCCTATACGTAGTATATAGAGACAAAATAGAAGAAACCGTTCATAATTTTCTGACCAAT
GAAGAATCATCAACGCTATCACTTTCTGTTCACAAAGTATGCGCAATCCACATCGGTATAGAA
TATAATCGGGGATGCCTTTATCTTGAAAAAATGCACCCGCAGCTTCGCTAGTAATCAGTAAAC
GCGGGAAGTGGAGTCAGGCTTTTTTATGGAAGAGAAAATAGACACCAAAGTAGCCTTCTTCT
AACCTTAACGGACCTACAGTGCAAAAAGTTATCAAGAGACTGCATTATAGAGCGCACAAAGG
AGAAAAAAAGTAATCTAAGATGCTTTGTTAGAAAAATAGCGCTCTCGGGATGCATTTTTGTAG
AACAAAAAAGAAGTATAGATTCTTTGTTGGTAAAATAGCGCTCTCGCGTTGCATTTCTGTTCTG
TAAAAATGCAGCTCAGATTCTTTGTTTGAAAAATTAGCGCTCTCGCGTTGCATTTTTGTTTTAC
AAAAATGAAGCACAGATTCTTCGTTGGTAAAATAGCGCTTTCGCGTTGCATTTCTGTTCTGTAA
AAATGCAGCTCAGATTCTTTGTTTGAAAAATTAGCGCTCTCGCGTTGCATTTTTGTTCTACAAA
ATGAAGCACAGATGCTTCGTT
LexA (DNA-binding domain) 
ATGAAAGCGTTAACGGCCAGGCAACAAGAGGTGTTTGATCTCATCCGTGATCACATCAGCCAG
ACAGGTATGCCGCCGACGCGTGCGGAAATCGCGCAGCGTTTGGGGTTCCGTTCCCCAAACGCG
GCTGAAGAACATCTGAAGGCGCTGGCACGCAAAGGCGTTATTGAAATTGTTTCCGGCGCATCA
CGCGGGATTCGTCTGTTGCAGGAAGAGGAAGAAGGGTTGCCGCTGGTAGGTCGTGTGGCTGCC
GGTGAACCACTTCTGGCGCAACAGCATATTGAAGGTCATTATCAGGTCGATCCTTCCTTATTCA
AGCCGAATGCTGATTTCCTGCTGCGCGTCAGCGGGATGTCGATGAAAGATATCGGCATTATGG
ATGGTGACTTGCTGGCAGTGCATAAAACTCAGGATGTACGTAACGGTCAGGTCGTTGTCGCAC
GTATTGATGACGAAGTTACCGTTAAGCGCCTGAAAAAACAGGGCAATAAAGTCGAACTGTTGC
CAGAAAATAGCGAGTTTAAACCAATTGTCGTTGACCTTCGTCAGCAGAGCTTCACCATTGAAG
GGCTGGCGGTTGGGGTTATTCGCAACGGCGACTGGCTG
VP16 (activation domain) 
CGGCCCCCCCGACCGATGTCAGCCTGGGGACGAGCTCCACTTAGACGGCGAGGACGTGGCG
ATGGCGCATGCCGACGCGCTAGACGATTTCGATCTGGACATGTTGGGGGACGGGGATTCCCCG
GGGCCGGGATTTACCCCCCACGACTCCGCCCCCTACGGCGCTCTGGATATGGCCGACTTCGAG
TTTGAGCAGATGTTTACCGATGCCCTTGGAATTGACGAGTACGGTGGG
B4.2 (activation domain) 
ATGGGTGCTCCTCCAAAAAAGAAGAGAAAGGTAGCTGGTATCAATAAAGATATCGAGGAGTG
CAATGCCATCATTGAGCAGTTTATCGACTACCTGCGCACCGGACAGGAGATGCCGATGGAAAT
GGCGGATCAGGCGATTAACGTGGTGCCGGGCATGACGCCGAAAACCATTCTTCACGCCGGGCC
GCCGATCCAGCCTGACTGGCTGAAATCGAATGGTTTTCATGAAATTGAAGCGGATGTTAACGA
TACCAGCCTCTTGCTGAGTGGAGATGCCTCCTACCCTTATGA
ADH1 prom (promoter) 
ATGAAAGCGTTAACGGCCAGGCAACAAGAGGTGTTTGATCTCATCCGTGATCACATCAGCCAG
ACAGGTATGCCGCCGACGCGTGCGGAAATCGCGCAGCGTTTGGGGTTCCGTTCCCCAAACGCG
GCTGAAGAACATCTGAAGGCGCTGGCACGCAAAGGCGTTATTGAAATTGTTTCCGGCGCATCA
CGCGGATTCGTCTGTTGCAGGAAGAGGAAGAAGGGTTGCCGCTGGTAGGTCGTGTGGCTGCCG
GTGAACCACTTCTGGCGCAACAGCATATTGAAGGTCATTATCAGGTCGACCTTCCTTATTCAAG
CCGAATGCTGATTTCCTGCTGCGCGTCAGCGGGATGTCGATGAAAGATATCGGCATTATGGAT
GGTGACTTGCTGGCAGTGCATAAAACTCAGGATGTACGTAACGGTCAGGTCGTTGTCGCACGT
ATTGATGACGAAGTTACCGTTAAGCGCCTGAAAAAACAGGGCAATAAAGTCGAACTGTTGCC
AGAAAATAGCGAGTTTAAACCAATTGTCGTAGATCTTCGTCAGCAGAGCTTCACCATTGAAGG
GCTGGCGGTTGGGGTTATTCGCAACGGCGACTGGCTGGAATTCCCGGGGATCCGTCGACCATG
GCGGCCGCTCGAGTCGACCTGCAGCCAAGCTAATTCCGGGCGAATTTCTTATGATTTATGA
GAL1-10 bi-directional prom (inducible bi-directional promoter) 
GGATCCCCTTTCAAAATTCTTACTTTTTTTTGGATGGACGCAAAGAAGTTTAATAATCATAT
TACATGGCATTACCACCATATACATATCCATATACATATCCATATCTAATCTTACTTATATGTT
GTGGAAATGTAAAGAGCCCCATTATCTTAGCCTAAAAAAACCTTCTCTTTGGAACTTTCAGTA
ATACGCTTAACTGCTCATTGCTATATTGAAGTACGGATTAGAAGCCGCCGAGCGGGTGACAGC
CCTCCGAAGGAAGACTCTCCTCCGTGCGTCCTCGTCTTCACCGGTCGCGTTCCTGAAACGCAGA
TGTGCCTCGCGCCGCACTGCTCCGAACAATAAAGATTCTACAATACTAGCTTTTATGGTTATGA
AGAGGAAAAATTGGCAGTAACCTGGCCCCACAAACCTTCAAATGAACGAATCAAATTAACAA
CCATAGGATGATAATGCGATTAGTTTTTAGCCTTATTTCTGGGGTAATTAATCAGCGAAGCGA
TGATTTTTGATCTATTAACAGATATATAAATGCAAAAACTGCATAACCACTTTAACTAATACTT
TCAACATTTTCGGTTTGTATTACTTCTTATTCAAATGTAATAAAAGTATCAACAAAAAATTGTT
AATATACCTCTATACTTTAACGTCAAGGAGAAAAAACCCGGG
GAL1 prom (inducible promoter) 
ACGGATTAGAAGCCGCCGAGCGGGTGACAGCCCTCCGAAGGAAGACTCTCCTCCGTGCGTCCT
CGTCTTCACCGGTCGCGTTCCTGAAACGCAGATGTGCCTCGCGCCGCACTGCTCCGAACAATA
AAGATTCTACAATACTAGCTTTTATGGTTATGAAGAGGAAAAATTGGCAGTAACCTGGCCCCA
CAAACCTTCAAATGAACGAATCAAATTAACAACCATAGGATGATAATGCGATTAGTTTTTTAG
CCTTATTTCTGGGGTAATTAATCAGCGAAGCGATGATTTTTGATCTATTAACAGATATATAAAT
GCAAAAACTGCATAACCACTTTAACTAATACTTTCAACATTTTCGGTTTGTATTACTTCTTATTC
AAATGTAATAAAAGTATCAACAAAAAATTGTTAATATACCTCTATACTTTAACGTCAAGGAGA
AAAAACC
ADH1 term (transcription stop sequence) 
AAGCTTTTCATAGGGTAGGGGAATTTCGGCATGCCGGTAGAGGTGTGGTCAATAAGAGCGAC
CTCATGCTATACCTGAGAAAGCAACCTGACCTACAGGAAAGAGTTACTCAAGAACAAGAATTT
TCGTTTTAAAACCTAAGAGTCACTTTAAAATTTGTATACACTTATTTTTTTATAACTTATTTAA
TAATAAAAATCATAAATCATAAGAAATTCGCTTATTTAGAAGTGTCAACAACGTATCTACCAA
CGATTTGACCCTTTTCCATCTTTTCGTAAATTTCTGGCAAGGTAGACAAGCCGACAACCTTGAT
TGGAGACTTGACCAAACCTCTGGCGAAGAA GTCCA
ADH2 term (transcription stop sequence) 
GAATTCGACACTTCTAAATAAGCGGATCTCCTATGCCTTCACGATTTATAGTTTCCATTATCAA
GTATGCCTATATTAGTATATAGCATCTTTAGATGACAGTGTTCGAAGTTTCACGAATAAAAGAT
AATATTCTACTTTTTGCTCCCACCGCGTTTGCTAGCACGAGTGAACACCATCCCTCGCCTGTGA
GTTGTACCCATTCCTCTAAACTGTAGACATGGTAGCTTCAGCAGTGTTCGTTATGTACGGCATC
CTCCAACAAACAGTCGGTTATAGTTTGTCCTGCTCCTCTGAATCGTCTCCCTCGATATTTCTCAT
TTTCCTTCGCATGCCCTCGAG
CRE GENE 
ATGTCCAATTTACTGACCGTACACCAAAATTTGCCTGCATTACCGGTCGATGCAACGAGTGAT
GAGGTTCGCAAGAACCTGATGGACATGTTCAGGGATCGCCAGGCGTTTTCTGAGCATACCTGG
AAAATGCTTCTGTCCGTTTGCCGGTCGTGGGCGGCATGGTGCAAGTTGAATAACCGGAAATGG
TTTCCCGCAGAACCTGAAGATGTTCGCGATTATCTTCTATATCTTCAGGCGCGCGGTCTGGCAG
TAAAAACTATCCAGCAACATTTGGGCCAGCTAAACATGCTTCATCGTCGGTCCGGGCTGCCAC
GACCAAGTGACAGCAATGCTGTTTCACTGGTTATGCGGCGGATCCGAAAAGAAAACGTTGATG
CCGGTGAACGTGCAAAACAGGCTCTAGCGTTCGAACGCACTGATTTCGACCAGGTTCGTTCAC
TCATGGAAAATAGCGATCGCTGCCAGGATATACGTAATCTGGCATTTCTGGGGATTGCTTATA
ACACCCTGTTACGTATAGCCGAAATTGCCAGGATCAGGGTTAAAGATATCTCACGTACTGACG
GTGGGAGAATGTTAATCCATATTGGCAGAACGAAAACGCTGGTTAGCACCGCAGGTGTAGAG
AAGGCACTTAGCCTGGGGGTAACTAAACTGGTCGAGCGATGGATTTCCGTCTCTGGTGTAGCT
GATGATCCGAATAACTACCTGTTTTGCCGGGTCAGAAAAAATGGTGTTGCCGCGCCATCTGCC
ACCAGCCAGCTATCAACTCGCGCCCTGGAAGGGATTTTTGAAGCAACTCATCGATTGATTTAC
GGCGCTAAGGATGACTCTGGTCAGAGATACCTGGCCTGGTCTGGACACAGTGCCCGTGTCGGA
GCCGCGCGAGATATGGCCCGCGCTGGAGTTTCAATACCGGAGATCATGCAAGCTGGTGGCTGG
ACCAATGTAAATATTGTCATGAACTATATCCGTACCCTGGATAGTGAAACAGGGGCAATGGTG
CGCCTGCTGGAAGATGGCGATTAG
PolyLINKER pMIC-BD1 and pMIC-BD2 and pBTM116
   GAATTCCCGGGGATCCGTCGACCTGCAG
POLYLINKER pVP16
   GGCCCAGCCGGCCGTCGACGCGGCCGCCTCGAG
POLYLINKERS plinker220 and pVP16mv1

GCGCGCATGCC….(VL)….TCCGGAGGTCGACCAGCGGTTCTGGAAACCAGGTTCCGGTGAA
GGCTCGAGCGGTACC….(VH)….GCTAGCGGCAAACCAATCCCAAACCCACTGCTGGGCCTGGA
TAGTACT

### REFERENCES

Baird, G.S., Zacharias, D.A. and Tsien, R.Y. (1999) Circular permutation and receptor insertion within green fluorescent proteins. Proc Natl Acad Sci USA 96, 11241-6.
Bartel, P.L., Chien, C., Sternglanz, R and Fields, S. (1993) Using the two-hybrid system to detect protein-protein interaction. In: D.A. Hartley (Ed) Cellular interaction in development: a practical approach. IRL Press, Oxford, p. 153-179.
Biocca, S., Neuberger, M.S. and Cattaneo, A. (1990) Expression and targeting of intracellular antibodies in mammalian cells. Embo J 9, 101-8.
Biocca, S., Ruberti, F., Tafani, M., Pierandrei-Amaldi, P. and Cattaneo, A. (1995) Redox state of single chain Fv fragments targeted to the endoplasmic reticulum, cytosol and mitochondria. Biotechnology (N Y) 13, 1110-5.
Capecchi, M.R. (1989) Altering the genome by homologous recombination. Science 244, 1288-92.
Cattaneo, A. and Biocca, S. (1997) Intracellular Antibodies: Development and Applications.
Cattaneo, A. and Biocca, S. (1999) The selection of intracellular antibodies. Trends in Biotechnology 17, 115-121.
Chen, Y.Q., Sengchanthalangsy, L.L., Hackett, A. and Ghosh, G. (2000) NF-kappaB p65 (Re1A) homodimer uses distinct mechanisms to recognize DNA targets. Structure Fold Des 8, 419-28.
Clarke, L. and Carbon, J. (1976) A colony bank containing synthetic Col El hybrid plasmids representative of the entire E. coli genome. Cell 9, 91-9.
Engberg, J., Andersen, P.S., Nielsen, L.K., Dziegiel, M., Johansen, L.K. and Albrechtsen, B. (1996) Phage-display libraries of murine and human antibody Fab fragments. Mol Biotechnol 6, 287-310.
Feldhaus, M.J., Siegel, R.W., Opresko, L.K., Coleman, J.R., Feldhaus, J.M., Yeung, Y.A., Cochran, J.R., Heinzelman, P., Colby, D., Swers, J., Graff, C., Wiley, H.S. and Wittrup, K.D. (2003) Flow-cytometric isolation of human antibodies from a nonimmune Saccharomyces cerevisiae surface display library. Nat Biotechnol 21, 163-70.
Fire, A. (1999) RNA-triggered gene silencing. Trends Genet 15, 358-63.
Glass, C.K. and Rosenfeld, M.G. (2000) The coregulator exchange in transcriptional functions of nuclear receptors. Genes Dev 14, 121-41.
Griffiths, A.D., Malmqvist, M., Marks, J.D., Bye, J.M., Embleton, M.J., McCafferty, J., Baier, M., Holliger, K.P., Gorick, B.D., Hughes-Jones, N.C. and et al. (1993) Human anti-self antibodies with high specificity from phage display libraries. Embo J 12, 725-34.
Griffiths, A.D., Williams, S.C., Hartley, O., Tomlinson, LM., Waterhouse, P., Crosby, W.L., Kontermann, R.E., Jones, P.T., Low, N.M., Allison, T.J. and et al. (1994) Isolation of high affinity human antibodies directly from large synthetic repertoires. Embo J 13, 3245-60.
Hollenberg, S.M., Sternglanz, R., Cheng, P.F. and Weintraub, H. (1995) Identification of a new family of tissue -specific basic helix-loop-helix proteins with a two-hybrid system. Molecular and Cellular Biology 15, 3813-3822.
Kortt, A.A., Dolezal, O., Power, B.E. and Hudson, P.J. (2001) Dimeric and trimeric antibodies: high avidity scFvs for cancer targeting. Biomol Eng 18, 95-108.
Kunsch, C., Ruben, S.M. and Rosen, C.A. (1992) Selection of optimal kappa B/Rel DNA-binding motifs: interaction of both subunits of NF-kappa B with DNA is required for transcriptional activation. Mol Cell Biol 12, 4412-21.
Orlandi, R., Gussow, D.H., Jones, P.T. and Winter, G. (1992) Cloning immunoglobulin variable domains for expression by the polymerase chain reaction. 1989. Biotechnology 24,527-31.
Persic, L., Righi, M., Roberts, A., Hoogenboom, H.R., Cattaneo, A. and Bradbury, A. (1997) Targeting vectors for intracellular immunisation. Gene 187,1-8.
Prasher, D.C., Eckenrode, V.K., Ward, W.W., Prendergast, F.G. and Cormier, M.J. (1992) Primary structure of the Aequorea victoria green-fluorescent protein. Gene 111, 229-33.
Proba, K., Honegger, A. and Pluckthun, A. (1997) A natural antibody missing a cysteine in VH: consequences for thermodynamic stability and folding. J Mol Biol 265, 161-72.
Proba, K., Worn, A., Honegger, A. and Pluckthun, A. (1998) Antibody scFv fragments without disulfide bonds made by molecular evolution. J Mol Biol 275, 245-53.
Rhee, Y., Gurel, F., Gafni, Y., Dingwall, C. and Citovsky, V. (2000) A genetic system for detection of protein nuclear import and export. Nat Biotechnol 18, 433-7.
Rossi, J.J. (1999) Ribozymes, genomics and therapeutics. Chem Biol 6, R33-7.
Sblattero, D. and Bradbury, A. (2000) Exploiting recombination in single bacteria to make large phage antibody libraries. Nat Biotechnol 18, 75-80.
Sheets, M.D., Amersdorfer, P., Finnem, R., Sargent, P., Lindquist, E., Schier, R., Hemingsen, G., Wong, C., Gerhart, J.C., Marks, J.D. and Lindqvist, E. (1998) Efficient construction of a large nonimmune phage antibody library: the production of high-affinity human single-chain antibodies to protein antigens (Sheets et al.). Proc Natl Acad Sci U S A 95, 6157-62.
Siegel, M.S. and Isacoff, E.Y. (1997) A genetically encoded optical probe of membrane voltage. Neuron 19, 735-41.
Tanaka, T., Chung, G.T., Forster, A., Lobato, M.N. and Rabbitts, T.H. (2003) De novo production of diverse intracellular antibody libraries. Nucleic Acids Res 31, e23.
Tavernarakis, N., Wang, S.L., Dorovkov, M., Ryazanov, A. and Driscoll, M. (2000) Heritable and inducible genetic interference by double-stranded RNA encoded by transgenes. Nat Genet 24,180-3.
Todorovska, A., Roovers, R.C., Dolezal, O., Kortt, A.A., Hoogenboom, H.R. and Hudson, P.J. (2001) Design and application of diabodies, triabodies and tetrabodies for cancer targeting. J Immunol Methods 248, 47-66.
Tomlinson, I. and Holliger, P. (2000) Methods for generating multivalent and bispecific antibody fragments. Methods Enzymol 326, 461-79.
Uetz, P., Giot, L., Cagney, G., Mansfield, T.A., Judson, R.S., Knight, J.R., Lockshon, D., Narayan, V., Srinivasan, M., Pochart, P., Qureshi-Emili, A., Li, Y., Godwin, B., Conover, D., Kalbfleisch, T., Vijayadamodar, G., Yang, M., Johnston, M., Fields, S. and Rothberg, J.M. (2000) A comprehensive analysis of protein-protein interactions in Saccharomyces cerevisiae. Nature 403, 623-7.
Vaughan, T.J., Williams, A.J., Pritchard, K., Osbourn, J.K., Pope, A.R, Earnshaw, J.C., McCafferty, J., Hodits, R.A., Wilton, J. and Johnson, K.S. (1996) Human antibodies with sub-nanomolar affinities isolated from a large non-immunized phage display library (Uetz et al.). Nat Biotechnol 14, 309-14.
Visintin, M. and Cattaneo, A. (2001) Selecting intracellular antibodies using the two-hybrid system. In: R. Kontermann, & Dubel, S., eds (Ed) Antibody Engineering, Vol. 1, Springer Lab Manual. Springer, Heidelberg, Germany, p. 790.
Visintin, M., Settanni, G., Maritan, A., Graziosi, S., Marks, J.D. and Cattaneo, A. (2002) The intracellular antibody capture technology (IACT): towards a consensus sequence for intracellular antibodies. J Mol Biol 317, 73-83.
Visintin, M., Tse, E., Axelson, H., Rabbitts, T.H. and Cattaneo, A. (1999) Selection of antibodies for intracellular function using a two-hybrid in vivo system. In: Proc Natl Acad Sci USA, Vol. 96, p. 11723-8.
Visintin, M., Tse, E., Axelson, H., Rabbitts, T.H. and Cattaneo, A. (1999) Selection of antibodies for intracellular function using a two-hybrid in vivo system. In: Proc Natl Acad Sci USA, Vol. 96, p. 11723-8.
Wu, A.M. and Yazaki, P.J. (2000) Designer genes: recombinant antibody fragments for biological imaging. Q J Nucl Med 44,268-83.
Zhong, H., SuYang, H., Erdjument-Bromage, H., Tempst, P. and Ghosh, S. (1997) The transcriptional activity of NF-kappaB is regulated by the IkappaB-associated PKAc subunit through a cyclic AMP-independent mechanism. Cell 89,413-24.
Zhong, H., Voll, R.E. and Ghosh, S. (1998) Phosphorylation of NF-kappa B p65 by PKA stimulates transcriptional activity by promoting a novel bivalent interaction with the coactivator CBP/p300. Mol Cell 1, 661-71.

## Claims

1. A method to isolate intracellular antibodies able to neutralize an interaction between a known protein ligand x and a known protein ligand y inside a cell, comprising the steps of:
a) obtaining a recombinant yeast strain wherein:
i) a sequence encoding the protein ligand x and a sequence encoding the protein ligand y are cloned into a first yeast expression vector, but
ii) at least one of x or y is expressed in an inducible manner, and
iii) the interaction between the protein ligand x and the protein ligand y would lead to the production either of a repressor of an essential function of the yeast cell or of a toxic agent, so that non viable yeast cells would be obtained;
b) transforming the recombinant yeast strain of point a) with an expression library of intracellular antibodies by means of a second yeast expression vector;
c) selecting transformed recombinant yeast strain of point b) by inducing the expression of both ligand x and ligand y; and by isolating recipient yeast clones able to grow;
d) isolating intracellular antibodies able to neutralize the interaction between the protein ligand x and the protein ligand y from recipient recombinant yeast clones able to grow, wherein the expression library of intracellular antibodies is obtained from lymphocytes of non immunized mice and is expressed directly in the yeast cells without utilizing the phage display screening.

2. A method to isolate intracellular antibodies able to neutralize an interaction between a known protein ligand x and a known protein ligand y inside a cell, comprising the steps of:
a) obtaining a recombinant yeast strain having a mat-a or mat-alpha sexual competence wherein:
i) a sequence encoding the protein ligand x and a sequence encoding the protein ligand y are closed into a first yeast expression vector, but
ii) at least one of x or y is expressed in an inducible manner, and
iii) the interaction between the protein ligand x and the protein ligand y would lead to the production either of a repressor of an essential function of the yeast cell or of a toxic agent, so that non viable yeast cells would be obtained;
b) obtaining an expression library of intracellular antibodies by means of a second yeast expression vector in a yeast strain with a sexual competence opposite to that of the yeast strain in point a);
c) exposing yeasts of point a) and recombinant yeasts of point b) to conditions able to promote sexual mating and the expression of the ligands x and y;
d) selecting transformed yeast clones by inducing the expression of both ligand x and ligand y; and by isolating recipient yeast clones able to grow;
e) isolating intracellular antibodies able to neutralize the interaction between the protein ligand x and the protein ligand y from recipient recombinant yeast clones able to grow, wherein the expression library of intracellular antibodies is obtained from lymphocytes of non immunized mice and is expressed directly in the yeast cells without utilizing the phage display screening.

3. The method according to any of previous claims wherein the recombinant yeast strain is obtained by:
a) cloning a first sequence encoding the protein ligand x and a second sequence encoding the protein ligand y into the first yeast expression vector, each first and second sequence being under the control of each one of two portions of bidirectional promoters, the first sequence being fused to a sequence encoding a first molecule, and the second sequence being fused to a sequence encoding a second molecule, so that when the protein ligand x and the protein ligand y interact the production either of a repressor of an essential function of the yeast cell or of a toxic agent is induced;
b) transforming the recipient yeast strain with the yeast expression vector of point a); and
c) selecting transformed yeasts.

4. The method according to claim 3 wherein the bidirectional promoters are the promoters Gal 1 and Gal 10.

5. The method according to claim 3 wherein the first and the second molecule leads to the production of a repressor able to shut down the transcription of the yeast HIS3 gene.

6. The method according to any of previous claims in which the expression library of intracellular antibodies is obtained in a scFv or in a single domain antibody format.

7. A method to simultaneously identify both a protein ligand x able to bind to a known protein ligand y inside an eukaryotic cell, and an intracellular antibody able to neutralize said interaction between the protein ligand x and the known protein ligand y, comprising the steps of:
a) cloning a cDNA library containing sequences encoding protein ligand x and a sequence encoding the protein ligand y into a first yeast expression vector, the cDNA library sequences being under the control of one of two portions of a bidirectional inducible promoter, the cDNA library sequences being fused to a sequence encoding a first molecule; and the sequence encoding the protein ligand y being under the control of the other of two portions of the bidirectional promoter, and being fused to a sequence encoding a second molecule, so that when the protein ligand x and the protein ligand y interact said first molecule and said second molecule interact too, in a way to induce the production either of a repressor of an essential function of the yeast cell or of a toxic agent;
b) transforming a yeast strain with mat-a or mat-alpha sexual competence with the recombinant first yeast expression vector of point a);
c) selecting transformed yeasts;
d) obtaining an expression library of intracellular antibodies in a yeast strain having a sexually competence opposite to that of the yeast strain in point a) by means of a second yeast expression vector;
e) submitting the yeasts of point c) and the yeasts of point d) to such conditions to promote sexual reproduction and the expression of the bidirectional promoter in an environment able to induce the expression of the protein ligand x and protein ligand y;
f) selecting viable yeast clones in which the interaction between the protein ligand x and the protein ligand y is disrupted by the intracellular antibody;
g) identifying the protein ligand x and the intracellular antibody,
wherein the expression library of intracellular antibodies is obtained from lymphocytes of non immunized mice and is expressed directly in the yeast cells without utilizing the phage display screening.

## Patentansprüche

1. Verfahren zum Isolieren intrazellulärer Antikörper, die zum Neutralisieren einer Wechselwirkung zwischen einem bekannten Proteinliganden x und einem bekannten Proteinliganden y in einer Zelle geeignet sind, das die folgenden Schritte umfasst:
a) Erhalten eines rekombinanten Hefestammes, wobei:
i) eine den Proteinliganden x kodierende Sequenz und eine den Proteinliganden y kodierende Sequenz in einen ersten Hefeexpressionsvektor kloniert werden, aber
ii) mindestens eine von x oder y induzierbar exprimiert wird, und
iii) die Wechselwirkung zwischen dem Proteinliganden x und dem Proteinliganden y zur Produktion entweder eines Repressors einer essenziellen Funktion der Hefezelle oder eines toxischen Mittels führen würde, sodass keine lebensfähigen Hefezellen erhalten werden würden;
b) Transformieren des rekombinanten Hefestammes von Punkt a) mit einer Expressionsbibliothek intrazellulärer Antikörper mithilfe eines zweiten Hefeexpressionsvektors;
c) Auswählen des transformierten rekombinanten Hefestammes von Punkt b) durch Induzieren der Expression von Ligand x und Ligand y und durch Isolieren wachstumsfähiger Empfänger-Hefeklone;
d) Isolieren intrazellulärer Antikörper, die zum Neutralisieren der Wechselwirkung zwischen dem Proteinliganden x und dem Proteinliganden y in der Lage sind, aus rekombinanten wachstumsfähigen Empfänger-Hefeklonen,
wobei die Expressionsbibliothek intrazellulärer Antikörper von Lymphozyten nicht-immunisierter Mäuse erhalten wird und in den Hefezellen ohne Anwendung des Phagenpräsentations durch musterungs verfahrens direkt exprimiert wird.

2. Verfahren zum Isolieren intrazellulärer Antikörper, die zum Neutralisieren einer Wechselwirkung zwischen einem bekannten Proteinliganden x und einem bekannten Proteinliganden y in einer Zelle geeignet sind, das die folgenden Schritte umfasst:
a) Erhalten eines rekombinanten Hefestammes, der eine Paarungskompetenz vom Typ mat-a oder mat-alpha aufweist, wobei:
i) eine den Proteinliganden x kodierende Sequenz und eine den Proteinliganden y kodierende Sequenz in einen ersten Hefeexpressionsvektor kloniert werden, aber
ii) mindestens eine von x oder y induzierbar exprimiert wird, und
iii) die Wechselwirkung zwischen dem Proteinliganden x und dem Proteinliganden y zur Produktion entweder eines Repressors einer essenziellen Funktion der Hefezelle oder eines toxischen Mittels führen würde, sodass keine lebensfähigen Hefezellen erhalten werden würden;
b) Erhalten einer Expressionsbibliothek intrazellulärer Antikörper mithilfe eines zweiten Hefeexpressionsvektors in einem Hefestamm mit einer der Paarungskompetenz des Hefestammes in Punkt a) gegenüberstehenden Paarungskompetenz;
c) Aussetzen von Hefen aus Punkt a) und rekombinanter Hefen von Punkt b) gegenüber Bedingungen, welche zum Fördern der geschlechtlichen Paarung und der Expression der Liganden x und y geeignet sind;
d) Auswählen transformierter Hefeklone durch Induzieren der Expression von Ligand x und von Ligand y und durch Isolieren wachstumsfähiger Empfänger-Hefeklone;
e) Isolieren intrazellulärer Antikörper, die zum Neutralisieren der Wechselwirkung zwischen dem Proteinliganden x und dem Proteinliganden y in der Lage sind, aus rekombinanten wachstumsfähigen Empfänger-Hefeklonen,
wobei die Expressionsbibliothek intrazellulärer Antikörper von Lymphozyten nicht-immunisierter Mäuse erhalten wird und in den Hefezellen ohne Anwendung des Phagenpräsentations durch musterungs verfahrens direkt exprimiert wird.

3. Verfahren nach einem der vorherigen Ansprüche, wobei der rekombinante Hefestamm folgenderweise erhalten wird:
a) durch Klonieren einer ersten Sequenz, welche den Proteinliganden x kodiert, und einer zweiten Sequenz, welche den Proteinliganden y kodiert, in den ersten Hefeexpressionsvektor, wobei jede erste und zweite Sequenz unter der Kontrolle von jedem der zwei Anteile bidirektionaler Promotoren steht, wobei die erste Sequenz mit einer ein erstes Molekül kodierenden Sequenz fusioniert ist und die zweite Sequenz mit einer ein zweites Molekül kodierenden Sequenz fusioniert ist, so dass die Produktion entweder eines Repressors einer essenziellen Funktion der Hefezelle oder eines toxischen Mittels induziert wird, wenn der Proteinligand x und der Proteinligand y wechselwirken;
b) Transformieren des Empfänger-Hefestammes mit dem Hefeexpressionsvektor von Punkt a); und
c) Auswählen transformierter Hefen.

4. Verfahren nach Anspruch 3, wobei es sich bei den bidirektionalen Promotoren um die Promotoren Gal 1 und Gal 10 handelt.

5. Verfahren nach Anspruch 3, wobei das erste und das zweite Molekül zu der Produktion eines Repressors führen, der zum Abschalten der Transkription des Hefegens HIS3 in der Lage ist.

6. Verfahren nach einem der vorherigen Ansprüche, wobei die Expressionsbibliothek intrazellulärer Antikörper in einem scFv-Format oder in einem Einzel domänen antikör per format erhalten wird.

7. Verfahren zum gleichzeitigen Identifizieren sowohl eines Proteinliganden x, der zum Binden an einen bekannten Proteinliganden x in einer eukaryotischen Zelle in der Lage ist, als auch eines intrazellulären Antikörpers, der zum Neutralisieren der Wechselwirkung zwischen dem Proteinliganden x und dem bekannten Proteinliganden y in der Lage ist, welches die folgenden Schritte umfasst:
a) Klonieren einer cDNA-Bibliothek, welche Sequenzen enthält, die Proteinliganden x kodieren, und eine Sequenz, welche den Proteinliganden y kodiert, in einen ersten Hefeexpressionsvektor, wobei die Sequenzen in der cDNA-Bibliothek unter der Kontrolle von einem der zwei Anteile eines bidirektionalen Promotors stehen, die Sequenzen in der cDNA-Bibliothek mit einer ein erstes Molekül kodierenden Sequenz fusioniert sind; und die den Proteinliganden y kodierende Sequenz unter der Kontrolle des anderen der zwei Anteile des bidirektionalen Promotors steht und mit einer ein zweites Molekül kodierenden Sequenz fusioniert ist, sodass, wenn der Proteinligand x und der Proteinligand y wechselwirken, das erste Molekül und das zweite Molekül ebenfalls auf eine Weise wechselwirken, um die Produktion entweder eines Repressors einer essenziellen Funktion der Hefezelle oder eines toxischen Mittels zu induzieren;
b) Transformieren eines Hefestammes mit Paarungskompetenz von Typ mat-a oder mat-alpha mit dem rekombinanten ersten Hefeexpressionsvektor von Punkt a) ;
c) Auswählen transformierter Hefen;
d) Erhalten einer Expressionsbibliothek intrazellulärer Antikörper mithilfe eines zweiten Hefeexpressionsvektors in einem Hefestamm mit einer der Paarungskompetenz des Hefestammes in Punkt a) gegenüberstehenden Paarungskompetenz;
e) Aussetzen der Hefen aus Punkt c) und der Hefen von Punkt d) gegenüber Bedingungen, welche zum Fördern der geschlechtlichen Paarung und der Expression des bidirektionalen Promotors in einer zum Induzieren der Expression des Proteinliganden x und des Proteinliganden y geeigneten Umgebung geeignet sind;
f) Auswählen lebensfähiger Hefeklone, in denen die Wechselwirkung zwischen dem Proteinliganden x und dem Proteinliganden y durch den intrazellulären Antikörper unterbrochen ist;
g) Identifizieren des Proteinliganden x und des intrazellulären Antikörpers,
wobei die Expressionsbibliothek intrazellulärer Antikörper von Lymphozyten nicht-immunisierter Mäuse erhalten wird und in den Hefezellen ohne Anwendung des Phagenpräsentations durch musterungs verfahrens direkt exprimiert wird.

## Revendications

1. Procédé d'isolement d'anticorps intracellulaires capables de neutraliser une interaction entre un ligand protéique x connu et un ligand protéique y connu à l'intérieur d'une cellule, ledit procédé comprenant les étapes de :
a) obtention d'une souche de levure recombinante dans laquelle :
i) une séquence codant pour le ligand protéique x et une séquence codant pour le ligand protéique y sont clonées dans un premier vecteur d'expression de levure, mais
ii) au moins l'un de x ou de y est exprimé d'une manière inductible, et
iii) l'interaction entre le ligand protéique x et le ligand protéique y conduirait à la production soit d'un répresseur d'une fonction essentielle de la cellule de levure ou d'un agent toxique, de telle sorte que des cellules de levure non viables seraient obtenues ;
b) transformation de la souche de levure recombinante du point a) avec une banque d'expression d'anticorps intracellulaires au moyen d'un second vecteur d'expression de levure ;
c) sélection de clones de souches de levures transformés du point b) par induction de l'expression des deux ligand x et ligand y ; et par isolement de clones de levure receveurs capables de croitre ;
d) isolement d'anticorps intracellulaires capables de neutraliser l'interaction entre le ligand protéique x et le ligand protéique y de clones à partir de levure recombinants receveurs capables de croitre, dans lequel la banque d'expression d'anticorps intracellulaires est obtenue à partir de lymphocytes de souris non immunisées et est exprimée directement dans les cellules de levure sans utiliser le criblage par présentation phagique.

2. Procédé d'isolement d'anticorps intracellulaires capables de neutraliser une interaction entre un ligand protéique x connu et un ligand protéique y connu à l'intérieur d'une cellule, ledit procédé comprenant les étapes de :
a) obtention d'une souche de levure recombinante ayant un type de compétence sexuelle mat-a ou mat-alpha, dans lequel :
i) une séquence codant pour le ligand protéique x et une séquence codant pour le ligand protéique y sont clonées dans un premier vecteur d'expression de levure, mais
ii) au moins l'un de x ou de y est exprimé d'une manière inductible, et
iii) l'interaction entre le ligand protéique x et le ligand protéique y conduirait à la production soit d'un répresseur d'une fonction essentielle de la cellule de levure ou d'un agent toxique, de telle sorte que des cellules de levure non viables seraient obtenues ;
b) obtention d'une banque d'expression d'anticorps intracellulaires au moyen d'un second vecteur d'expression de levure dans une souche de levure avec une compétence sexuelle opposée à celle de la souche de levure du point a) ;
c) exposition de levures du point a) et des levures recombinantes du point b) à des conditions capables de promouvoir l'accouplement sexuel et l'expression des ligands x et y ;
d) sélection de clones de levures transformés par induction de l'expression des deux ligand x et ligand y ; et par isolement de clones de levure receveurs capables de croitre ;
e) isolement d'anticorps intracellulaires capables de neutraliser l'interaction entre le ligand protéique x et le ligand protéique y à partir de clones de levure recombinants receveurs capables de croitre, dans lequel la banque d'expression d'anticorps intracellulaires est obtenue à partir de lymphocytes de souris non immunisées et est exprimée directement dans les cellules de levure sans utiliser le criblage par présentation phagique.

3. Procédé selon l'une quelconque des revendications précédentes, dans lequel la souche de levure recombinante est obtenue par :
a) clonage d'une première séquence codant pour le ligand protéique x et d'une seconde séquence codant pour le ligand protéique y dans le premier vecteur d'expression de levure, chacune de la première séquence et de la seconde séquence se trouvant sous le contrôle de chacune de deux parties de promoteurs bidirectionnels, la première séquence étant fusionnée à une séquence codant pour une première molécule, et la seconde séquence étant fusionnée à une séquence codant pour une seconde molécule, de telle sorte que lorsque le ligand protéique x et le ligand protéique y interagissent, la production soit d'un répresseur d'une fonction essentielle de la cellule de levure ou d'un agent toxique est induite ;
b) transformation de la souche de levure receveur avec le vecteur d'expression de levure du point a) ; et
c) sélection de levures transformées.

4. Procédé selon la revendication 3, dans lequel les promoteurs bidirectionnels sont les promoteurs Gal 1 et Gal 10.

5. Procédé selon la revendication 3, dans lequel les première et seconde molécules conduisent à la production d'un répresseur capable d'arréter la transcription du gène HIS3 de levure.

6. Procédé selon l'une quelconque des revendications précédentes, dans lequel la banque d'expression d'anticorps intracellulaires est obtenue dans un format d'anticorps scFv ou à domaine unique.

7. Procédé d'identification simultanée d'un ligand protéique x capable de se lier à un ligand protéique y connu à l'intérieur d'une cellule eucaryote, et d'un anticorps intracellulaire capable de neutraliser ladite interaction entre le ligand protéique x et le ligand protéique y connu, ledit procédé comprenant les étapes de :
a) clonage d'une banque d'ADNc contenant des séquences codant pour le ligand protéique x et une séquence codant pour le ligand protéique y dans un premier vecteur d'expression de levure, les séquences de la banque d'ADNc étant sous le contrôle de l'une de deux parties d'un promoteur bidirectionnel inductible, les séquences de la banque d'ADNc étant fusionnées à une séquence codant pour une première molécule ; et la séquence codant pour le ligand protéique y étant sous le contrôle des deux parties du promoteur bidirectionnel, et étant fusionnée à une séquence codant pour une seconde molécule, de telle sorte que lorsque le ligand protéique x et le ligand protéique y interagissent ladite première molécule et ladite seconde molécule interagissent également, de manière à induire la production soit d'un répresseur d'une fonction essentielle de la cellule de levure ou d'un agent toxique ;
b) transformation d'une souche de levure avec un type de compétence sexuelle mat-a ou mat-alpha avec le premier vecteur d'expression de levure recombinant du point a) ;
c) sélection de levures transformées ;
d) obtention d'une banque d'expression d'anticorps intracellulaires dans une souche de levure ayant une compétence sexuelle opposée à celle de la souche de levure du point a) au moyen d'un second vecteur d'expression de levure ;
e) soumission des levures du point c) et des levures du point d) à de telles conditions pour promouvoir la reproduction sexuelle et l'expression du promoteur bidirectionnel dans un environnement capable d'induire l'expression du ligand protéique x et du ligand protéique y ;
f) sélection de clones de levure viables dans lesquels l'interaction entre le ligand protéique x et le ligand protéique y est perturbée par l'anticorps intracellulaire ;
g) identification du ligand protéique x et de l'anticorps intracellulaire,
dans lequel la banque d'expression d'anticorps intracellulaires est obtenue à partir de lymphocytes de souris non immunisées et est exprimée directement dans les cellules de levure sans utiliser le criblage par présentation phagique.
